# EUROPÄISCHE PATENTSCHRIFT

(11) **EP 2 294 160 B1**
(45) Veröffentlichungstag und Bekanntmachung des Hinweises auf die Patenterteilung: **17.10.2018**
(21) Anmeldenummer: 09757154.1
(22) Anmeldetag: 07.05.2009
(51) Int. Cl.: C09K 11/06, H01L 51/50, C07F 11/00, C07F 15/00, C07D 471/22, C07D 513/22, C07F 1/00, C09B 57/10

(54) **ELEKTRONISCHE VORRICHTUNG ENTHALTEND METALLKOMPLEXE**
ELECTRONIC DEVICE COMPRISING METAL COMPLEXES
DISPOSITIF ÉLECTRONIQUE CONTENANT DES COMPLEXES MÉTALLIQUES

(30) Priorität: 05.06.2008 DE 102008027005
(43) Veröffentlichungstag der Anmeldung: 16.03.2011
(73) Patentinhaber: Merck Patent GmbH, 64293 Darmstadt (DE)
(72) Erfinder: STOESSEL, Philipp, 60487 Frankfurt am Main (DE); BREUNING, Esther, 64372 Ober-Ramstadt (DE)
(86) Internationale Anmeldenummer: PCT/EP2009/003277
(87) Internationale Veröffentlichungsnummer: WO 2009/146770

(56) Entgegenhaltungen:
- Y. SUZUKI ET.AL.: "Preparation of N-(p-tolyl)azacalix[n](2,6)pyridines constructed of various numbers of the recurring unit" SYNLETT, Bd. 2, 2005, Seiten 263-266, XP002540273
- I. DESPOTOVIC ET.AL.: "Derivatives of azacalix[3](2,6)pyridine are strong neutral organic superbases: a DFT study" ORGANIC LETTERS, Bd. 9, Nr. 6, 2007, Seiten 1101-1104, XP002540274
- T. KANBARA ET.AL.: "New proton-sponge-like macrocyclic compound: synergistic hydrogen bonds of aminopyridine" EUR. J. ORG. CHEM., 2006, Seiten 3314-3316, XP002540275
- I. DESPOTOVIC ET.AL.: "Hyperstrong neutral organic bases: phosphazeno azacalix[3](2,6)pyridines" ORGANIC LETTERS, Bd. 9, Nr. 23, 2007, Seiten 4709-4712, XP002540276
- X.-Q. WEI ET.AL.: "Synthesis of novel light emitting calix[4]arene derivatives and their luminescent properties" MATERIALS CHEMISTRY AND PHYSICS, Bd. 102, 2007, Seiten 214-218, XP002540277
- C. LEGNANI ET.AL.: "Tunable blue organic light emitting diode based on aluminium calixarene supramolecular complex" APPLIED PHYSICS LETTERS, Bd. 85, Nr. 1, 2004, Seiten 10-12, XP002540278
- YUHEI TSUKAHARA ET AL: "A Thiacalix[3]pyridine Copper(I) Complex as a Highly Active Catalyst for the Olefin Aziridination Reaction", CHEMISTRY LETTERS, vol. 37, no. 4, 5 April 2008 (2008-04-05), pages 452-453, XP055315929, JAPAN ISSN: 0366-7022, DOI: 10.1246/cl.2008.452
- GEORGE R. NEWKOME ET AL: "Chemistry of heterocyclic compounds. 136. 1,3,5-Tri[2,6]pyridacyclohexaphane-2,4,6-t rione ketals: synthesis, structural analysis, and complexation", THE JOURNAL OF ORGANIC CHEMISTRY, vol. 55, no. 22, 1 October 1990 (1990-10-01), pages 5714-5719, XP055315939, US ISSN: 0022-3263, DOI: 10.1021/jo00309a015

## Beschreibung

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, jedoch immer noch Verbesserungsbedarf, insbesondere im Hinblick auf die Stabilität der Metallkomplexe, Effizienz, Betriebsspannung und Lebensdauer. Hier sind daher weitere Verbesserungen wünschenswert. Auch bei anderen in organischen Elektrolumineszenzvorrichtungen verwendeten Verbindungen, wie beispielsweise Matrixmaterialien und Ladungstransportmaterialien, gibt es noch Verbesserungsbedarf.

Gemäß dem Stand der Technik werden in phosphoreszierenden OLEDs als Triplettemitter meist Iridiumkomplexe eingesetzt. Eine Verbesserung dieser OLEDs konnte dadurch erzielt werden, dass Metallkomplexe mit polypodalem Liganden bzw. Kryptate eingesetzt wurden, wodurch die Komplexe eine höhere thermische Stabilität aufweisen, was zu einer höheren Lebensdauer der OLEDs führt (WO 04/081017, WO 05/113563, WO 06/008069). Jedoch sind noch weitere Verbesserungen der Komplexe wünschenswert, um diese in hochwertigen und langlebigen Elektrolumineszenzvorrichtungen, beispielsweise für Fernseher oder Computermonitore, einsetzen zu können.

Auch in anderen Funktionen in organischen Elektrolumineszenzvorrichtungen werden Metallkomplexe eingesetzt, beispielsweise Alq₃ (Tris(hydroxychinolinat)aluminium) als Elektronentransportmaterial oder BAlq (z. B. T. Tsuji et al., Journal of the Society of Information Display 2005, 13(2), 117-122) als Triplettmatrixmaterial oder als Lochblockiermaterial. Auch bei diesen Materialien sind noch weitere Verbesserungen erforderlich für deren Anwendung in hochwertigen Elektrolumineszenzvorrichtungen.

Aufgabe der vorliegenden Erfindung ist daher die Bereitstellung neuer organischer Elektrolumineszenzvorrichtungen enthaltend Metallkomplexe. Dabei können die Metallkomplexe, insbesondere abhängig vom verwendeten Metall, als Emitter, als Matrixmaterialien, als Lochblockiermaterialien, als Elektronentransportmaterialien oder auch in anderen Funktionen in der OLED eingesetzt werden. Insbesondere Verbesserungsbedarf besteht noch bei rot, grün und blau phosphoreszierenden Metallkomplexen.

Überraschend wurde gefunden, dass bestimmte organische Elektrolumineszenzvorrichtungen enthaltend die unten näher beschriebenen Metallchelatkomplexe diese Aufgabe lösen und zu deutlichen Verbesserungen der organischen Elektrolumineszenzvorrichtung führen, insbesondere hinsichtlich der Lebensdauer, der Effizienz und der Stabilität gegenüber Temperaturbelastung. Dies gilt insbesondere für grün und blau phosphoreszierende Elektrolumineszenzvorrichtungen. Organische Elektrolumineszenzvorrichtungen, welche diese Komplexe enthalten, sind daher der Gegenstand der vorliegenden Erfindung. Ein weiterer Gegenstand der vorliegenden Erfindung sind besonders geeignete Metallkomplexe, die in organischen Elektrolumineszenzvorrichtungen Anwendung finden können.

Stand der Technik sind organische Elektrolumineszenzvorrichtungen, die Metallkomplexe mit tridentaten oder höher dentaten Liganden enthalten (WO 04/108857), wobei der Ligand eine lineare Kettenstruktur darstellt. Aus dieser Offenbarung geht jedoch nicht hervor, dass es möglich ist, einen tridentaten Liganden in Form eines Makrocyclus zu verwenden, und dass dies Vorteile bezüglich der Anwendung des Komplexes haben könnte.

Spezielle Metallkomplexe mit tridentatem Liganden sind weiterhin aus der US 2008/0067925 bekannt. Darin sind drei koordinierende Aryl- bzw. Heteroarylgruppen mit zwei bivalenten Bindungsgruppen zu einem tridentaten linearen Liganden verknüpft, welcher an ein Metall der Gruppe 8 bis 10 koordiniert, insbesondere an Iridium oder Platin. An Platin binden diese Liganden zusammen mit einem monodentaten Liganden unter Bildung eines quadratisch-planaren Komplexes, bei dem die koordinierenden Atome der Liganden sowie das Metallatom in einer Ebene liegen. Aus dieser Offenbarung geht jedoch nicht hervor, dass es möglich ist, diese tridentaten Liganden auch in Form eines Makrocyclus zu verwenden, und dass dies Vorteile bezüglich der Anwendung des Komplexes haben könnte. Ein entsprechender makrocyclischer Ligand führt insbesondere zu einer anderen Koordinationsgeometrie am Metall, so dass keine quadratisch-planare Koordination mehr möglich ist und dass die Koordination in oktaedrischen Komplexen nur facial erfolgen kann.

Metallkomplexe mit tridentaten, makrocyclischen Liganden sind generell bekannt (z. B. WO 07/079585, EP 1531193). In diesen Anmeldungen ist jedoch nur die Verwendung dieser Metallkomplexe als katalytisch wirksame Aktivatoren für anorganische Persauerstoffverbindungen in Reinigungslösungen für harte Oberflächen und zur Entfernung von Sauerstoff in Wasser enthaltenden Systemen beschrieben. Eine Eignung der Metallkomplexe für organische elektronische Vorrichtungen, insbesondere für organische Elektrolumineszenzvorrichtungen geht aus diesen Anmeldungen nicht hervor.

X.-Q. Wei et al. (Materials Chemistry and Physics 2007, 102, 214-218) offenbaren Calix[4]arenderivate, an welche über eine nicht-konjugierte Gruppe eine Phenanthrolin- bzw. Bipyridingruppe gebunden ist, welche als Ligand für Lanthanidkomplexe dient. Das Calix[4]aren dient dabei als Anknüpfungspunkt für den Liganden zur Verbesserung der Löslichkeit und der hydrophoben Eigenschaften, koordiniert jedoch nicht direkt an das Metall.

Y. Suzuki et.al. (Synlettt, Bd. 2, 2005, Seiten 263-266) offenbaren N-(p-Tolyl)azacalixpyridine und deren Cu(I)-Komplexe. Der offenbarte Ligand ist vom Umfang des Anspruchs 13 mittels Disclaimers ausgenommen.

George R. Newkome et.al (The Journal of Organic Chemistry, Bd. 55, Nr. 22, 1. Oktober 1990 (1990-10-01), Seiten 5714-5719) offenbaren Calix[3]pyridine und einen entsprechenden Cu-Komplex, der vom Umfang des Anspruchs 11 mittels Disclaimer ausgenommen ist.

Gegenstand der Erfindung sind somit elektronische Vorrichtungen, enthaltend mindestens einen Metallkomplex der folgenden Formel (3), 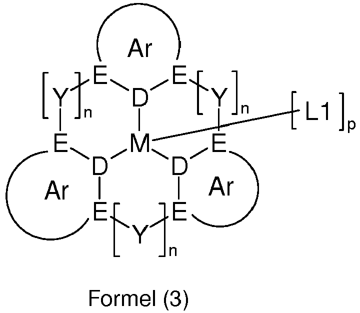 wobei für die verwendeten Symbole und Indizes gilt:
- M: ist ein Übergangsmetall, ein Lanthanid, ein Alkalimetall, ein Erdalkalimetall oder ein Hauptgruppenmetall der dritten oder vierten Hauptgruppe;
- D: ist bei jedem Auftreten gleich oder verschieden C, N, P, C-O⁻, C-S⁻, C-NR₂ oder C-PR₂, wobei die letzten vier genannten Gruppen über O, S, N bzw. P als exocyclische Donoratome an das Metall binden, oder C-N=C, wobei diese Gruppe über den Kohlenstoff der exocyclischen Isonitrilgruppe an das Metall bindet;
- E: ist bei jedem Auftreten gleich oder verschieden C oder N;
- Ar: ist bei jedem Auftreten gleich oder verschieden eine Gruppe, die zusammen mit der Gruppe E-D-E eine Aryl- oder Heteroarylgruppe mit 5 bis 40 aromatischen Ringatomen aufspannt und die durch einen oder mehrere Reste R substituiert sein kann; oder Ar ist, falls D für ein Carben-Kohlenstoffatom steht, eine Gruppe, die zusammen mit der Gruppe E-D-E eine cyclische gesättigte Gruppe mit 5 bis 10 Ringatomen darstellt;
- Y: ist gleich oder verschieden bei jedem Auftreten BR¹, B(R¹)₂⁻, C(R¹)⁻, C(R¹)₂, Si(R¹)⁻, Si(R¹)₂, C(=O), C(=NR), N⁻, NR¹, N(R¹)₂⁺, PR¹, P(R¹)₂⁺, AsR¹, As(R¹)₂⁺, P(=O)R¹, As(=O)R¹, P(=S)R¹, As(=S)R¹, O, S, S(R¹)⁺, Se, Te, S(=O), S(=O)₂, Se(=O), Se(=O)₂, Te(=O) oder Te(=O)₂;
- R: ist bei jedem Auftreten gleich oder verschieden H, Deuterium, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination dieser Systeme; dabei können zwei oder mehrere dieser Substituenten R auch miteinander ein mono- oder polycyclisches, aliphatisches, aromatisches und/oder benzoannelliertes Ringsystem bilden;
- R¹: ist gleich oder verschieden bei jedem Auftreten R oder eine Gruppe L2;
- R²: ist bei jedem Auftreten gleich oder verschieden H, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches oder aromatisches Ringsystem bilden;
- L2: ist bei jedem Auftreten gleich oder verschieden eine Donorgruppe mit 1 bis 40 C-Atomen, die eine weitere Bindung bzw. Koordination an das Metall M eingehen kann und die durch einen oder mehrere Reste R substituiert sein kann;
- n: ist bei jedem Auftreten gleich oder verschieden 0, 1, 2, 3, 4, 5 oder 6, wobei n = 0 bedeutet, dass die Gruppe Y nicht vorhanden ist und eine Einfachbindung zwischen den beiden Gruppen L vorliegt, mit der Maßgabe, dass nicht alle Indizes n gleichzeitig für 0 stehen können;
- L1: ist bei jedem Auftreten gleich oder verschieden ein mono-, bi-, tri-, tetra-, penta- oder hexadentater Ligand, der an das Metall M bindet;
- p: ist 0, 1, 2, 3, 4, 5, 6, 7, 8 oder 9.

Dabei wird der Index p so gewählt, dass die Koordinationszahl am Metall M insgesamt, je nach Metall, der für dieses Metall üblichen Koordinationszahl entspricht. Dies ist für Hauptgruppen- und Übergangsmetalle je nach Metall üblicherweise die Koordinationszahl 4, 5 oder 6, d. h. für Hauptgruppen- und Übergangsmetalle ist der Index p üblicherweise 1, 2 oder 3, falls diese Koordinationsstellen nicht durch weitere an den Liganden Ar gebundene Donorgruppen abgesättigt werden. Insbesondere für Lanthanide sind auch Koordinationszahlen bis zu 12 bekannt. Es ist generell bekannt, dass Metallkoordinationsverbindungen abhängig vom Metall und von der Oxidationsstufe des Metalls unterschiedliche Koordinationszahlen aufweisen, also eine unterschiedliche Anzahl von Liganden binden. Da die bevorzugten Koordinationszahlen von Metallen bzw. Metallionen in verschiedenen Oxidationsstufen zum allgemeinen Fachwissen des Fachmanns auf dem Gebiet der metallorganischen Chemie bzw. der Koordinationschemie gehören, ist es für den Fachmann ein Leichtes, je nach Metall und dessen Oxidationsstufe und je nach genauer Struktur des Liganden eine geeignete Anzahl weiterer Liganden L1 zu verwenden und somit den Index p geeignet zu wählen.

Das Metall M in Verbindungen der Formel (3) ist bevorzugt ein Übergangsmetall, ein Alkalimetall, ein Erdalkalimetall, ein Hauptgruppenmetall der 3. oder 4. Hauptgruppe oder ein Lanthanid.

Unter einer elektronischen Vorrichtung wird eine elektronische Vorrichtung verstanden, welche Anode, Kathode und mindestens eine Schicht enthält, wobei diese Schicht mindestens eine organische oder metallorganische Verbindung bzw. Metallkoordinationsverbindung enthält. Die erfindungsgemäße organische elektronische Vorrichtung enthält also Anode, Kathode und mindestens eine Schicht, welche mindestens eine Verbindung der oben aufgeführten Formel (3) enthält. Dabei sind bevorzugte organische elektronische Vorrichtungen ausgewählt aus der Gruppe bestehend aus organischen Elektrolumineszenzvorrichtungen (= organischen Leuchtdioden, OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung gemäß der oben aufgeführten Formel (3). Besonders bevorzugt sind organische Elektrolumineszenzvorrichtungen.

Unter einem Donoratom im Sinne der vorliegenden Erfindung wird ein Atom verstanden, das mindestens ein freies Elektronenpaar aufweist und dadurch in der Lage ist, an ein Metallatom bzw. Metallion zu binden. Dabei kann das Donoratom neutral oder negativ oder positiv geladen sein. Bevorzugt ist das Donoratom neutral oder negativ geladen. Beispiele für neutrale Donoratome sind Stickstoff, welcher in einem Heteroaromaten wie z. B. Pyridin gebunden ist, oder Kohlenstoff in Form eines Carbens. Beispiele für anionische Donoratome sind Kohlenstoff, welcher Teil eines Aromaten oder Heteroaromaten ist, wie z. B. ein Kohlenstoffatom in einer Phenylgruppe, oder Stickstoff, welcher Teil einer Fünfring-heteroaromatischen Gruppe ist, wie z. B. Stickstoff im Pyrrol, welches über den Stickstoff bindet. Unter einem exocyclischen Donoratom im Sinne dieser Erfindung wird ein Donoratom verstanden, welches nicht Teil der Gruppe Ar ist, sondern welches als Substituent an Ar gebunden ist und welches mindestens ein freies Elektronenpaar aufweist und dadurch in der Lage ist, an ein Metallatom zu binden. Beispiele für exocyclische Donoratome sind Sauerstoff in Form eines Phenolats, Schwefel in Form eines Thiolats, Stickstoff in Form eines Nitrils, Amins, Imins, Amids oder Imids, Phosphor in Form eines Phosphins oder Phosphits oder Kohlenstoff in Form eines Isonitrils oder Acetylids.

Bei dem Liganden des Metallkomplexes der Formel (3) handelt es sich um einen mindestens tridentaten, makrocyclischen Liganden, welcher über die drei Gruppen L an das Metall M bindet. Unter einem Makrocyclus im Sinne dieser Erfindung wird ein Cyclus verstanden, welcher mindestens 10 Ringatome aufweist. Dabei sei betont, dass der Ligand auch mehr als drei Koordinationsstellen aufweisen kann und beispielsweise tetradentat, pentadentat oder hexadentat sein kann, z. B. falls an die Gruppen Y Substituenten gebunden sind, die ebenfalls an das Metall M binden können, wie unten noch genauer ausgeführt wird. Auch wenn der Komplex der Formel (3) planar gezeichnet ist, ist diese Struktur nicht notwendigerweise planar. Vielmehr nimmt der Ligand analog zu Calixarenen typischerweise eine Kelch-artige Konformation ein, bei der die Donoratome zur geschlossenen Seite des Kelchs deuten und so in einer Konformation vorliegen, welche geeignet ist, an ein Metall zu binden. Am Metall sind somit weitere Koordinationsstellen sterisch zugänglich, an denen weitere Liganden, wie in der Struktur der Formel (3) durch L1 beschrieben, binden können. So sind beispielsweise tetraedrische Komplexe möglich, ebenso oktaedrische Komplexe, in denen der makrocyclische Ligand facial gebunden ist. Genauso können in Verbindungen der Formel (3) je nach Struktur auch Substituenten, die an die Gruppen Y gebunden sind, an das Metall M binden. Die Konformation der Verbindungen gemäß Formel (3) ist im Folgenden schematisch dargestellt, wobei D allgemein für ein Donoratom steht, welches an das Metall koordiniert:

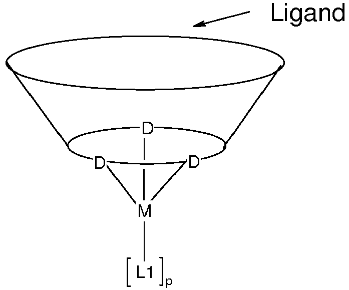

Im Folgenden werden die Ausführungsformen der Verbindung gemäß Formel (3) beschrieben, wie sie bevorzugt in der organischen elektronischen Vorrichtung verwendet wird.

Bevorzugt sind Verbindungen gemäß Formel (3), dadurch gekennzeichnet, dass diese nicht geladen, d. h. elektrisch neutral, sind. Dies wird auf einfache Weise dadurch erreicht, dass die Ladung der Gruppen L und der verbrückenden Einheiten Y und gegebenenfalls vorhandener Liganden L1 so gewählt wird, dass sie die Ladung des komplexierten Metallatoms M kompensieren.

Bevorzugt sind weiterhin Verbindungen gemäß Formel (3), dadurch gekennzeichnet, dass die Summe der Valenzelektronen um das Metallatom 18 beträgt. Diese Bevorzugung ist durch die besondere Stabilität dieser Metallkomplexe begründet (siehe z. B. Elschenbroich, Salzer, Organometallchemie, Teubner Studienbücher, Stuttgart 1993**).**

Dabei sei an dieser Stelle explizit betont, dass die Reste R¹, welche in derselben Gruppe Y gebunden sind, auch miteinander ein Ringsystem bilden können. So können auch zwei Reste R¹, welche an dasselbe Kohlenstoffatom gebunden sind, beispielsweise wenn die Gruppe Y für C(R¹)₂ steht, miteinander ein Ringsystem bilden und so zu Spiro-Strukturen führen. Beispiele für mögliche Ringsysteme sind hier Fluorenartige Gruppen, wenn beide Gruppen R¹ für Phenylgruppen stehen, die miteinander ein Ringsystem bilden, oder 1,3-Dioxolane, wenn beide Gruppen R¹ für Alkoxygruppen stehen, die miteinander ein Ringsystem bilden.

Ebenso sei an dieser Stelle explizit betont, dass auch Reste R¹, welche an der Gruppe Y gebunden sind, mit Resten R, welche an der Gruppe L2 gebunden sind, ein Ringsystem bilden können.

Steht das Symbol D für Kohlenstoff, so weist dieser, je nach Ausführungsform, formal eine negative Ladung auf, d. h. der entsprechende freie Ligand ohne das Metall M würde an dieser Stelle eine Gruppe C-H enthalten, oder er steht für ein neutrales Carben-Kohlenstoffatom. Steht das Symbol D für Stickstoff, so ist dieser, je nach Ausführungsform, entweder ein neutrales Donoratom oder weist formal eine negative Ladung auf, d. h. der entsprechende freie Ligand ohne das Metall M würde an dieser Stelle eine Gruppe N-H enthalten. Steht das Symbol D für Phosphor, so ist dies ein neutrales Donoratom.

Unter einer Donorgruppe, wie für L2 definiert, wird im Sinne dieser Erfindung ein Substituent bzw. eine chemische Gruppe verstanden, die mindestens ein Donoratom aufweist, welches in der Lage ist, an das Metall M zu binden.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden. Ein cyclisches Carben im Sinne dieser Erfindung ist eine cyclische Gruppe, welche über ein neutrales C-Atom an das Metall bindet. Dabei kann die cyclische Gruppe gesättigt oder ungesättigt sein. Bevorzugt sind hierbei Arduengo-Carbene, also solche Carben, bei welchen an das Carben-C-Atom zwei Stickstoffatome gebunden sind. Dabei wird ein Fünfring-Arduengo-Carben bzw. ein anderes ungesättigtes Fünfring-Carben ebenfalls als eine Arylgruppe im Sinne dieser Erfindung angesehen.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, tert-Penyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Trifluormethyl, Pentafluorethyl oder 2,2,2-Trifluorethyl verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl oder Cyclooctenyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind Verbindungen gemäß Formel (3), in denen M für ein Übergangsmetall, insbesondere für ein tetrakoordiniertes, ein pentakoordiniertes oder ein hexakoordiniertes Übergangsmetall steht, besonders bevorzugt ausgewählt aus der Gruppe bestehend aus Chrom, Molybdän, Wolfram, Rhenium, Ruthenium, Osmium, Rhodium, Iridium, Nickel, Palladium, Platin, Kupfer, Silber und Gold, insbesondere Molybdän, Wolfram, Rhenium, Ruthenium, Osmium, Iridium, Platin und Gold. Ganz besonders bevorzugt sind Iridium und Platin. Die Metalle können dabei in verschiedenen Oxidationsstufen vorliegen. Bevorzugt sind dabei die oben genannten Metalle in den Oxidationsstufen Cr(0), Cr(II), Cr(III), Cr(IV), Cr(VI), Mo(0), Mo(II), Mo(III), Mo(IV), Mo(VI) W(0), W(II), W(III), W(IV), W(VI), Re(I), Re(II), Re(III), Re(IV), Ru(II), Ru(III), Os(II), Os(III), Os(IV), Rh(I), Rh(III), Ir(I), Ir(III), Ir(IV), Ni(0), Ni(II), Ni(IV), Pd(II), Pt(II), Pt(IV), Cu(I), Cu(II), Cu(III), Ag(I), Ag(II), Au(I), Au(III) und Au(V); ganz besonders bevorzugt sind Mo(0), W(0), Re(I), Ru(II), Os(II), Rh(III), Ir(III) und Pt(II).

Bevorzugt sind weiterhin Verbindungen der Formel (3), in denen M für ein Hauptgruppenmetall, ausgewählt aus der Gruppe bestehend aus Lithium, Natrium, Magnesium, Aluminium, Gallium, Indium oder Zinn, oder für Scanium, Yttrium oder Lanthan steht. Insbesondere bevorzugt sind Li(I), Na(I), Mg(II), Al(III), Ga(III), In(III), Sc(III), Y(III) oder La(III), ganz besonders bevorzugt Al(III).

In einer bevorzugten Ausführungsform der Erfindung steht der Index n bei jedem Auftreten gleich oder verschieden für 0, 1, 2 oder 3, mit der Maßgabe, dass nicht alle Indizes n gleichzeitig für 0 stehen, besonders bevorzugt für 0, 1 oder 2, mit der Maßgabe, dass nicht alle Indizes n gleichzeitig für 0 stehen. In einer ganz besonders bevorzugten Ausführungsform der Erfindung steht der Index n bei jedem Auftreten gleich oder verschieden für 1 oder 2, insbesondere für 1.

In einer bevorzugten Ausführungsform der Erfindung steht Y gleich oder verschieden bei jedem Auftreten für C(R¹)₂, C(R¹)⁻, C(=O), NR¹, PR¹, P(=O)R¹, O oder S, besonders bevorzugt für C(R¹)₂, C(=O) oder NR¹.

In einer weiteren bevorzugten Ausführungsform der Erfindung steht eine Gruppe Y oder in einer besonders bevorzugten Ausführungsform zwei Gruppen Y oder in einer ganz besonders bevorzugten Ausführungsform alle drei Gruppen Y für BR¹, C(R¹)₂, Si(R¹)₂, NR¹, PR¹, AsR¹, P(=O)R¹, As(=O)R¹, P(=S)R¹ oder As(=S)R¹ und der Substituent R¹ bzw. einer der Substituenten R¹ im Falle von C(R¹)₂ und Si(R¹)₂ steht für eine Gruppe L2. Eine bevorzugte Ausführungsform der Erfindung sind also organische elektronische Vorrichtungen enthaltend die Verbindungen gemäß den folgenden Formeln (4), (5) und (6), 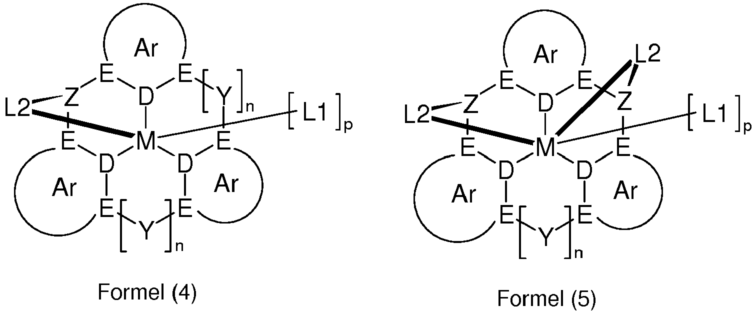 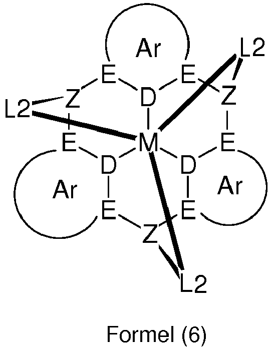 wobei Z gleich oder verschieden bei jedem Auftreten für B, B(R¹)⁻, C⁻, CR¹, SiR¹, N, P, As, P(=O), As(=O), P(=S) oder As(=S) steht, bevorzugt für CR¹, N, P oder P(=O)R¹, und die weiteren Symbole und Indizes die oben aufgeführten Bedeutungen haben.

Dabei sind die Verbindungen der Formel (5) und der Formel (6) spezielle Ausführungsformen der Verbindungen gemäß Formel (4). In Verbindungen der Formel (6) binden die drei aromatischen Gruppen über das Donoratom D facial an M und die drei Gruppen L2 binden facial an das Metall.

Eine bevorzugte Ausführungsform der Verbindungen gemäß Formel (6) sind die Verbindungen der Formel (6a), 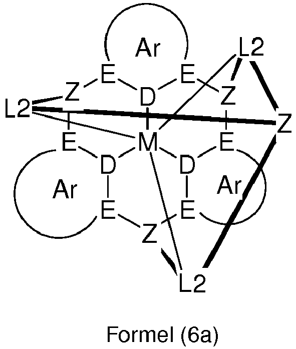 wobei die verwendeten Symbole dieselbe Bedeutung haben, wie oben beschrieben.

Dabei sei nochmals erwähnt, dass die Substituenten R an L2 mit den Substituenten R¹ an Z ein Ringsystem bilden können.

Auch wenn die Verbindungen der Formel (4), (5) und (6) flach dargestellt sind, nehmen auch diese typischerweise eine dreidimensionale Struktur analog zu den oben dargestellten Verbindungen der Formel (3) ein, wie im Folgenden schematisch für Verbindungen der Formel (6) dargestellt, wobei D allgemein für ein Donoratom steht:

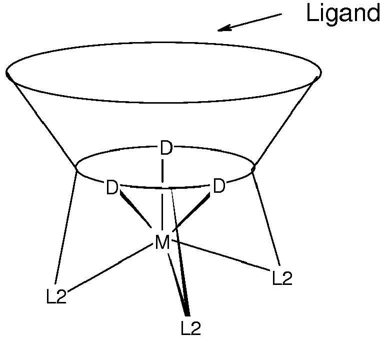

Wenn mehrere Substituenten R an unterschiedlichen Ligandengruppen L2 miteinander ein Ringsystem bilden, ist auch die Bildung von Kryptaten möglich, wie im Folgenden schematisch dargestellt, wobei V ganz allgemein für eine verbrückende Einheit steht, welche durch Ringschluss mehrerer Substituenten R entsteht:

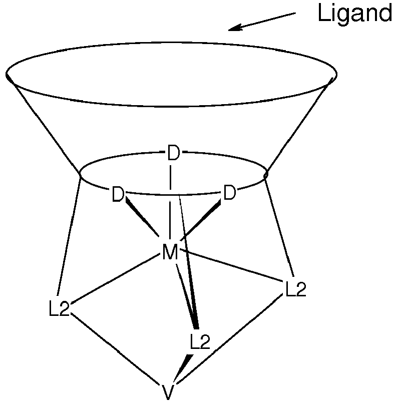

Wenn in der oben abgebildeten Struktur die verbrückende Gruppe V für eine Gruppe Z steht, erhält man Verbindungen der oben aufgeführten Formel (6a).

In einer weiteren bevorzugten Ausführungsform von Verbindungen gemäß Formel (3) ist der Index p = 1, 2 oder 3 und der Ligand L1 ist ein weiterer makrocyclischer Ligand, d. h. der Metallkomplex enthält zwei, drei oder vier der makrocyclischen Liganden. Diese bevorzugte Ausführungsform von Verbindungen gemäß Formel (1) wird durch Verbindungen der Formel (7) dargestellt: 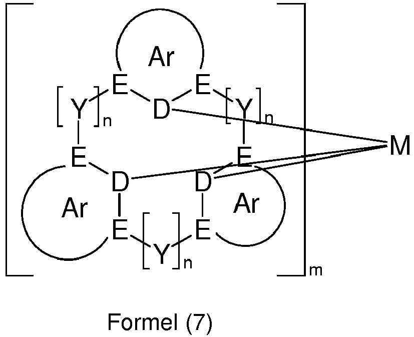 wobei die verwendeten Symbole und Indizes die oben genannten Bedeutungen haben und m, je nach dem verwendeten Metall, für 2, 3 oder 4 steht. Dabei steht m für Hauptgruppen- und Übergangsmetalle bevorzugt für 2; für Lanthanide kann m auch für 3 oder 4 stehen. Weiterhin können in diesen Komplexen auch zwei oder mehrere der makrocyclischen Liganden über eine Brücke verknüpft sein, indem zwei oder mehrere Gruppen R bzw. R¹ miteinander verknüpft sind.

Auch die Verbindungen der Formel (7) nehmen typischerweise eine dreidimensionale Struktur analog zu den oben dargestellten Verbindungen der Formel (3) ein, wie im Folgenden schematisch für m = 2 dargestellt, wobei D allgemein für ein Donoratom steht:

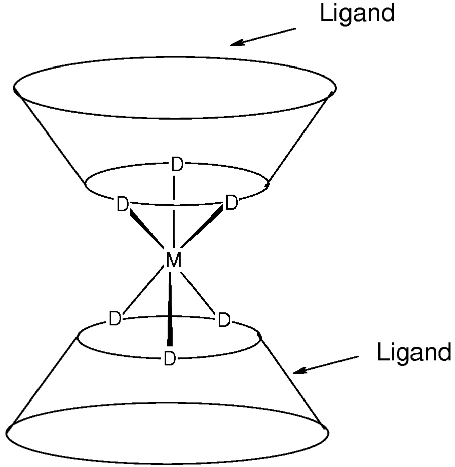

In einer bevorzugten Ausführungsform von Verbindungen gemäß Formel (3) bis (7) steht das Symbol D gleich oder verschieden bei jedem Auftreten für C oder N. Dabei ist der Kohlenstoff entweder formal negativ geladen, d. h. er würde im Liganden ohne das Metall eine negative Ladung aufweisen, oder er ist neutral und es handelt sich um ein Carben-Kohlenstoffatom. Wenn das Symbol D für N oder ein formal negatives Kohlenstoffatom steht, stehen bevorzugt gleichzeitig beide Symbole E, welche an dieses D binden, für C. Wenn das Symbol D für ein Carben-Kohlenstoffatom steht, steht bevorzugt mindestens ein Symbol E, besonders bevorzugt beide Symbole E, welche an dieses D binden, für N, d. h. es handelt sich bevorzugt um Arduengo-Carbene. Diese Bevorzugung ist mit der besonderen Stabilität dieser Carbene begründet.

In einer weiteren bevorzugten Ausführungsform der Formel (3) bis (7) ist die Aryl- bzw. Heteroarylgruppe, die durch E-D-E zusammen mit Ar gebildet wird, eine Aryl- oder Heteroarylgruppe mit 5 bis 20 aromatischen Ringatomen, besonders bevorzugt mit 5 bis 14 aromatischen Ringatomen. Diese kann jeweils durch einen oder mehrere Reste R substituiert sein. Besonders bevorzugte Aryl- oder Heteroarylgruppen sind Benzol, Phenol, Thiophenol, Naphthalin, Anthracen, Phenanthren, Thiophen, Pyrrol, Furan, Pyridin, Pyrimidin, Pyrazin, Pyridazin, Triazin, Benzothiophen, Indol, Benzofuran, Chinolin, Isochinolin, Chinoxalin, Imidazol, Pyrazol, Benzimidazol, Oxazol, Thiazol, Benzoxazol oder Benzthiazol, die jeweils durch R substituiert sein können. Besonders bevorzugt sind weiterhin Arduengo-Carbene.

Insbesondere bevorzugt sind Verbindungen gemäß Formel (3) bis (7), in denen die Aryl- oder Heteroarylgruppe, die durch Ar zusammen mit der Gruppe E-D-E aufgespannt wird, für eine Gruppe der folgenden Formel (8) bis (20) steht, wobei jeweils durch die gestrichelte Bindung die Bindung dieser Gruppe im Liganden angedeutet wird, also die Bindung zu den Gruppen Y, und wobei * jeweils die Position der Koordination an das Metall M kennzeichnet:

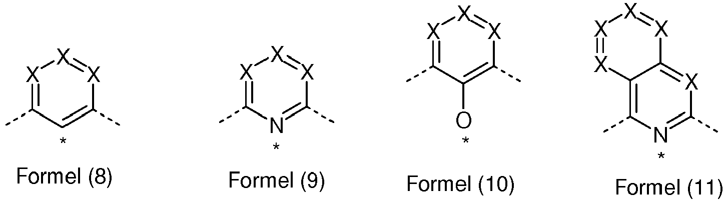

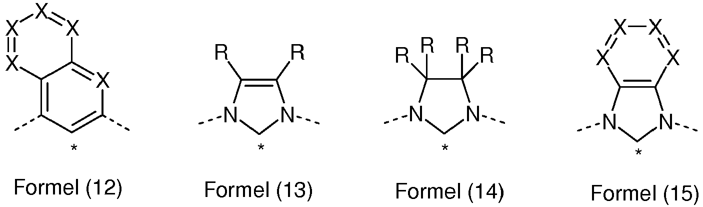

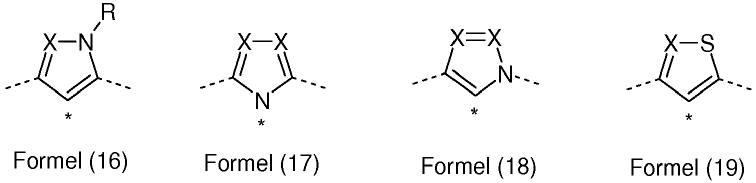

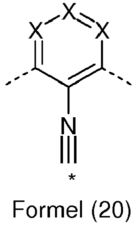

Dabei haben die verwendeten Symbole dieselbe Bedeutung, wie oben beschrieben, und X steht gleich oder verschieden bei jedem Auftreten für CR oder N mit der Maßgabe, dass maximal drei Symbole X in jeder Gruppe für N stehen. Bevorzugt stehen maximal zwei Symbole X in jeder Gruppe für N, besonders bevorzugt steht maximal ein Symbol X in jeder Gruppe für N, ganz besonders bevorzugt stehen alle Symbole X für CR.

In einer weiteren bevorzugten Ausführungsform der Erfindung enthält die Verbindung gemäß Formel (3) bis (7) mindestens eine direkte Metall-Kohlenstoff-Bindung, bevorzugt mindestens zwei direkte Metall-Kohlenstoff-Bindungen, besonders bevorzugt drei direkte Metall-Kohlenstoff-Bindungen. Dies können Bindungen des Donoratoms D, wenn dieses gleich Kohlenstoff ist, an das Metall in Verbindungen gemäß Formel (3) bis (7). Dies können aber auch Bindungen der Gruppe L2 an das Metall sein in Verbindungen gemäß Formel (4) bis (6).

In einer weiteren bevorzugten Ausführungsform der Erfindung ist die Gruppe L2 gleich oder verschieden bei jedem Auftreten eine Aryl- oder Heteroarylgruppe mit 5 bis 40 aromatischen Ringatomen, welche durch einen oder mehrere Reste R substituiert sein kann und/oder welche auch ein exocyclisches Donoratom aufweisen kann, oder eine neutrale oder anionische Donorgruppe, welche über Sauerstoff, Stickstoff, Phosphor oder Schwefel an das Metall bindet und welche durch einen oder mehrere Reste R substituiert sein kann. Dabei binden die Aryl- bzw. Heteroarylgruppen bevorzugt über die Position ortho zur Verknüpfung an Z an das Metall. Bevorzugte Aryl- bzw. Heteroarylgruppen sind Benzol, 2-Phenol, 2-Thiophenol, Naphthalin, Anthracen, Phenanthren, Pyridin, Chinolin, Isochinolin, Pyrazin, Chinoxalin, Pyrimidin, Pyridazin, Triazin, Pyrrol, Indol, Imidazol, Furan, Benzofuran, Benzimidazol, Pyrazol, Triazol, Oxazol, Thiazol, Thiophen, Benzothiophen, Benzoxazol oder Benzthiazol, welche jeweils durch einen oder mehrere Reste R substituiert sein kann. Dabei erfolgt in diesen Gruppen die Bindung an die Gruppe Z des Liganden und die Bindung an das Metall bevorzugt über zwei direkt benachbarte Atome in diesen Gruppen, also über ortho-Positionen des Benzols, etc. Je nach Gruppe sind die oben genannten Gruppen neutral koordinierende Gruppen, beispielsweise Pyridin, welches über neutrales N-Atom bindet, oder es sind anionisch koordinerende Gruppen, beispielsweise Benzol, Thiophen und Phenol, welche über ein negativ geladenes C-Atom bzw. O-Atom binden. Weitere bevorzugte Gruppen L2 sind ungesättigte oder gesättigte cyclische Arduengo-Carbene, insbesondere ungesättigte cyclische Arduengo-Carbene, welche jeweils mit einem oder mehreren Resten R substituiert sein können, und Alkene oder Imine, welche jeweils mit einem oder mehreren Resten R substituiert sein können.

Wenn die Gruppe L2 eine Aryl- oder Heteroarylgruppe bzw. ein Alken oder Imin darstellt, so ist diese besonders bevorzugt gewählt aus Gruppen der Formel (21) bis (49), wobei jeweils durch die gestrichelte Bindung die Bindung dieser Gruppe im Liganden angedeutet wird, also die Bindung zu der Gruppe Z, wobei * jeweils die Position der Koordination an das Metall M kennzeichnet und wobei die verwendeten Symbole die oben aufgeführten Bedeutungen haben:

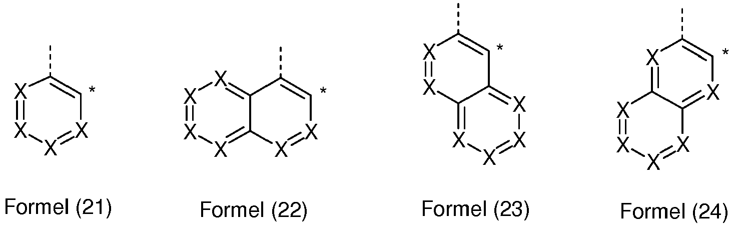

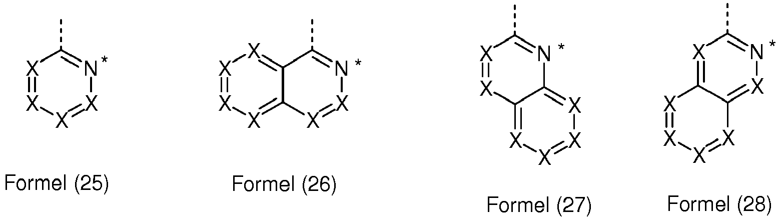

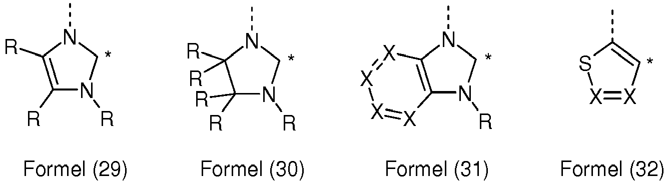

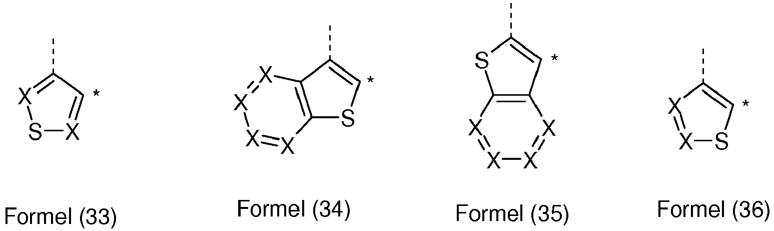

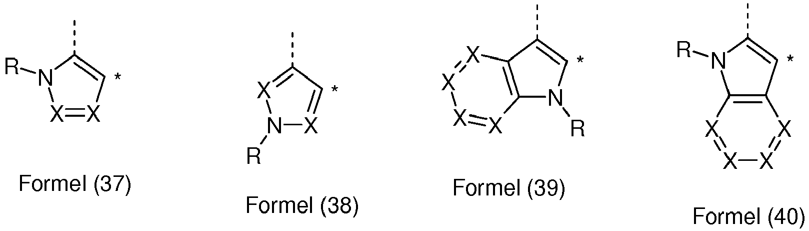

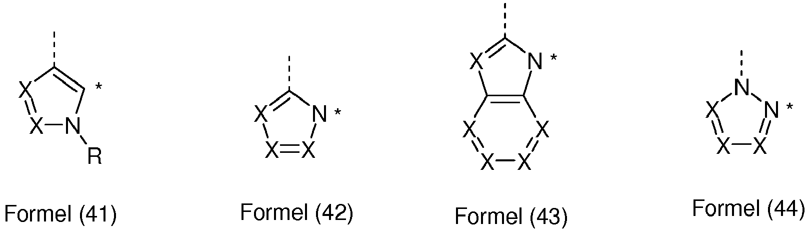

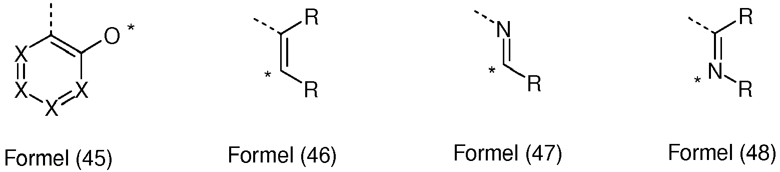

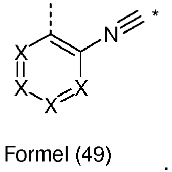

Besonders bevorzugte Ausführungsformen der Erfindung sind Verbindungen, in denen die Gruppen (8) bis (20) mit den Gruppen (21) bis (49) kombiniert werden.

Weiterhin bevorzugt kann L2 eine neutrale oder anionische Donorgruppe darstellen, wobei es sich bevorzugt um eine monodentate oder um eine bidentate chelatisierende Gruppe, besonders bevorzugt um eine monodentate Gruppe, handelt. Dabei sind die Donoratome bevorzugt Kohlenstoff, Sauerstoff, Stickstoff, Phosphor oder Schwefel, besonders bevorzugt Stickstoff oder Sauerstoff.

Bevorzugte Kohlenstoff-haltige Donorgruppen sind Acetylide und aliphatische bzw. aromatische Isonitrile.

Bevorzugte Stickstoff-haltige Donorgruppen sind außer den oben aufgeführten aromatischen Stickstoffheterocyclen aliphatische Amine, bevorzugt mit C₁-C₂₀-Alkylgruppen, besonders bevorzugt mit C₁-C₁₀-Alkylgruppen, ganz besonders bevorzugt mit C₁-C₄-Alkylgruppen, aliphatische cyclische Amine, beispielsweise Pyrrolidin, Piperidin oder Morpholin, Nitrile, Amide, Imide und Imine, die jeweils mit Gruppen R substituiert oder unsubstituiert sein können.

Bevorzugte Phosphor-haltige Donorgruppen sind PF₂, P(NR₂)₂, wobei R gleich oder verschieden bei jedem Auftreten für eine C₁-C₂₀-Alkylgruppe oder eine Aryl- bzw. Heteroarylgruppe im Sinne der oben gegebenen Definition steht, Alkyl-, Aryl- oder gemischte Alkylarylphosphine, Alkylhalogen-, Arylhalogen- oder gemischte Alkylarylhalogenphosphine, wobei das Halogen jeweils F, Cl, Br oder I sein kann, Alkyl-, Aryl- oder gemischte Alkylarylphosphite oder Phosphaaromaten, wie beispielsweise Phosphabenzol, die jeweils mit Gruppen R substituiert oder unsubstituiert sein können. Dabei sind die Alkylgruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte Sauerstoff-haltige Donorgruppen sind außer dem oben aufgeführten Phenol Alkohole, Alkoholate, offenkettige oder cyclische, aliphatische oder aromatische Ether, Sauerstoffheterocyclen, wie z. B. Furan, Aldehyde, Ketone, Phosphinoxidgruppen, Phosphate, Phosphonate, Borate, Silicate, Sulfoxidgruppen, Carboxylate, Phenole, Phenolate, Oxime, Hydroxamate, β-Ketoketonate, β-Ketoester und β-Diester, die jeweils mit Gruppen R substituiert oder unsubstituiert sein können, wobei die letztgenannten Gruppen zweizähnig chelatisierende Liganden darstellen. Dabei sind die Alkylgruppen dieser Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte Schwefel-haltige Donorgruppen sind außer den oben aufgeführten Schwefelheteroaromaten aliphatische oder aromatische Thiole und Thiolate, offenkettige oder cyclische Thioether, Thiocarbonylgruppen, Phosphinsulfide und Thiocarboxylate, die jeweils mit Gruppen R substituiert oder unsubstituiert sein können. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Aus diesen Donorgruppen lassen sich auch zweizähnig chelatisierende Gruppen bilden, indem zwei dieser Gruppen, die gleich oder verschieden sein können und die gleiche oder unterschiedliche Donoratome aufweisen können, kombiniert werden. Diese Gruppen können auch durch einen oder mehrere Reste R substituiert sein. Beispiele für derartige zweizähnig chelatisierende Gruppen sind substituierte oder unsubstituierte β-Ketoketonate, β-Ketoester, β-Diester, Carboxylate abgeleitet von Aminocarbonsäuren, wie z. B. Pyridin-2-carbonsäure, Chinolin-2-carbonsäure, Glycin, Dimethylglycin, Alanin oder Dimethylaminoalanin, Iminoacetoacetonate, Hydroxamate, Pyridylphosphine, α-Phosphinocarboxylate, Glycolether, Etheralkoholate, Dialkoholate abgeleitet von Dialkoholen, wie z. B. Ethylenglykol oder 1,3-Propylenglykol, Dithiolate abgeleitet von Dithiolen, wie z. B. 1,2-Ethylendithiol oder 1,3-Propylendithiol, Diamine, wie z. B. Ethylendiamin, Propylendiamin oder cis- oder trans-Diaminocyclohexan, Imine, wie z. B. 2[1-(Phenylimino)ethyl]pyridin, 2[1-(2-Methylphenyl-imino)ethyl]pyridin, 2[1-(2,6-Di-*iso*-propylphenylimino)ethyl]pyridin, 2[1-(Methylimino)ethyl]pyridin, 2[1-(ethylimino)ethyl]pyridin, 2[1-(*Iso*-Propyl-imino)ethyl]pyridin oder 2[1-(*Tert*-Butylimino)ethyl]pyridin, Diimine, wie z. B. 1,2-Bis(methylimino)ethan, 1,2-Bis(ethylimino)ethan, 1,2-Bis(iso-propylimino)ethan, 1,2-Bis(*tert*-butylimino)ethan, 2,3-Bis(methylimino)-butan, 2,3-Bis(ethylimino)butan, 2,3-Bis(*iso*-propylimino)butan, 2,3-Bis(*tert-*butylimino)butan, 1,2-Bis(phenylimino)ethan, 1,2-Bis(2-methylphenyl-imino)ethan, 1,2-Bis(2,6-di-iso-propylphenylimino)ethan, 1,2-Bis(2,6-di-*tert-*butylphenylimino)ethan, 2,3-Bis(phenylimino)butan, 2,3-Bis(2-methyl-phenylimino)butan, 2,3-Bis(2,6-di-*iso*-propylphenylimino)butan oder 2,3-Bis(2,6-di-*tert*-butylphenylimino)butan, Diphosphine, wie z. B. Bis-diphenylphosphinomethan, Bis-diphenylphosphinoethan, Bis(diphenyl-phosphino)propan, Bis(dimethylphosphino)methan, Bis(dimethyl-phosphino)ethan, Bis(dimethylphosphino)propan, Bis(diethylphosphino)-methan, Bis(diethylphosphino)ethan, Bis(diethylphosphino)propan, Bis(di-*tert*-butylphosphino)methan, Bis(di-*tert*-butylphosphino)ethan, Bis(*tert-*butylphosphino)propan, Salicyliminate abgeleitet von Salicyliminen, wie z. B. Methylsalicylimin, Ethylsalicylimin oder Phenylsalicylimin, etc.

Ganz analog lassen sich auch dreizähnig oder höherzähnig chelatisierende Gruppen bilden.

Die Liganden L1 sind bevorzugt neutrale, monoanionische, dianionische oder trianionische Liganden, besonders bevorzugt neutrale oder monoanionische Liganden. Sie sind bevorzugt monodentat, bidentat oder tridentat, weisen also eine, zwei oder drei Koordinationsstellen auf. Bevorzugte neutrale, monodentate Liganden L1 sind ausgewählt aus Kohlenmonoxid, Isonitrilen, wie z. B. *tert*-Butyl-isonitril, Cyclohexylisonitril, Adamantylisonitril, Phenylisonitril, Mesitylisonitril, 2,6-Dimethylphenylisonitril, 2,6-Di-iso-propylphenylisonitril, 2,6-Di-tert-butylphenylisonitril, Aminen, wie z. B. Trimethylamin, Triethylamin, Morpholin, Phosphinen, wie z. B. Trifluorphosphin, Trimethylphosphin, Tricyclohexylphosphin, Tri-*tert-*butylphosphin, Triphenylphosphin, Tris(pentafluorphenyl)phosphin, Phosphiten, wie z. B. Trimethylphosphit, Triethylphosphit, Arsinen, wie z. B. Trifluorarsin, Trimethylarsin, Tricyclohexylarsin, Tri-*tert*-butylarsin, Triphenylarsinin, Tris(pentafluorphenyl)arsin, Stibinen, wie z. B. Trifluorstibin, Trimethylstibin, Tricyclohexylstibin, Tri-*tert*-butylstibin, Triphenylstibin, Tris(pentafluorphenyl)stibin, und stickstoffhaltigen Heterocyclen, wie z. B. Pyridin, Pyridazin, Pyrazin, Pyrimidin, Triazin.

Bevorzugte monoanionische, monodentate Liganden L1 sind ausgewählt aus Hydrid, Deuterid, den Halogeniden F, Cl, Br und I, Alkylacetyliden, wie z. B. Methyl-C≡C⁻, tert-Butyl-C≡C⁻, Arylacetyliden, wie z. B. Phenyl-C≡C⁻, Cyanid, Cyanat, Isocyanat, Thiocyanat, Isothiocyanat, aliphatischen oder aromatischen Alkoholaten, wie z. B. Methanolat, Ethanolat, Propanolat, *iso*-Propanolat, *tert*-Butylat, Phenolat, aliphatischen oder aromatischen Thioalkoholaten, wie z. B. Methanthiolat, Ethanthiolat, Propanthiolat, *iso-*Propanthiolat, tert-Thiobutylat, Thiophenolat, Amiden, wie z. B. Dimethylamid, Diethylamid, Di-*iso*-propylamid, Morpholid, Carboxylaten, wie z. B. Acetat, Trifluoracetat, Propionat, Benzoat, und anionischen, stickstoffhaltigen Heterocyclen, wie Pyrrolid, Imidazolid, Pyrazolid. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte di- bzw. trianionische Liganden sind O²⁻, S²⁻, Nitrene, welche zu einer Koordination der Form R-N=M führen, wobei R allgemein für einen Substituenten steht, oder N³⁻.

Bevorzugte neutrale oder mono- oder dianionische bidentate oder höherdentate Liganden L1 sind ausgewählt aus Diaminen, wie z. B. Ethylendiamin, N,N,N',N'-Tetramethylethylendiamin, Propylendiamin, N,N,N',N'-Tetramethylpropylendiamin, cis- oder trans-Diaminocyclohexan, cis- oder trans-N,N,N',N'-Tetramethyldiaminocyclohexan, Iminen, wie z. B. 2[1-(Phenylimino)ethyl]pyridin, 2[1-(2-Methylphenylimino)ethyl]pyridin, 2[1-(2,6-Di-*iso*-propylphenylimino)ethyl]pyridin, 2[1-(Methylimino)ethyl]pyridin, 2[1-(ethylimino)ethyl]pyridin, 2[1-(*Iso*-Propylimino)ethyl]pyridin, 2[1*-(Tert-*Butylimino)ethyl]pyridin, Diminen, wie z. B. 1,2-Bis(methylimino)ethan, 1,2-Bis(ethylimino)ethan, 1,2-Bis(*iso*-propylimino)ethan, 1,2-Bis(*tert*-butyl-imino)ethan, 2,3-Bis(methylimino)butan, 2,3-Bis(ethylimino)butan, 2,3-Bis-(*iso*-propylimino)butan, 2,3-Bis(*tert*-butylimino)butan, 1,2-Bis(phenylimino)-ethan, 1,2-Bis(2-methylphenylimino)ethan, 1,2-Bis(2,6-di-*iso*-propylphenyl-imino)ethan, 1,2-Bis(2,6-di-*tert*-butylphenylimino)ethan, 2,3-Bis(phenyl-imino)butan, 2,3-Bis(2-methylphenylimino)butan, 2,3-Bis(2,6-di-*iso*-propyl-phenylimino)butan, 2,3-Bis(2,6-di-*tert*-butylphenylimino)butan, Heterocyclen enthaltend zwei Stickstoffatome, wie z. B. 2,2'-Bipyridin, o-Phenanthrolin, Diphosphinen, wie z. B. Bis-diphenylphosphinomethan, Bis-diphenylphosphinoethan, Bis(diphenylphosphino)propan, Bis(dimethyl-phosphino)methan, Bis(dimethylphosphino)ethan, Bis(dimethylphosphino)-propan, Bis(diethylphosphino)methan, Bis(diethylphosphino)ethan, Bis(diethylphosphino)propan, Bis(di-*tert*-butylphosphino)methan, Bis(di-*tert*-butylphosphino)ethan, Bis(*tert*-butylphosphino)propan, 1,3-Diketonaten abgeleitet von 1,3-Diketonen, wie z. B. Acetylaceton, Benzoylaceton, 1,5-Diphenylacetylaceton, Dibenzoylmethan, Bis(1,1,1-tri-fluoracetyl)-methan, 3-Ketonaten abgeleitet von 3-Ketoestern, wie z. B. Acetessigsäureethylester, Carboxylate, abgeleitet von Aminocarbonsäuren, wie z. B. Pyridin-2-carbonsäure, Chinolin-2-carbonsäure, Glycin, N,N-Dimethylglycin, Alanin, N,N-Dimethylaminoalanin, Salicyliminaten abgeleitet von Salicyliminen, wie z. B. Methylsalicylimin, Ethylsalicylimin, Phenylsalicylimin, Dialkoholaten abgeleitet von Dialkoholen, wie z. B. Ethylenglykol, 1,3-Propylenglykol und Dithiolaten abgeleitet von Dithiolen, wie z. B. 1,2-Ethylendithiol, 1,3-Propylendithiol.

Bevorzugte tridentate Liganden sind Borate stickstoffhaltiger Heterocyclen, wie z. B. Tetrakis(1-imidazolyl)borat und Tetrakis(1-pyrazolyl)borat.

Bevorzugt sind weiterhin bidentate monoanionische Liganden L1, welche mit dem Metall einen cyclometallierten Fünfring mit mindestens einer Metall-Kohlenstoff-Bindung bilden. Dies sind insbesondere Liganden, wie sie allgemein im Gebiet der phosphoreszierenden Metallkomplexe für organische Elektrolumineszenzvorrichtungen verwendet werden, also Liganden vom Typ Phenylpyridin, Naphthylpyridin, Phenylchinolin, Phenylisochinolin, etc., welche jeweils durch einen oder mehrere Reste R substituiert sein können. Dem Fachmann auf dem Gebiet der phosphoreszierenden Elektrolumineszenzvorrichtungen ist eine Vielzahl derartiger Liganden bekannt, und er kann ohne erfinderisches Zutun weitere derartige Liganden als Ligand L1 für Verbindungen gemäß Formel (3) auswählen. Generell eignet sich dafür besonders die Kombination aus zwei Gruppen, wie sie oben durch die Formeln (21) bis (49) dargestellt sind, wobei eine Gruppe über ein neutrales Stickstoffatom oder ein Carbenatom bindet und die andere Gruppe über ein negativ geladenes Kohlenstoffatom oder ein negativ geladenes Stickstoffatom bindet. Der Ligand L1 kann dann aus den Gruppen der Formeln (21) bis (49) gebildet werden, indem diese Gruppen jeweils an der durch eine gestrichelte Bindung angedeuteten Bindung aneinander binden.

Ebenfalls bevorzugte Liganden L1 sind η⁵-Cyclopentadienyl, η⁵-Penta-methylcyclopentadienyl, η⁶-Benzol oder η⁷-Cycloheptatrienyl, welche jeweils durch einen oder mehrere Reste R substituiert sein können.

Ebenfalls bevorzugte Liganden L1 sind 1,3,5-cis-Cyclohexanderivate, insbesondere der Formel (50), 1,1,1-Tri(methylen)methanderivate, insbesondere der Formel (51) und 1,1,1-trisubstituierte Methane, insbesondere der Formel (52), 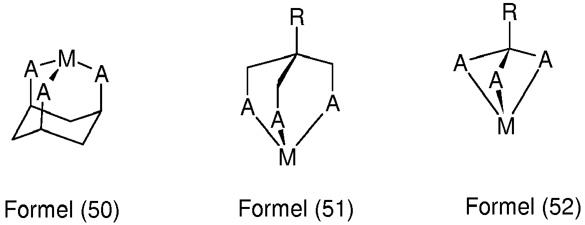 wobei in den Formeln jeweils die Koordination an das Metall M dargestellt ist, R die oben genannte Bedeutung hat und A, gleich oder verschieden bei jedem Auftreten, für O⁻, S⁻, COO⁻, P(R)₂ oder N(R)₂ steht.

Eine weitere bevorzugte Ausführungsform der Erfindung sind organische elektronische Vorrichtungen enthaltend mindestens eine Verbindung gemäß den Formeln (53), (54) und (55), 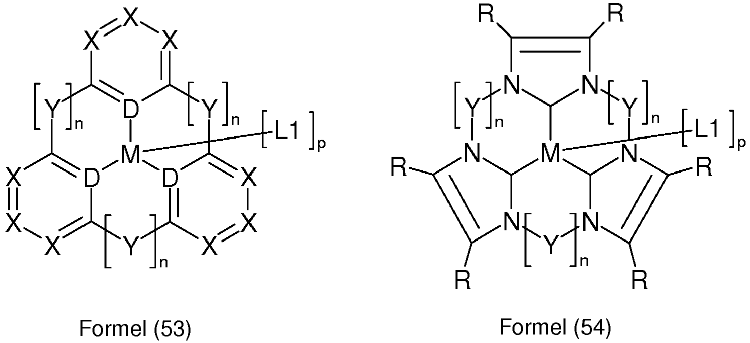 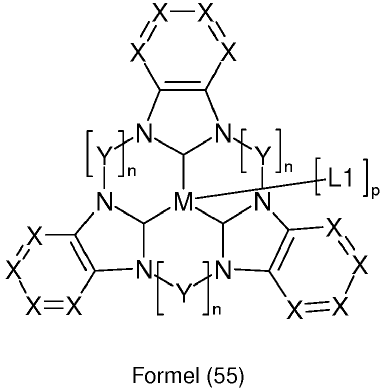 wobei M, X, Y, L1, R, R¹, n und p die oben genannten Bedeutungen haben und weiterhin gilt:
- D: steht bei jedem Auftreten gleich oder verschieden für C, N oder C-O⁻.

Eine besonders bevorzugte Ausführungsform der Erfindung sind Verbindungen der Formeln (53) bis (55), in denen eine Gruppe Y, zwei Gruppen Y oder alle drei Gruppen Y für BR¹, C(R¹)₂, Si(R¹)₂, NR¹, PR¹, AsR¹, P(=O)R¹, As(=O)R¹, P(=S)R¹ oder As(=S)R¹ stehen und der Substituent R¹ bzw. einer der Substituenten R¹ im Falle von C(R¹)₂ und Si(R¹)₂ für eine Gruppe L2 steht. Besonders bevorzugte Ausführungsformen der Erfindung sind also die Verbindungen gemäß den folgenden Formeln (56) bis (64), 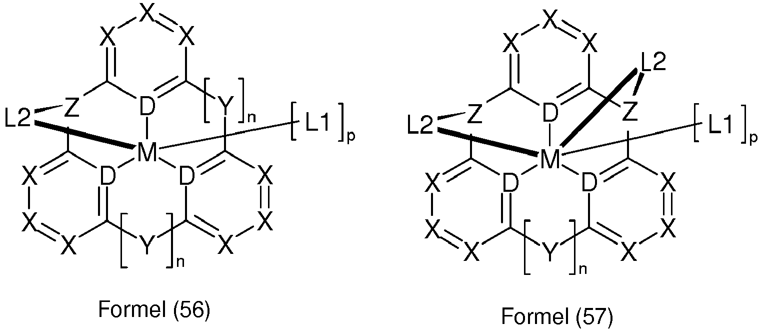 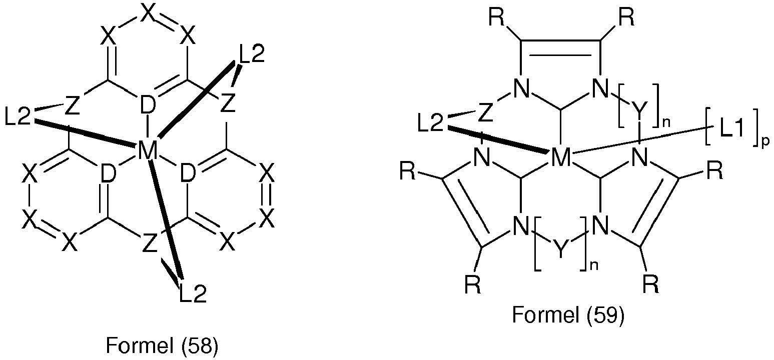 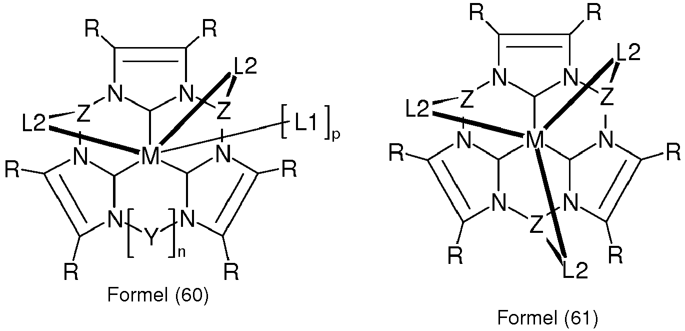 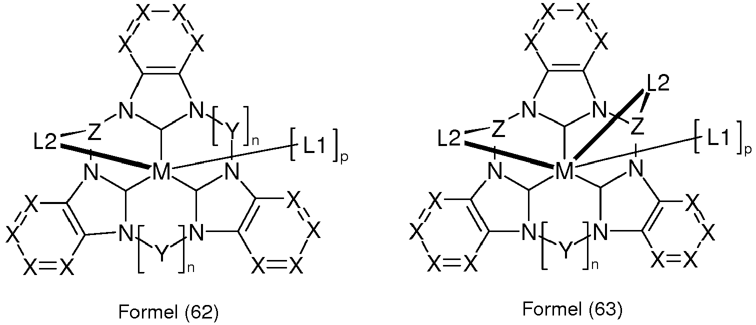 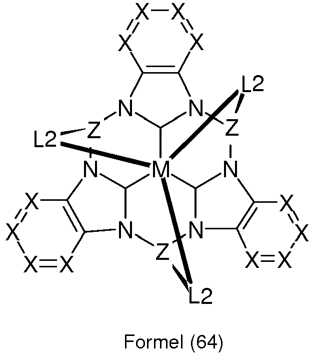 wobei die Symbole und Indizes die oben aufgeführten Bedeutungen haben. Dabei sind die Gruppen L2 bevorzugt, gleich oder verschieden bei jedem Auftreten, Gruppen der oben abgebildeten Formeln (21) bis (49).

Eine weitere besonders bevorzugte Ausführungsform der Verbindungen der Formel (8) sind die Verbindungen der folgenden Formel (65), 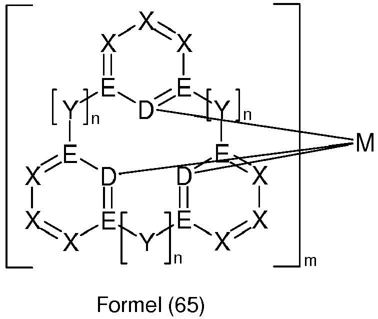 wobei die Symbole und Indizes dieselben Bedeutungen haben, wie oben beschrieben.

Bevorzugte Ausführungsformen der Verbindungen gemäß Formel (53) bis (55) bzw. (56) bis (65) sind solche, wie sie oben bereits für die Verbindungen gemäß Formel (3) bis (7) ausführlich aufgeführt wurden.

Bevorzugt sind weiterhin Verbindungen der Formeln (3) bis (7) bzw. (53) bis (65), in denen R bei jedem Auftreten gleich oder verschieden für H, Deuterium, F, CN, eine geradkettige Alkyl- oder Alkoxygruppe mit 1 bis 6 C-Atomen oder eine verzweigte oder cyclische Alkyl- oder Alkoxygruppe mit 3 bis 6 C-Atomen, die jeweils mit einem oder mehreren Resten R² substituiert sein kann, wobei eine oder mehrere nicht benachbarte CH₂-Gruppen durch R²C=CR², O oder S ersetzt sein können und ein oder mehrere H-Atome durch F ersetzt sein können, oder eine Aryl- oder Heteroarylgruppe mit 5 bis 16 aromatischen Ringatomen, welche jeweils durch einen oder mehrere Reste R² substituiert sein kann, oder eine Diarylaminogruppe mit 10 bis 20 aromatischen Ringatomen, welche durch einen oder mehrere Reste R² substituiert sein kann, oder eine Kombination dieser Systeme steht; dabei können zwei oder mehrere Substituenten R auch miteinander ein mono- oder polycyclisches aliphatisches, aromatisches und/oder benzoannelliertes Ringsystem bilden. Besonders bevorzugt steht das Symbol R, gleich oder verschieden bei jedem Auftreten, für H, Deuterium, F, eine geradkettige Alkylgruppe mit 1 bis 4 C-Atomen oder eine verzweigte Alkylgruppe mit 3 oder 4 C-Atomen, die jeweils mit einem oder mehreren Resten R² substituiert sein kann, wobei ein oder mehrere H-Atome durch F ersetzt sein können, oder eine Arylgruppe mit 6 bis 10 aromatischen Ringatomen, welche durch einen oder mehrere Reste R² substituiert sein kann; dabei können zwei oder mehrere Substituenten R auch miteinander ein mono- oder polycyclisches aliphatisches, aromatisches und/oder benzoannelliertes Ringsystem bilden.

Die Komplexe gemäß Formel (3) sind prinzipiell durch verschiedene Verfahren herstellbar, wobei sich jedoch die im Folgenden beschriebenen Verfahren als besonders gut geeignet herausgestellt haben. Dabei werden die Komplexe gemäß Formel (3) durch Umsetzung des makrocyclischen Liganden und gegebenenfalls weiteren Liganden L1 mit Metallalkoholaten gemäß Formel (66), mit Metallketoketonaten gemäß Formel (67) oder Metallhalogeniden gemäß Formel (68) erhalten, 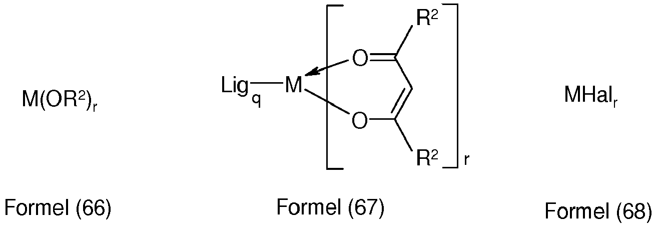 wobei M und R² dieselbe Bedeutung haben, wie oben beschrieben, und für die weiteren Symbole und Indizes gilt:
- Hal: ist bei jedem Auftreten gleich oder verschieden F, Cl, Br oder I;
- Lig: ist bei jedem Auftreten gleich oder verschieden ein neutraler oder monoanionischer, einzähniger oder zweizähniger Ligand, beispielsweise ein Halogenid oder Hydroxid;
- q: ist bei jedem Auftreten gleich oder verschieden 0, 1, 2, 3 oder 4, bevorzugt 0, 1 oder 2;
- r: ist bei jedem Auftreten gleich oder verschieden 1, 2, 3, 4 oder 5, wobei r in Formel (66) und (68) die Wertigkeit des Metalls M angibt;
dabei kann die Verbindung gemäß Formel (67) auch geladen sein und noch ein Gegenion enthalten; weiterhin können die Verbindungen gemäß Formel (66) bis (68), insbesondere gemäß Formel (68) auch als Hydrat vorliegen.

Ebenso ist eine komplexanaloge Synthese der Liganden möglich, indem Vorstufen des Liganden mit Metallverbindungen der Formel (66), (67) oder (68) umgesetzt werden und die so gebildeten Metallkomplexe dann weiter zum fertigen Liganden umgesetzt werden.

Die Synthese kann beispielsweise thermisch, photochemisch oder durch Mikrowellenstrahlung aktiviert werden. Die Synthese tris-ortho-metallierter Metallkomplexe ist allgemein in WO 02/060910, WO 04/085449, WO 04/108738 und WO 07/065523 beschrieben. Die in diesen Schriften ausgeführten Syntheseverfahren und bevorzugten Reaktionsbedingungen lassen sich auf die Synthese von Verbindungen gemäß Formel (3) analog anwenden. Bevorzugt als Ausgangsverbindung für Iridiumkomplexe sind Verbindungen gemäß Formel (67), insbesondere die Verbindung Na[IrCl₂(acac)₂], und Verbindungen gemäß Formel (68) in Form von Hydraten, insbesondere IrCl₃ Hydrat.

Durch diese Verfahren lassen sich die Komplexe leicht in hoher Reinheit, bevorzugt in einer Reinheit von > 99 % nach ¹H-NMR oder HPLC, besonders bevorzugt > 99.9 % erhalten.

Beispiele für bevorzugte Verbindungen gemäß Formel (3) sind die im Folgenden abgebildeten Verbindungen (1) bis (307). Diese Komplexe lassen sich unter anderem mit den oben erläuterten Synthesemethoden herstellen.

| | | | |
|---|---|---|---|
| 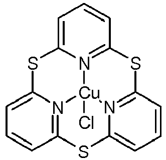 | 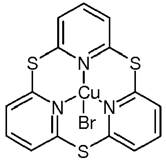 | | 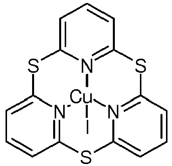 |
| (1) | (2) | | (3) |
| 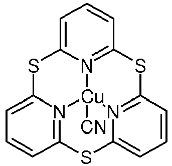 | 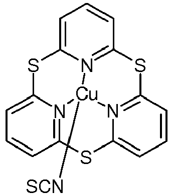 | | 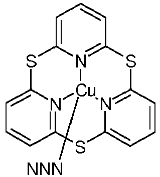 |
| (4) | (5) | | (6) |
| 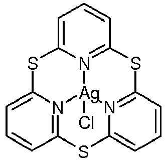 | 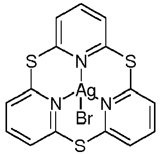 | | 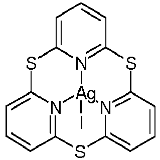 |
| (7) | (8) | | (9) |
| 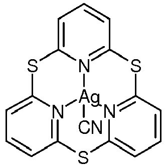 | 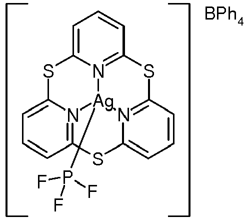 | | |
| (10) | (11) | | |
| 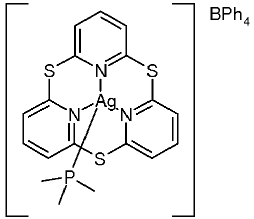 | | 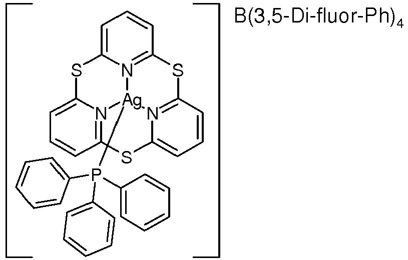 | |
| (12) | | (13) | |
| 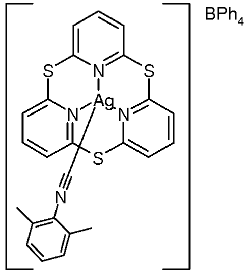 | | 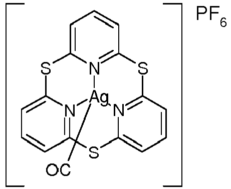 | |
| (14) | | (15) | |
| 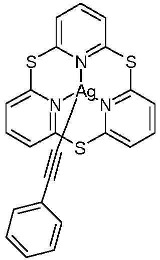 | 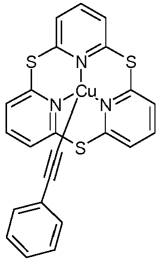 | | 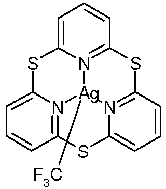 |
| (16) | (17) | | (18) |
| 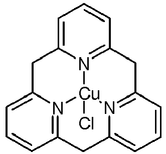 | 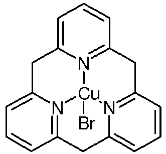 | | 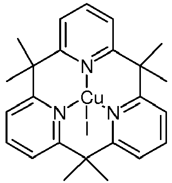 |
| (19) | (20) | | (21) |
| 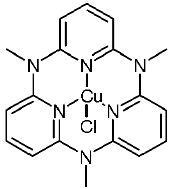 | 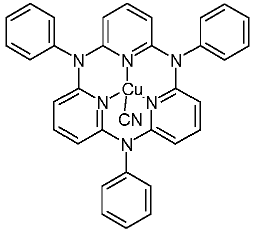 | | |
| (22) | (23) | | |
| 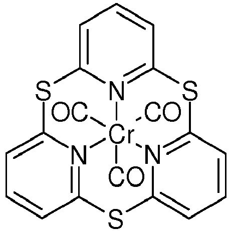 | 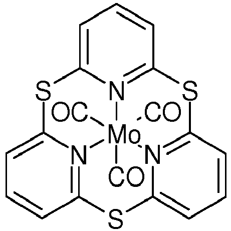 | | 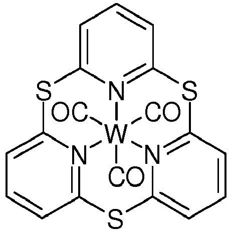 |
| (24) | (25) | | (26) |
| 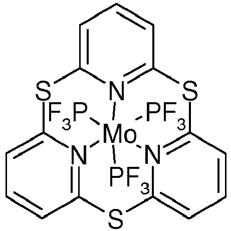 | 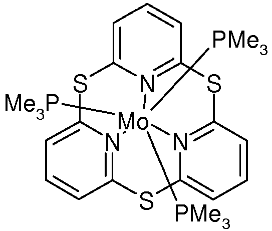 | | 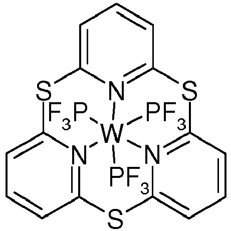 |
| (27) | (28) | | (29) |
| 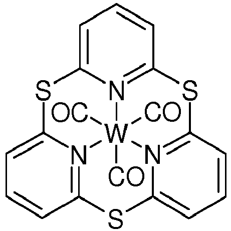 | 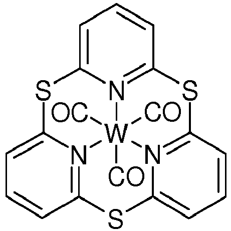 | | 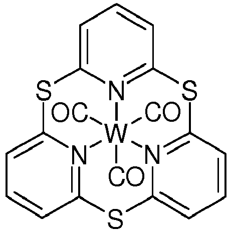 |
| (30) | (31) | | (32) |
| 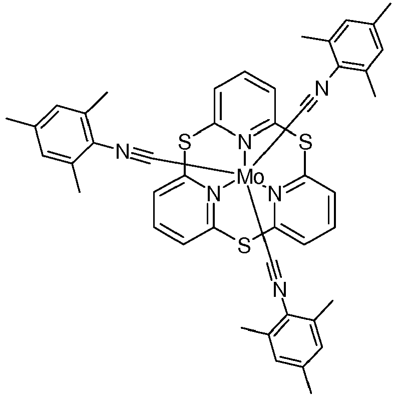 | | | |
| (33) | | | |
| 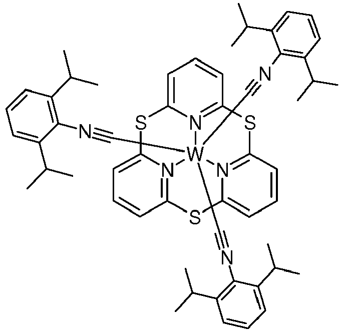 | | | |
| (34) | | | |
| 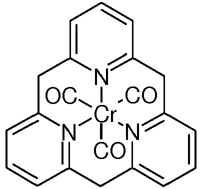 | 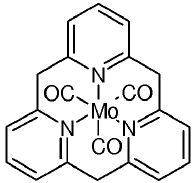 | | 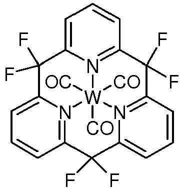 |
| (35) | (36) | | (37) |
| 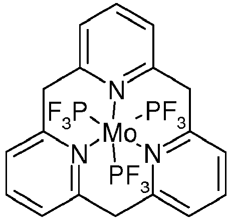 | 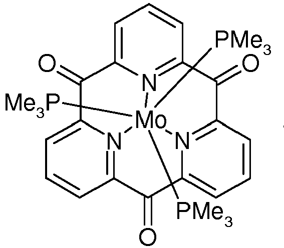 | | 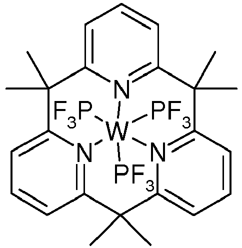 |
| (38) | (39) | | (40) |
| 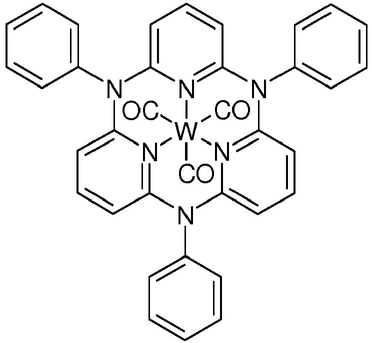 | | 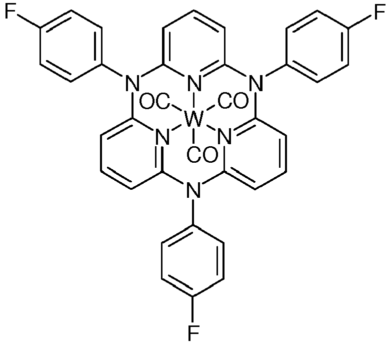 | |
| (41) | | (42) | |
| 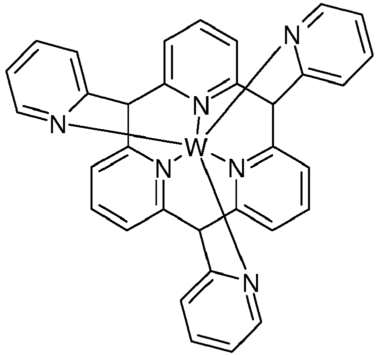 | | 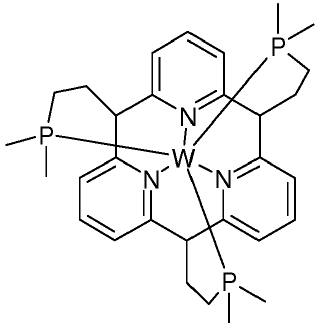 | |
| (43) | | (44) | |
| 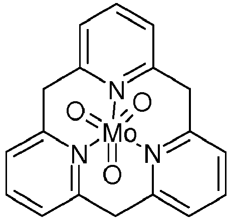 | 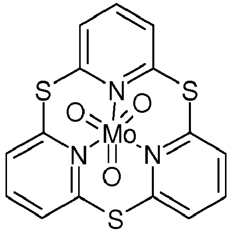 | | 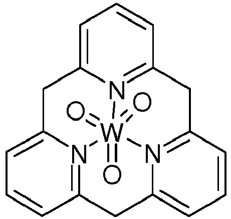 |
| (45) | (46) | | (47) |
| 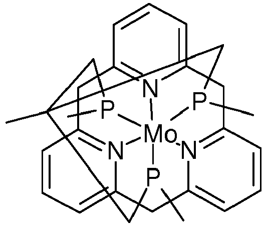 | 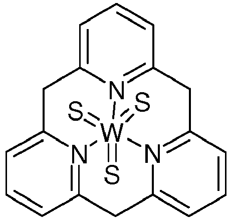 | | 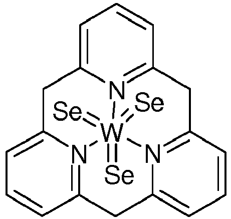 |
| (48) | (49) | | (50) |
| 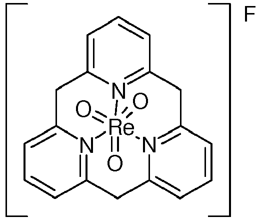 | 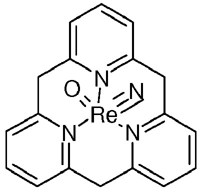 | | 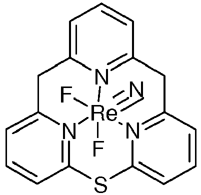 |
| (51) | (52) | | (53) |
| 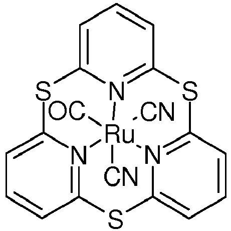 | 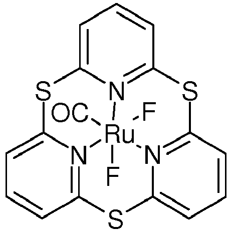 | | 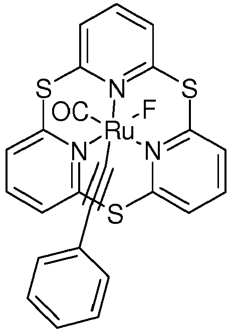 |
| (54) | (55) | | (56) |
| 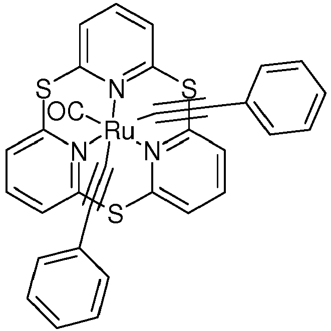 | | 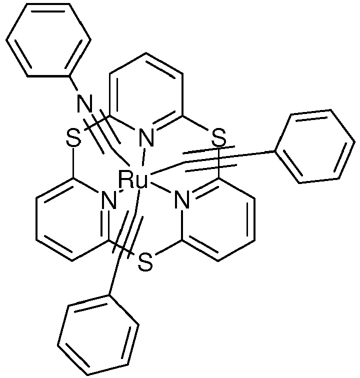 | |
| (57) | | (58) | |
| 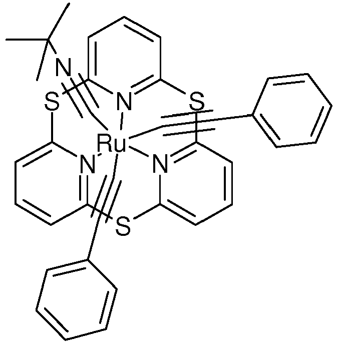 | | 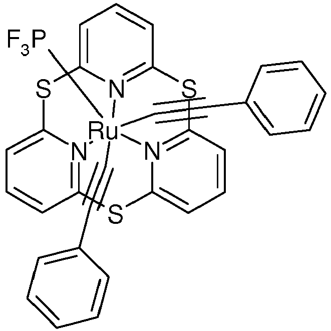 | |
| (59) | | (60) | |
| 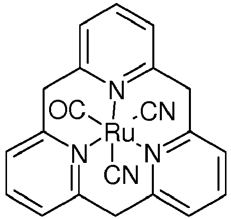 | 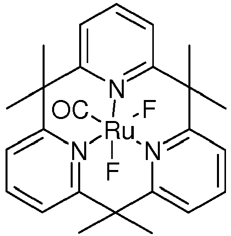 | | 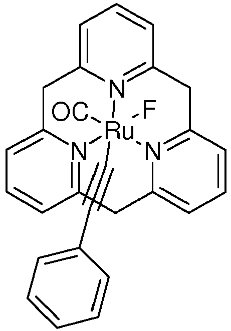 |
| (61) | (62) | | (63) |
| 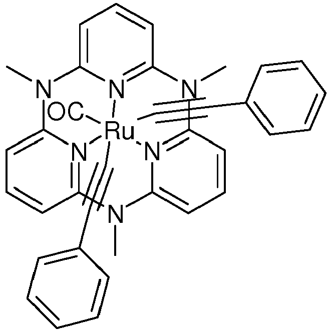 | | 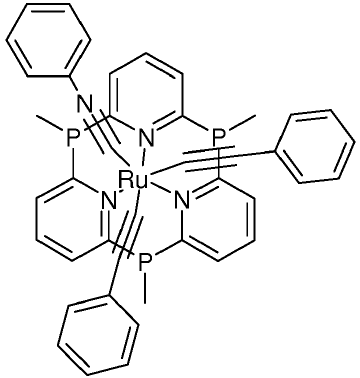 | |
| (64) | | (65) | |
| 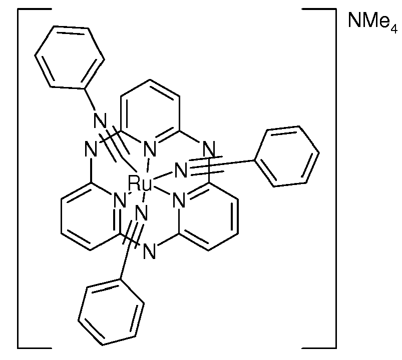 | | 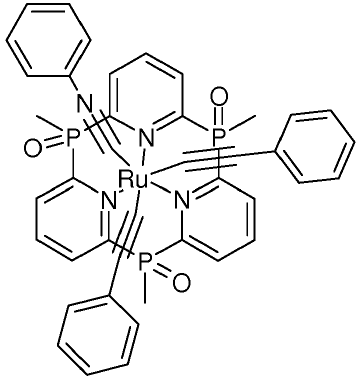 | |
| (66) | | (67) | |
| 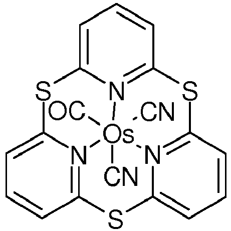 | 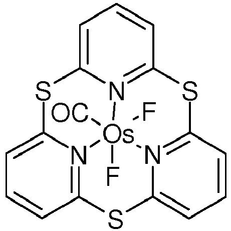 | | 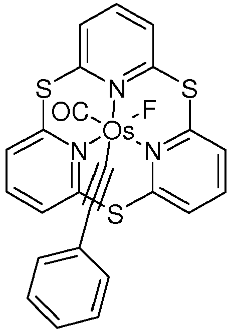 |
| (68) | (69) | | (70) |
| 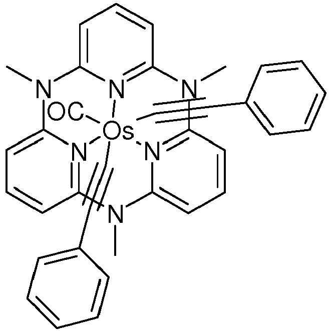 | | 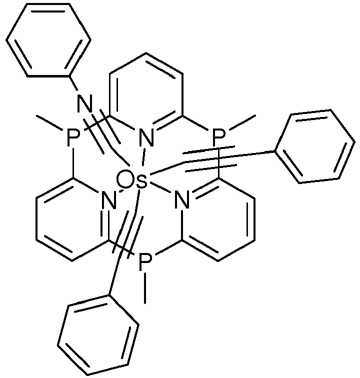 | |
| (71) | | (72) | |
| 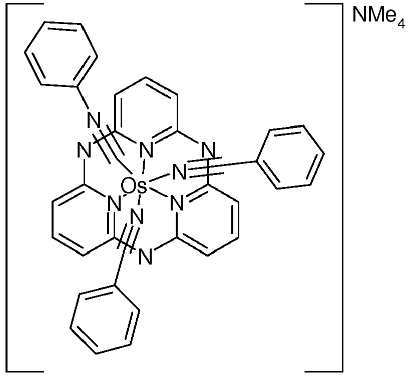 | | 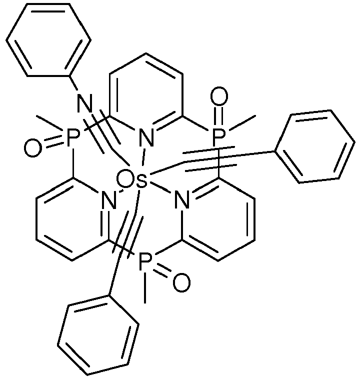 | |
| (73) | | (74) | |
| 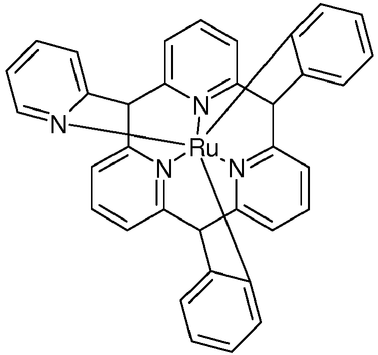 | | 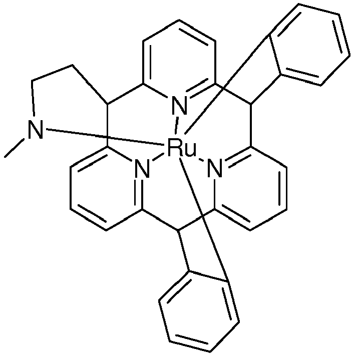 | |
| (75) | | (76) | |
| 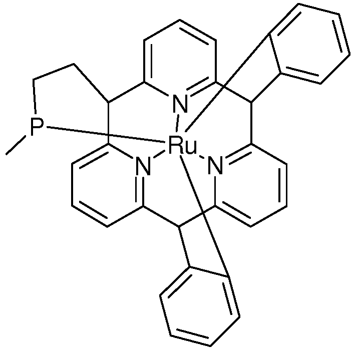 | | 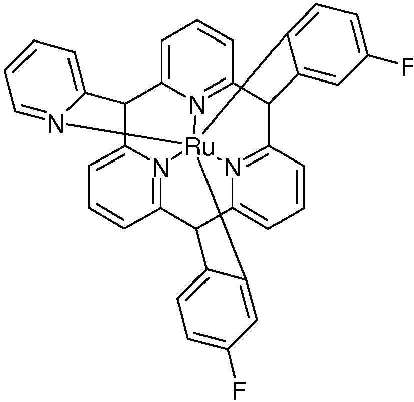 | |
| (77) | | (78) | |
| 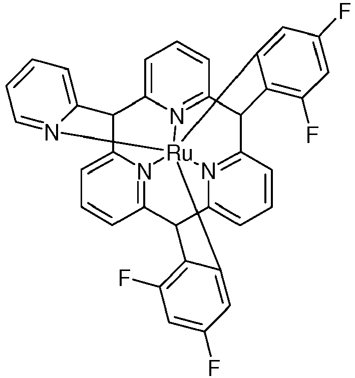 | | 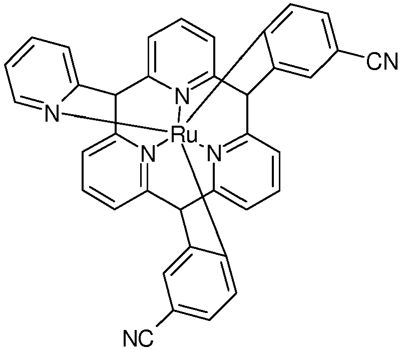 | |
| (79) | | (80) | |
| 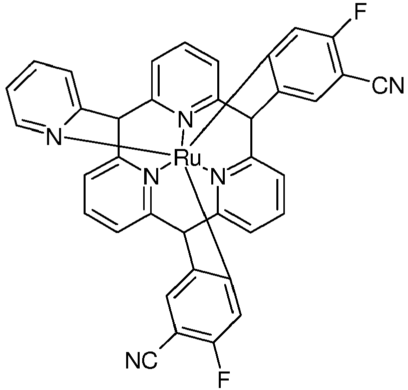 | | 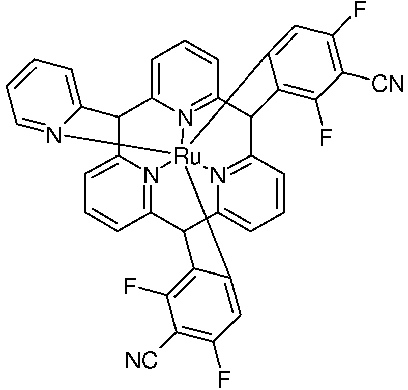 | |
| (81) | | (82) | |
| 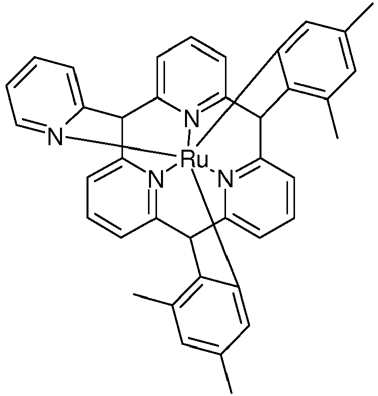 | | 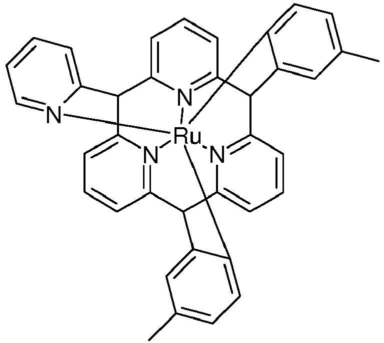 | |
| (83) | | (84) | |
| 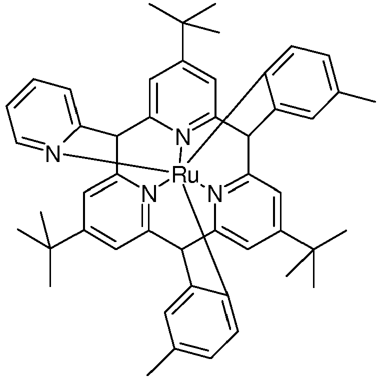 | | 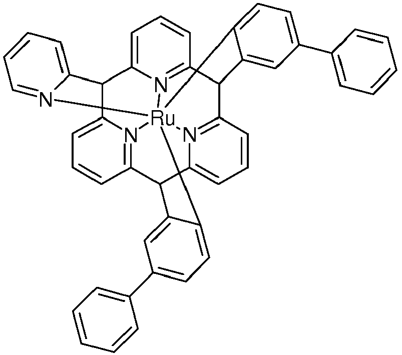 | |
| (85) | | (86) | |
| 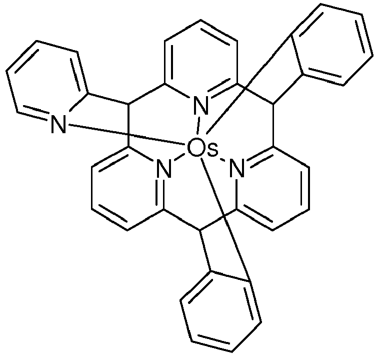 | | 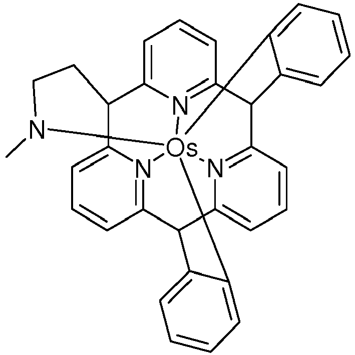 | |
| (87) | | (88) | |
| 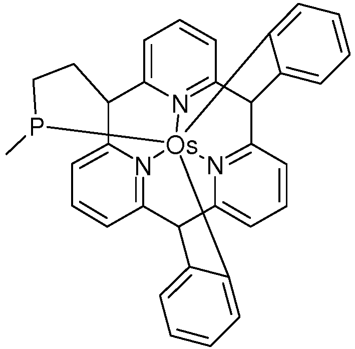 | | 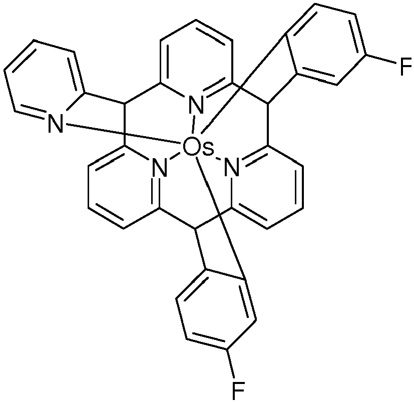 | |
| (89) | | (90) | |
| 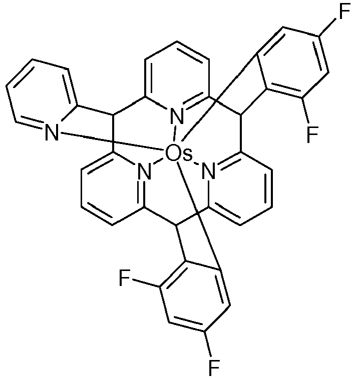 | | 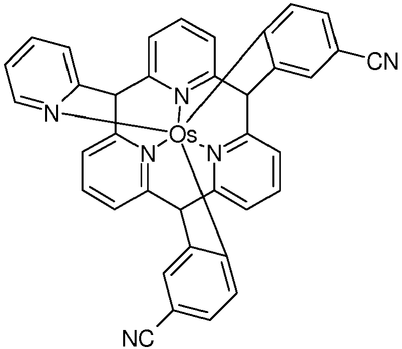 | |
| (91) | | (92) | |
| 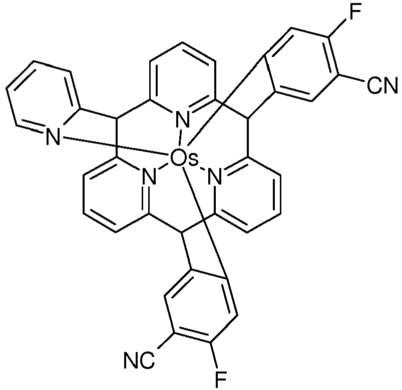 | | 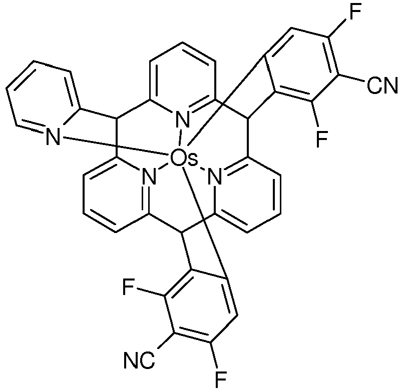 | |
| (93) | | (94) | |
| 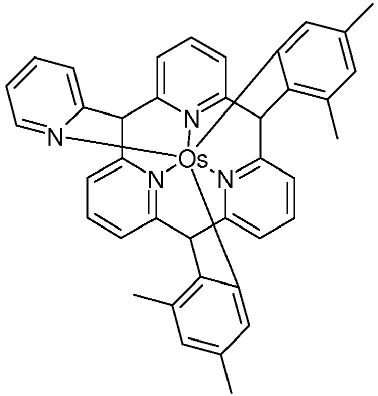 | | 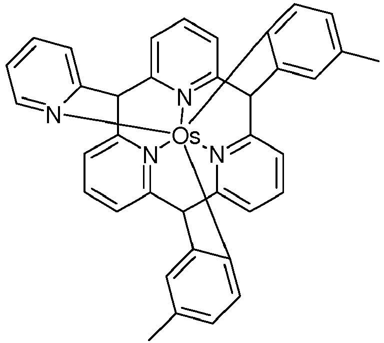 | |
| (95) | | (96) | |
| 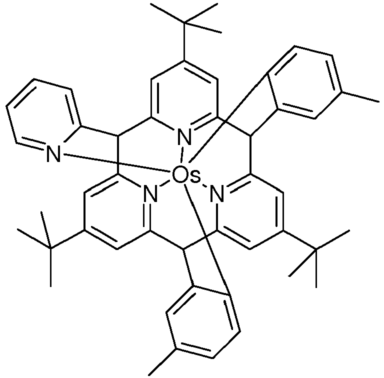 | | 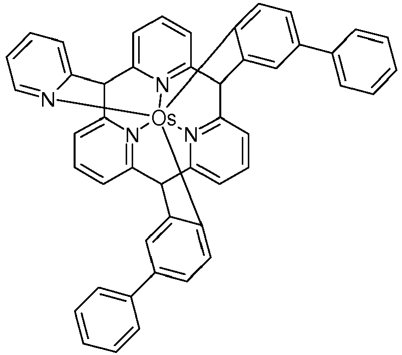 | |
| (97) | | (98) | |
| 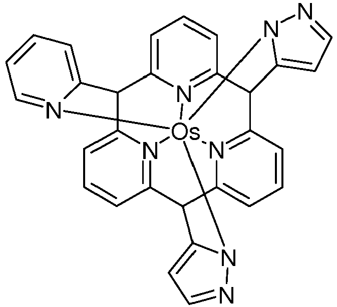 | | 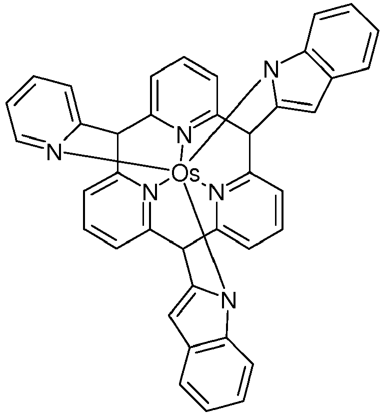 | |
| (99) | | (100) | |
| 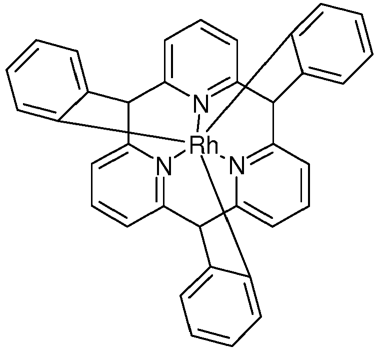 | | 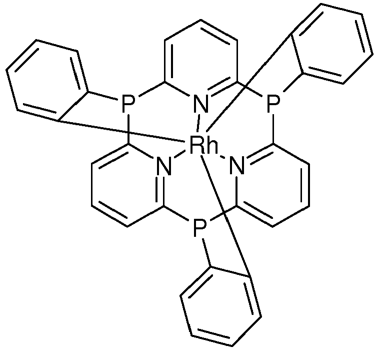 | |
| (101) | | (102) | |
| 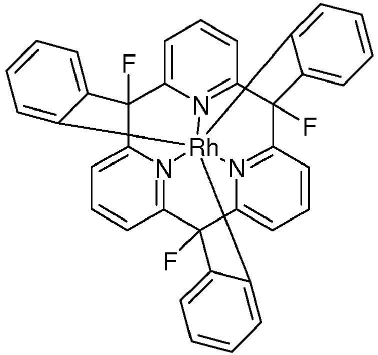 | | 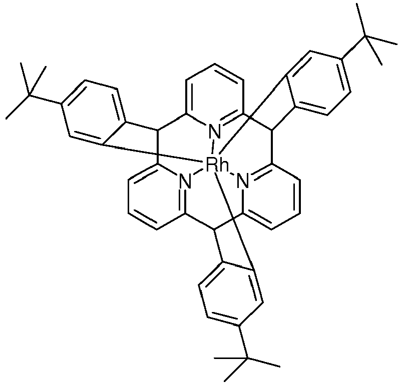 | |
| (103) | | (104) | |
| 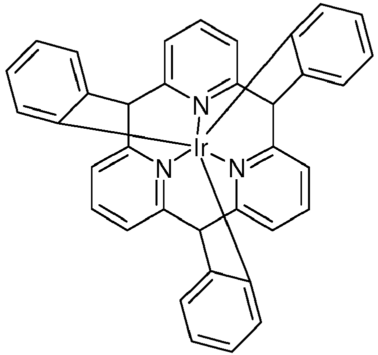 | | 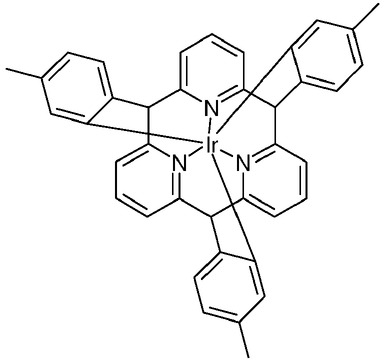 | |
| (105) | | (106) | |
| 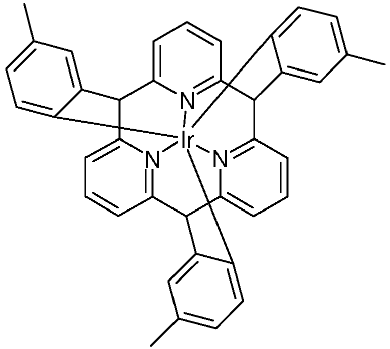 | | 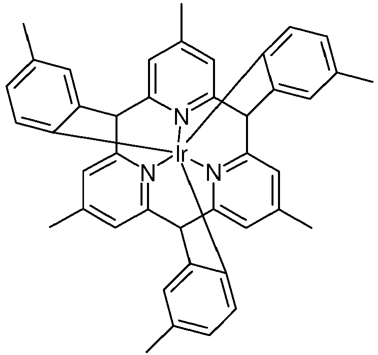 | |
| (107) | | (108) | |
| 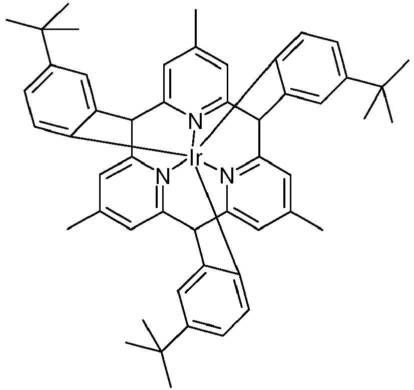 | | 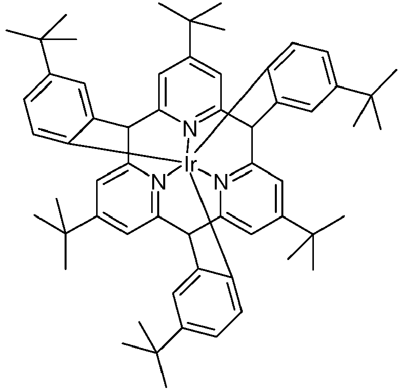 | |
| (109) | | (110) | |
| 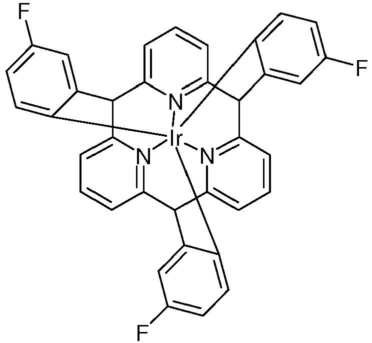 | | 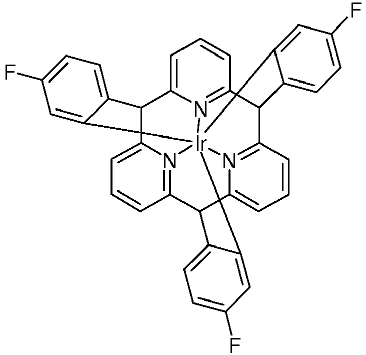 | |
| (111) | | (112) | |
| 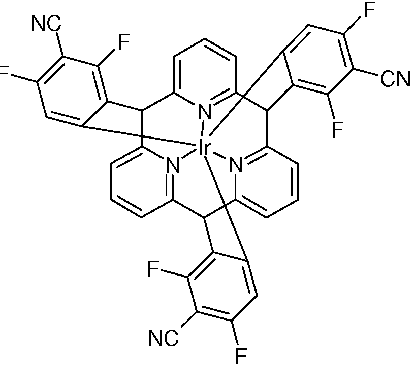 I | | 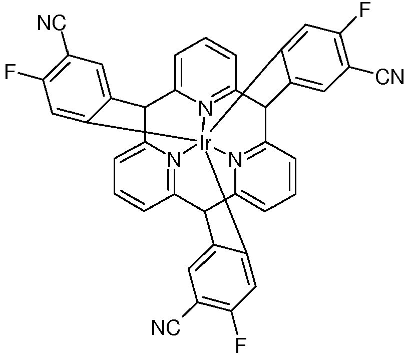 | |
| (113) | | (114) | |
| 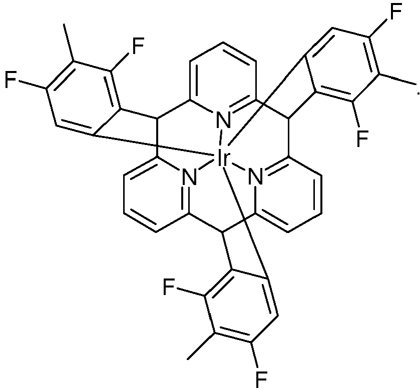 | | 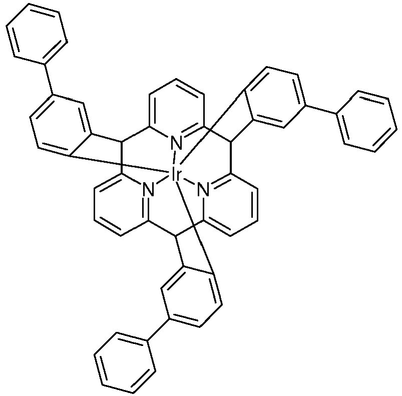 | |
| (115) | | (116) | |
| 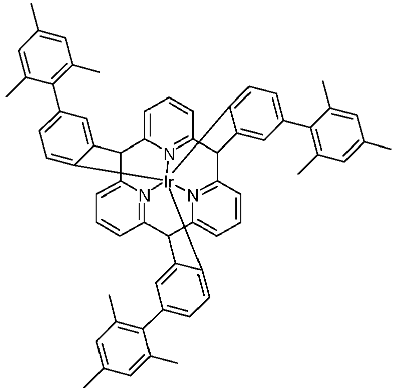 | | 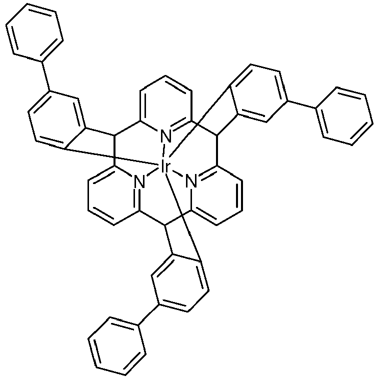 | |
| (117) | | (118) | |
| 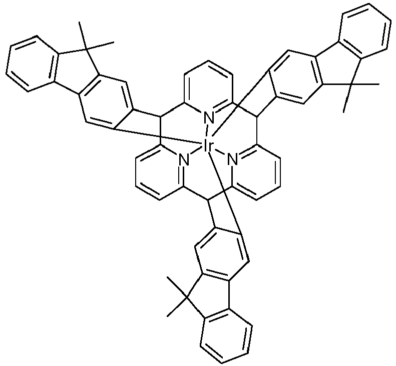 | | 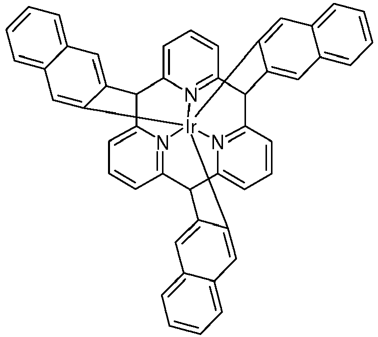 | |
| (119) | | (120) | |
| 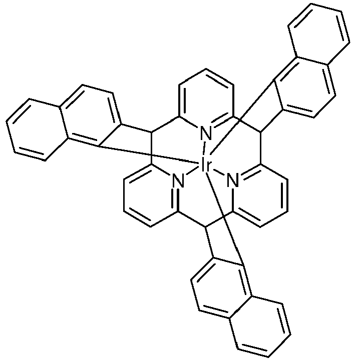 | | 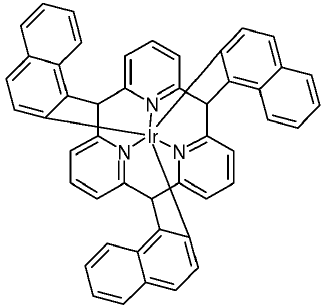 | |
| (121) | | (122) | |
| 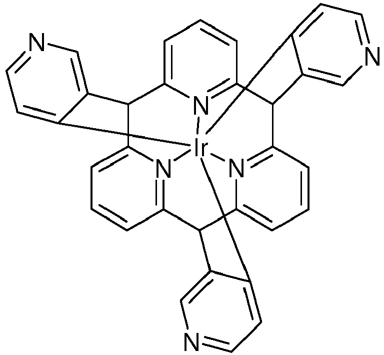 | | 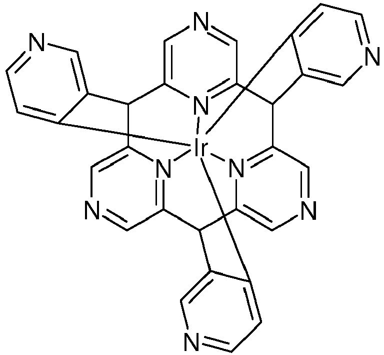 | |
| (123) | | (124) | |
| 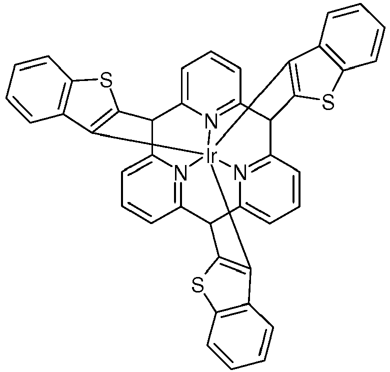 | | 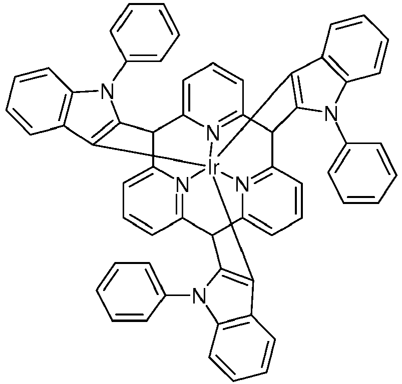 | |
| (125) | | (126) | |
| 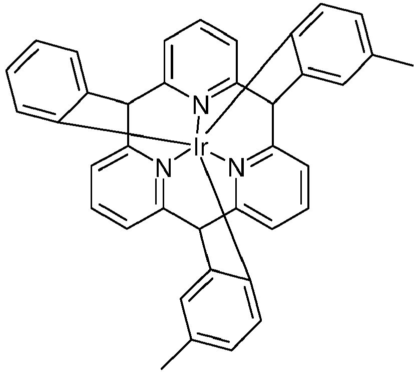 | | 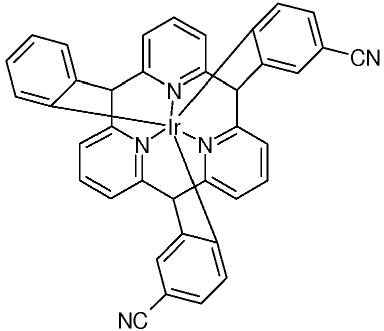 | |
| (127) | | (128) | |
| 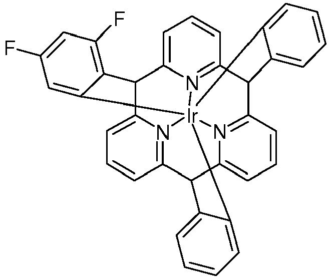 | | 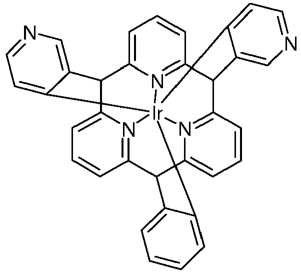 | |
| (129) | | (130) | |
| 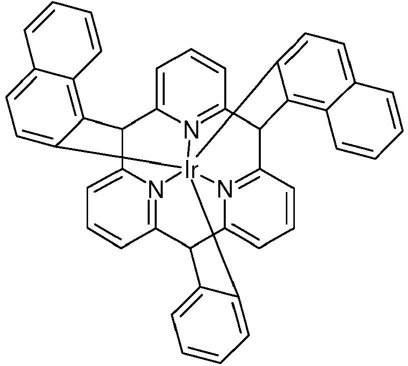 | | 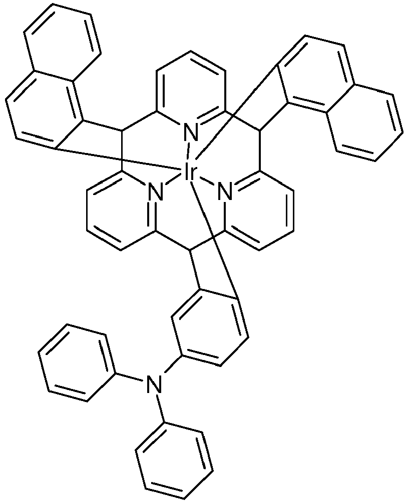 | |
| (131) | | (132) | |
| 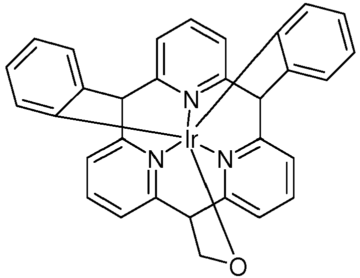 | | 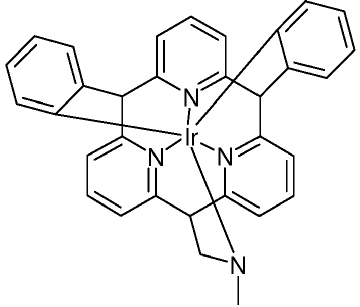 | |
| (133) | | (134) | |
| 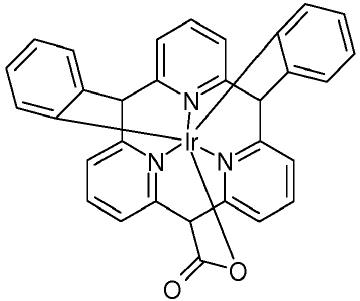 | | 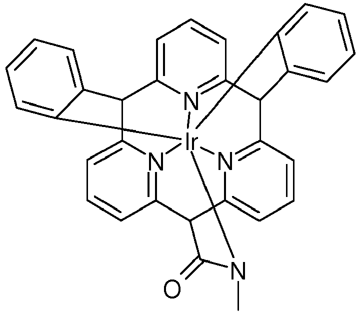 | |
| (135) | | (136) | |
| 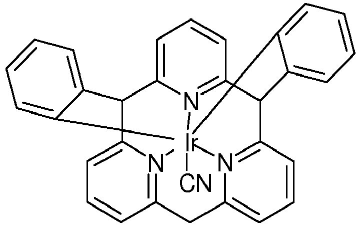 | | 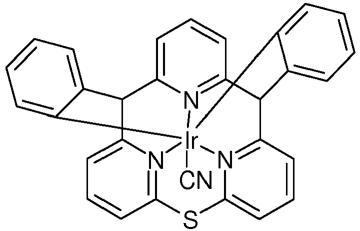 | |
| (137) | | (138) | |
| 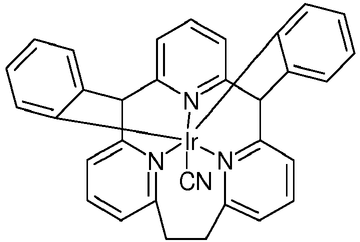 | | 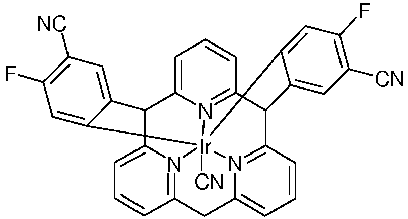 | |
| (139) | | (140) | |
| 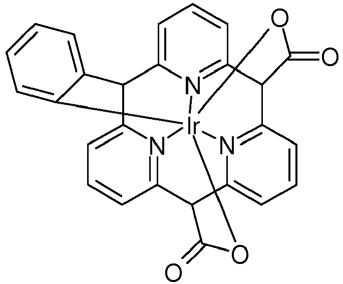 | | 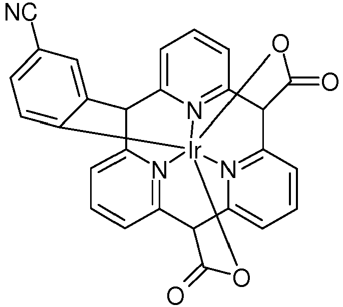 | |
| (141) | | (142) | |
| 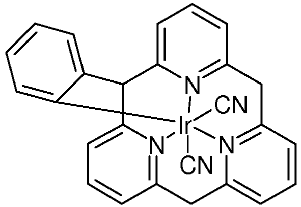 | | 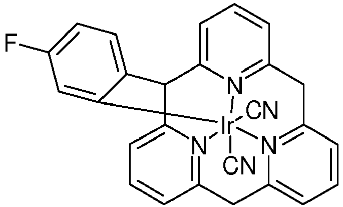 | |
| (143) | | (144) | |
| 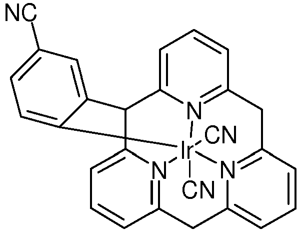 | | 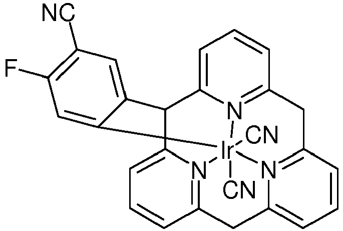 | |
| (145) | | (146) | |
| 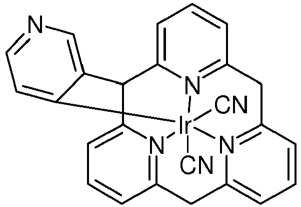 | | 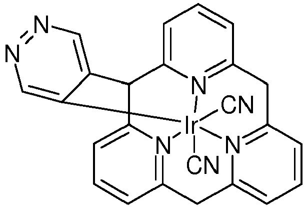 | |
| (147) | | (148) | |
| 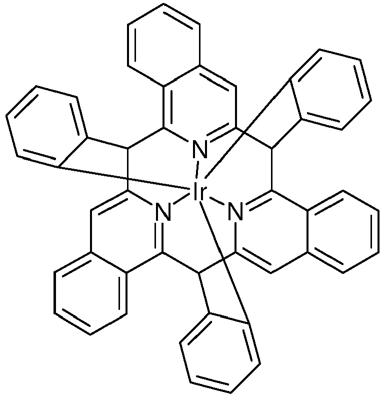 | | 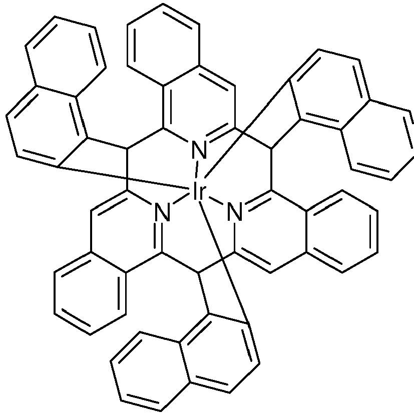 | |
| (149) | | (150) | |
| 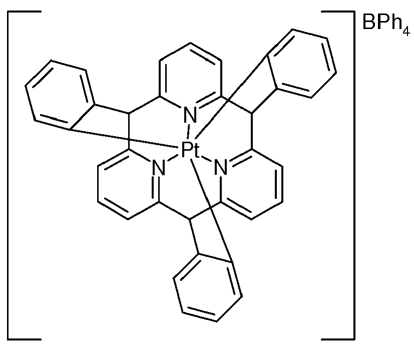 | | 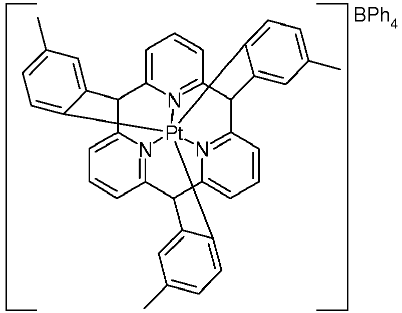 | |
| (151) | | (152) | |
| 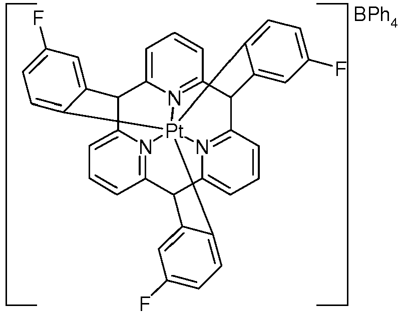 | | 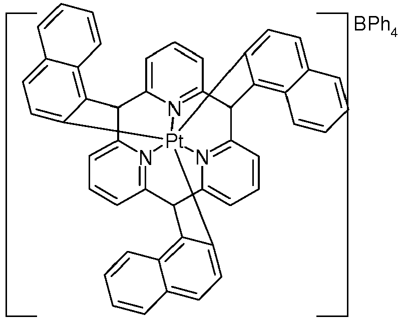 | |
| (153) | | (154) | |
| 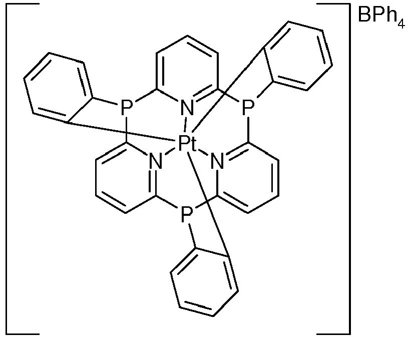 | | 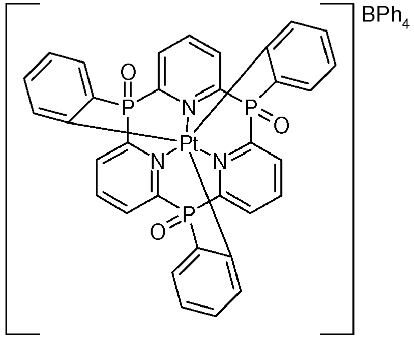 | |
| (155) | | (156) | |
| 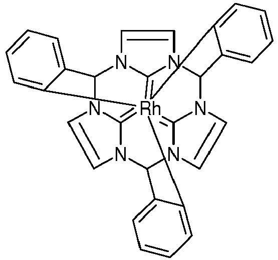 | | 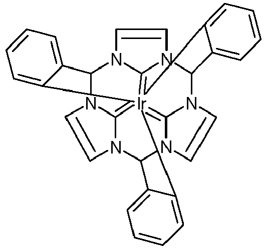 | |
| (157) | | (158) | |
| 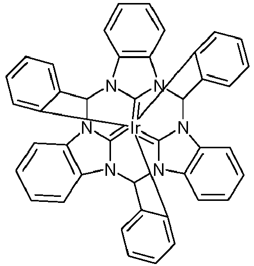 | | 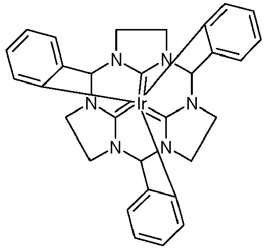 | |
| (159) | | (160) | |
| 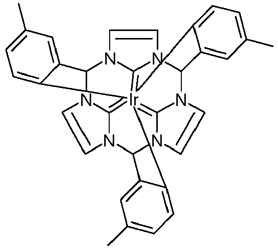 | | 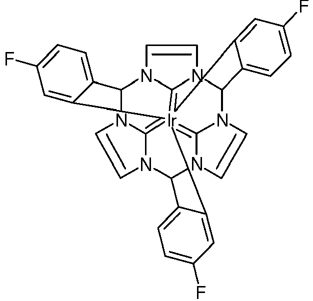 | |
| (161) | | (162) | |
| 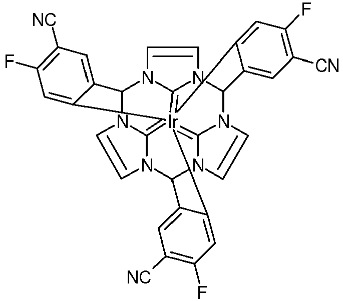 | | 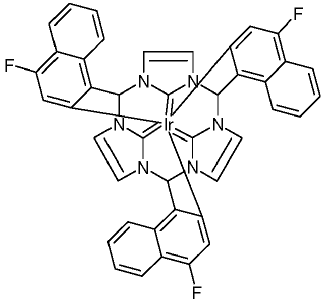 | |
| (163) | | (164) | |
| 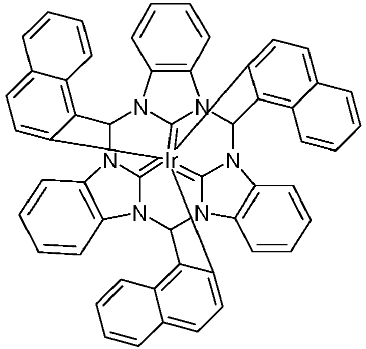 | | 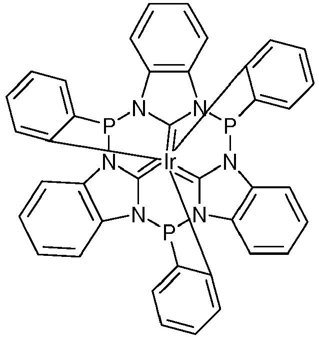 | |
| (165) | | (166) | |
| 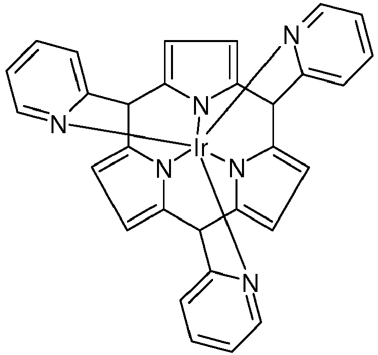 | | 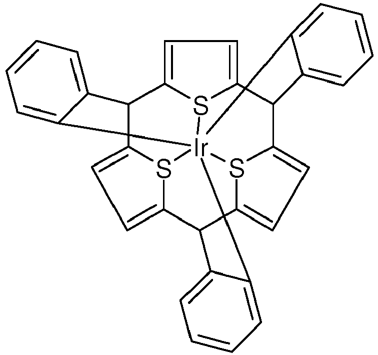 | |
| (167) | | (168) | |
| 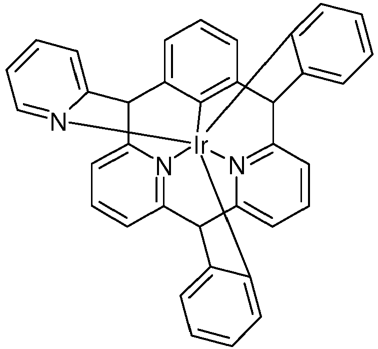 | | 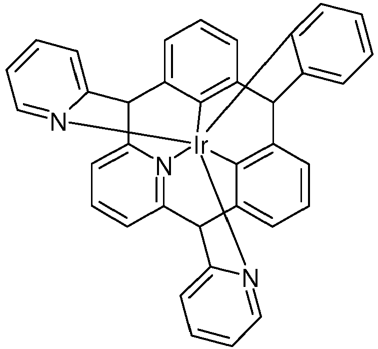 | |
| (169) | | (170) | |
| 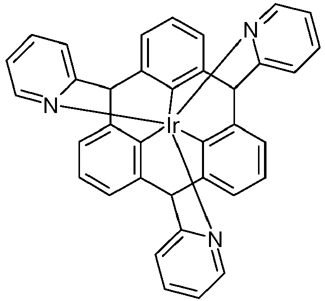 | | 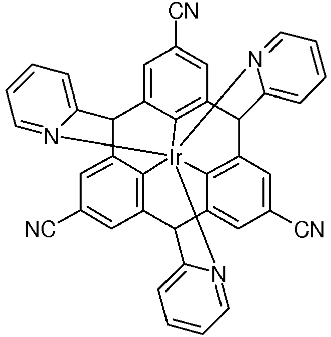 | |
| (171) | | (172) | |
| 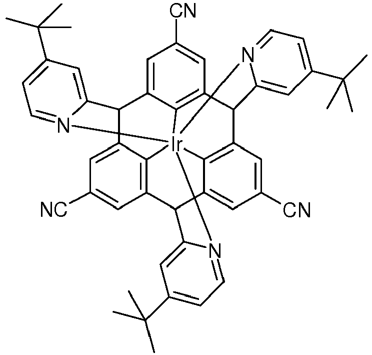 | | 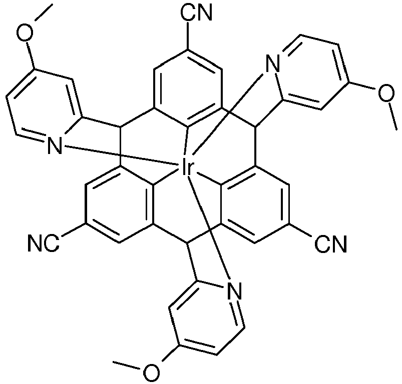 | |
| (173) | | (174) | |
| 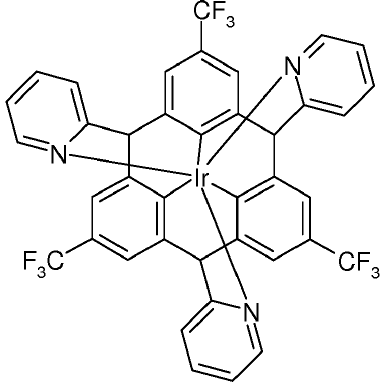 | | 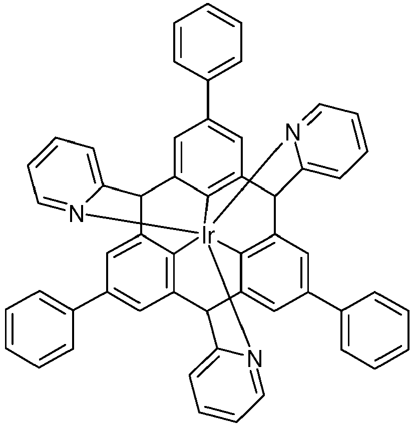 | |
| (175) | | (176) | |
| 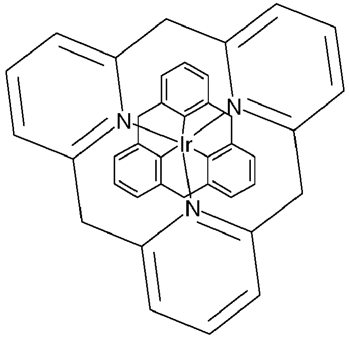 | | 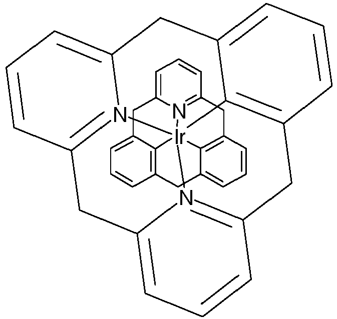 | |
| (177) | | (178) | |
| 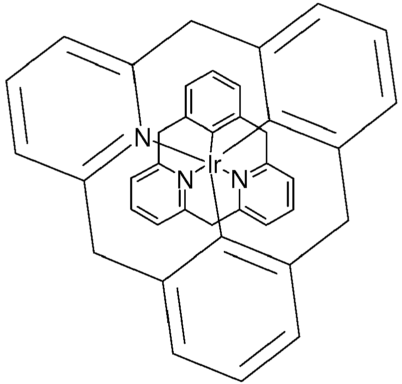 | | 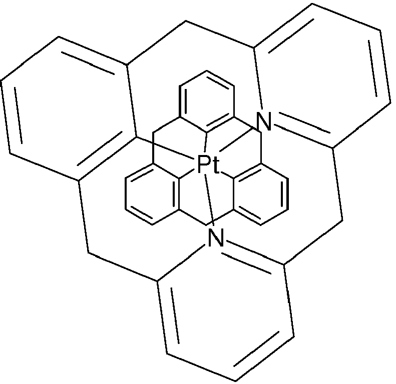 | |
| (179) | | (180) | |
| 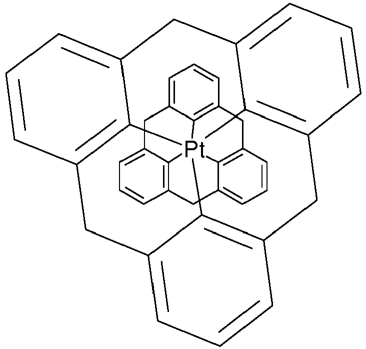 | | 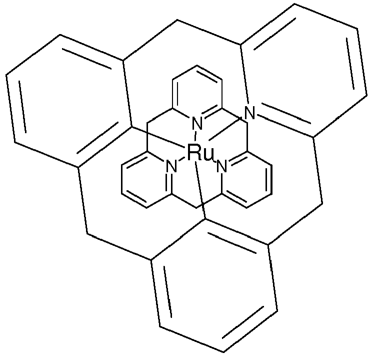 | |
| (181) | | (182) | |
| 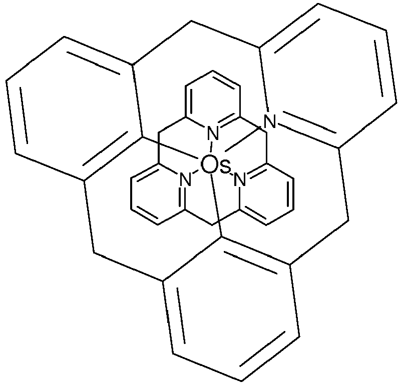 | | 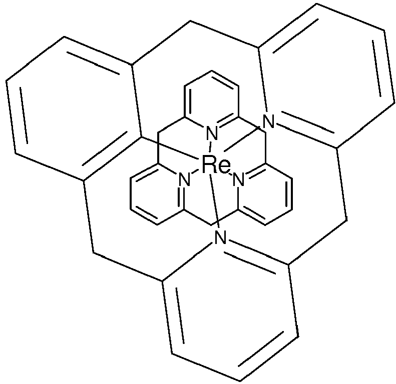 | |
| (183) | | (184) | |
| 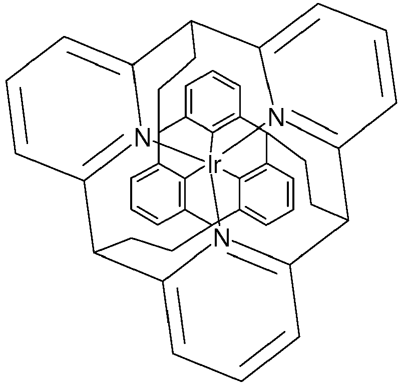 | | 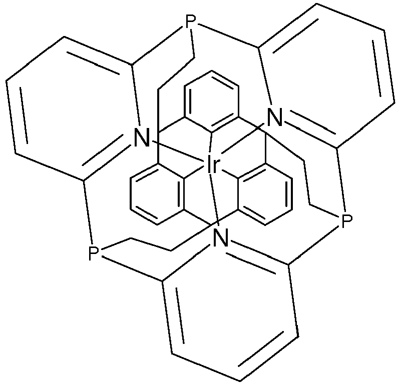 | |
| (185) | | (186) | |
| 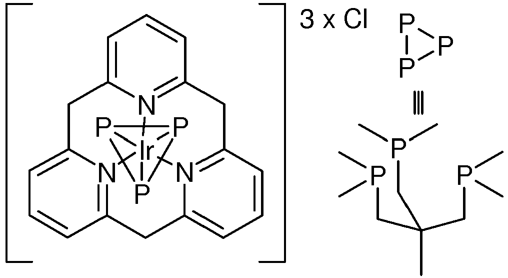 | | | |
| (187) | | | |
| | 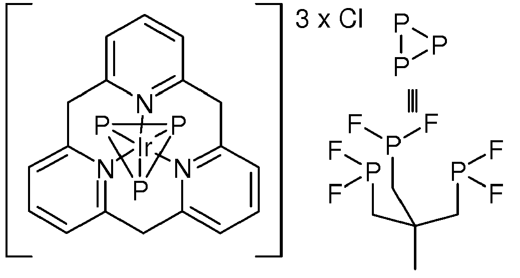 | | |
| (188) | | | |
| | 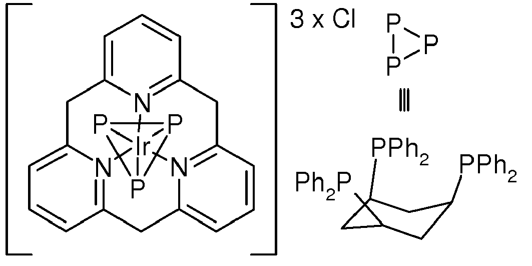 | | |
| (189) | | | |
| | 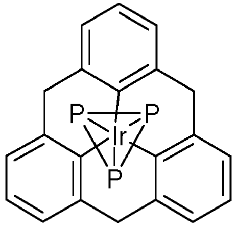 | 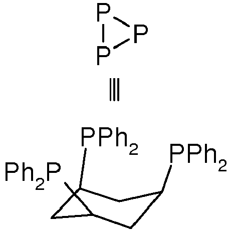 | |
| (190) | | | |
| | 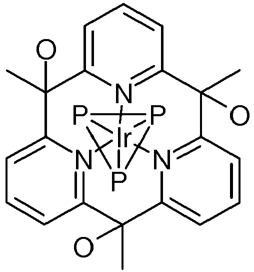 | 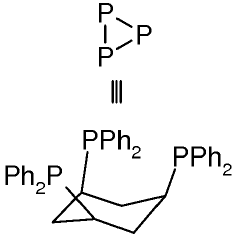 | |
| (191) | | | |
| | 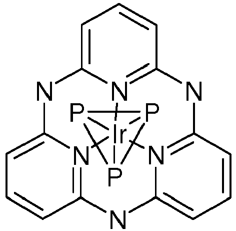 | 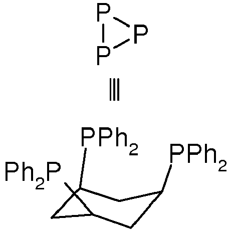 | |
| (192) | | | |
| | 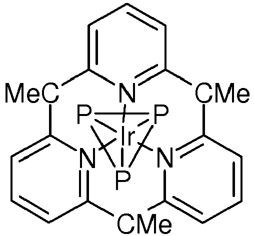 | 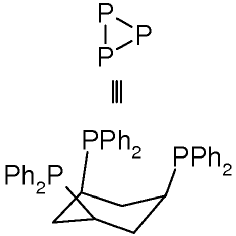 | |
| (193) | | | |
| | 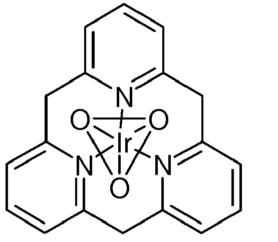 | 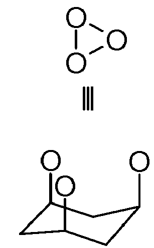 | |
| (194) | | | |
| | 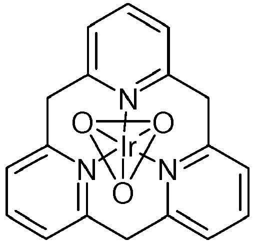 | 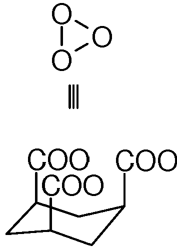 | |
| (195) | | | |
| 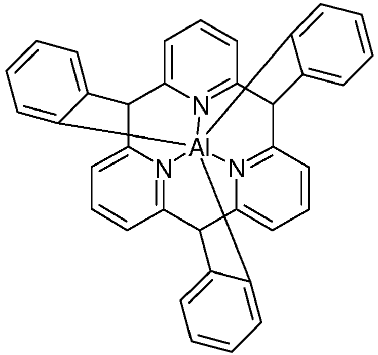 | | 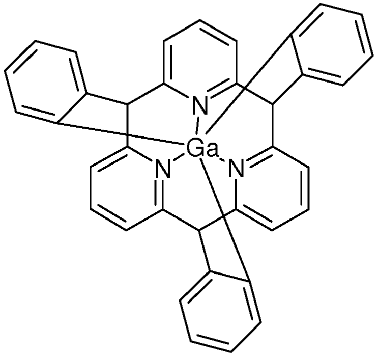 | |
| (196) | | (197) | |
| 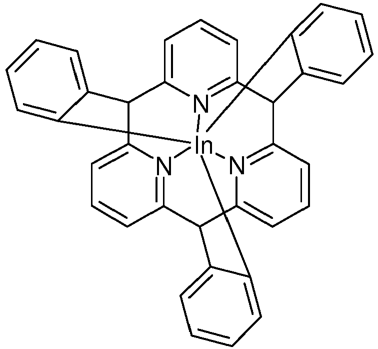 | | 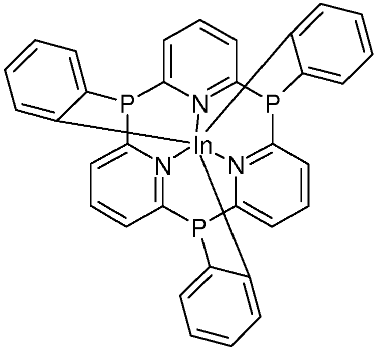 | |
| (198) | | (199) | |
| 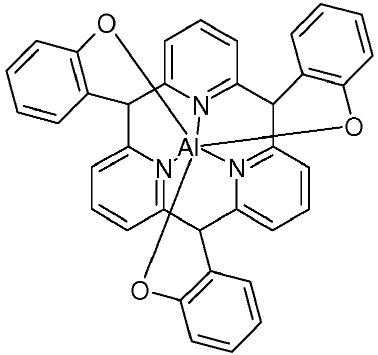 | | 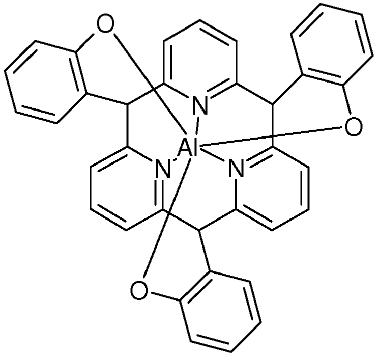 | |
| (200) | | (201) | |
| 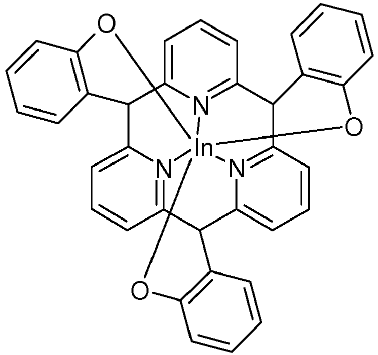 | | 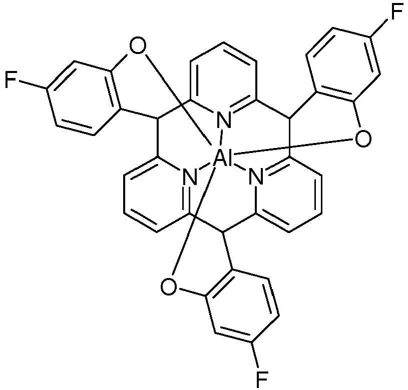 | |
| (202) | | (203) | |
| 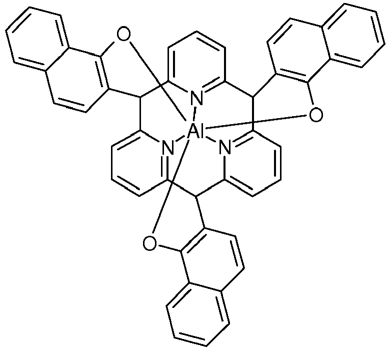 | | 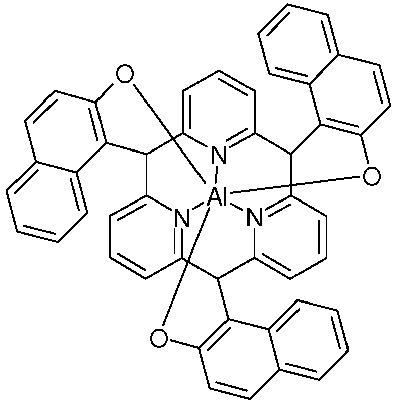 | |
| (204) | | (205) | |
| 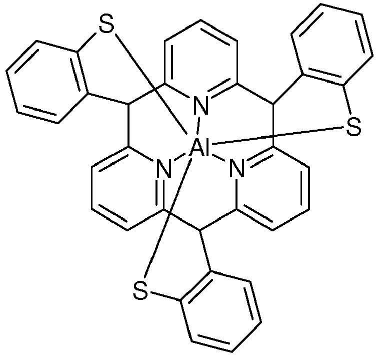 | | 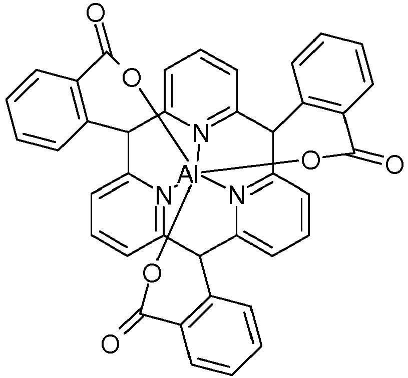 | |
| (206) | | (207) | |
| 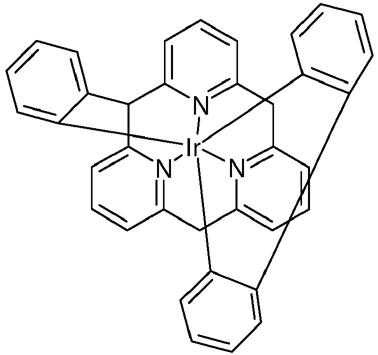 | | 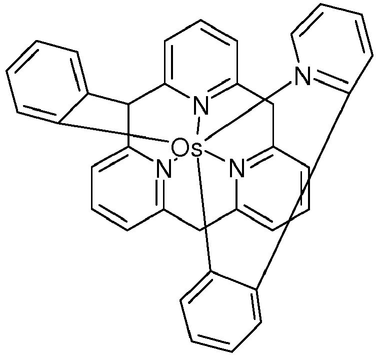 | |
| (208) | | (209) | |
| 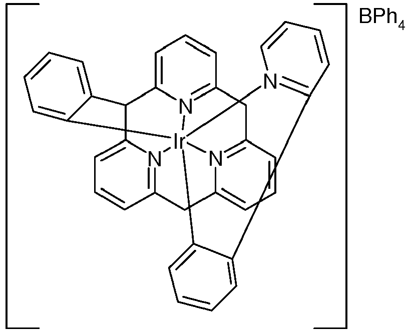 | | 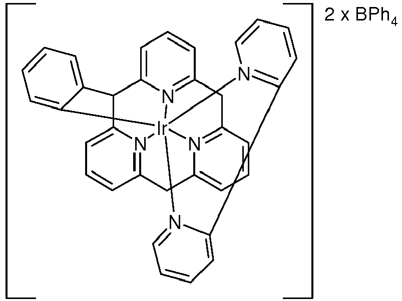 | |
| (210) | | (211) | |
| 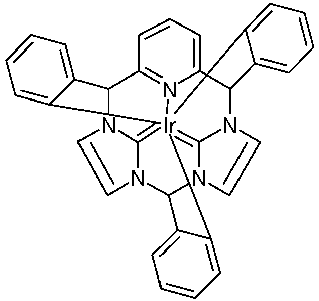 | | 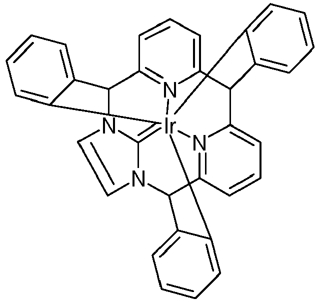 | |
| (212) | | (213) | |
| 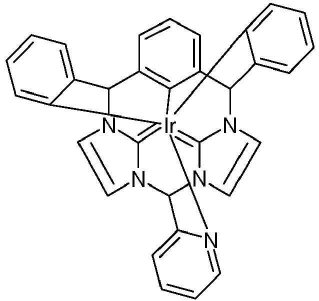 | | 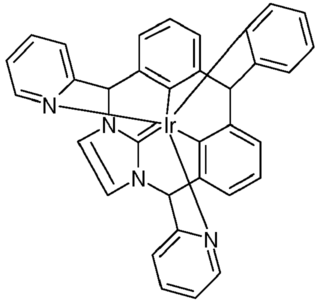 | |
| (214) | | (215) | |
| 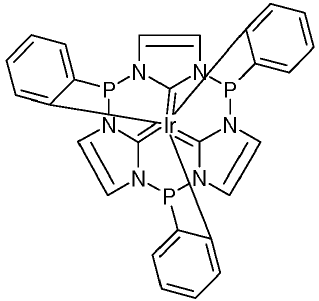 | | 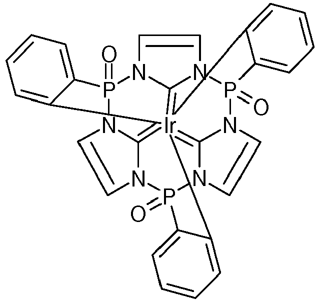 | |
| (216) | | (217) | |
| 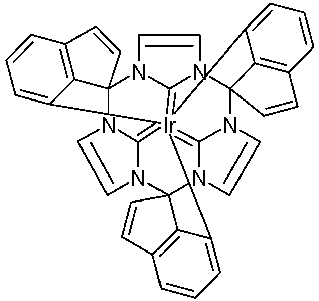 | | 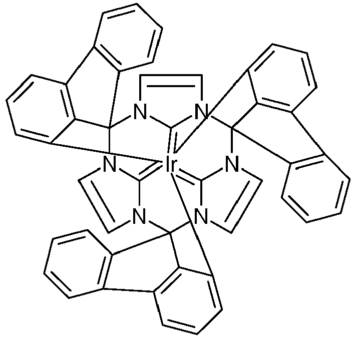 | |
| (218) | | (219) | |
| 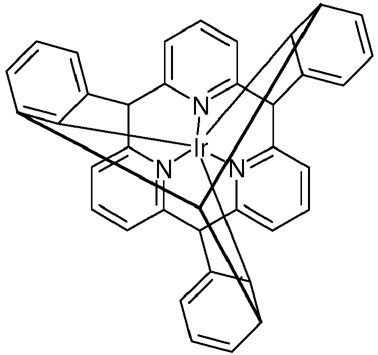 | | 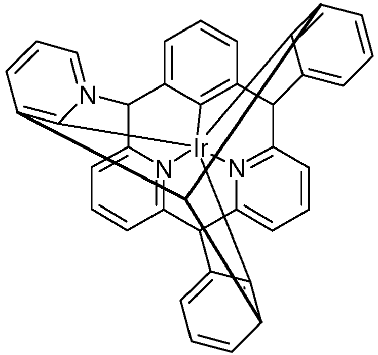 | |
| (220) | | (221) | |
| 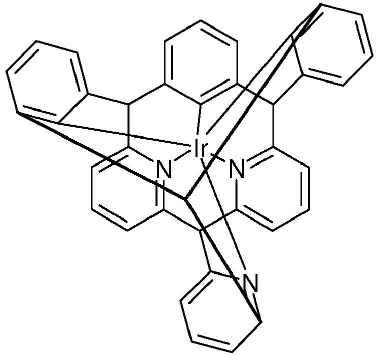 | | 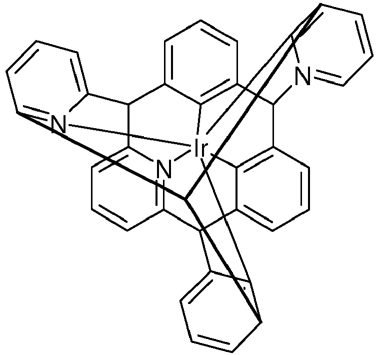 | |
| (222) | | (223) | |
| 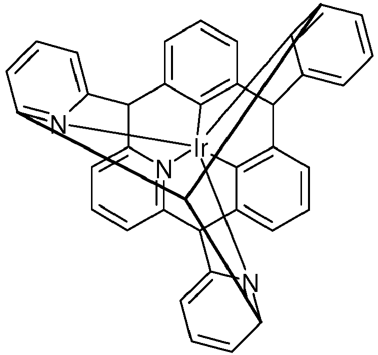 | | 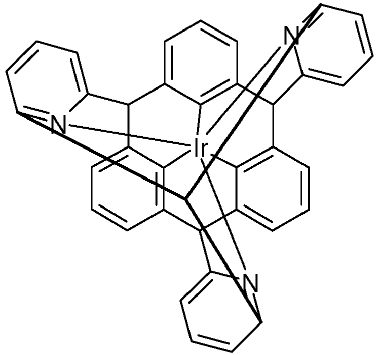 | |
| (224) | | (225) | |
| 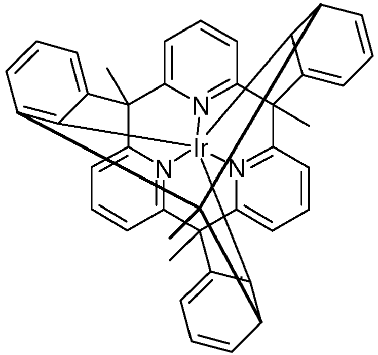 | | 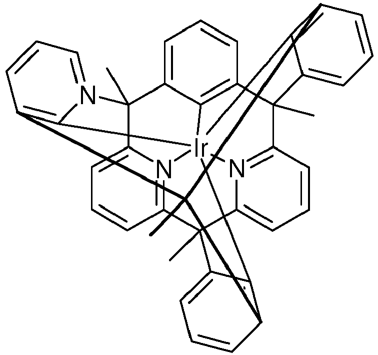 | |
| (226) | | (227) | |
| 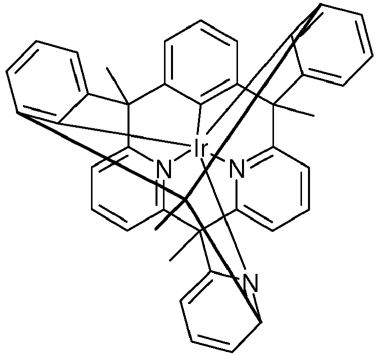 | | 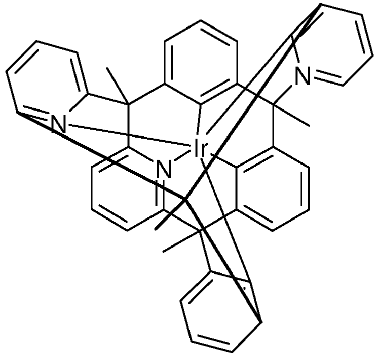 | |
| (228) | | (229) | |
| 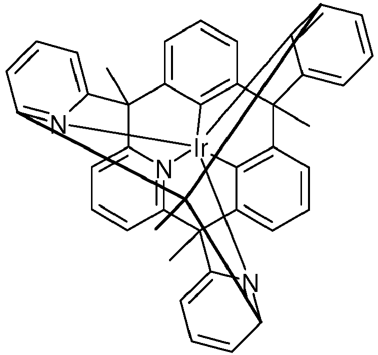 | | 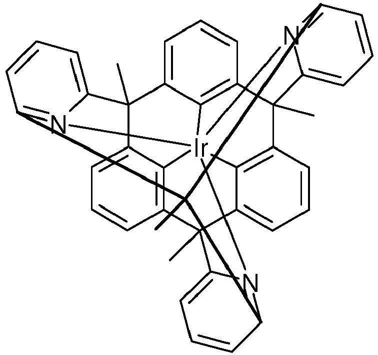 | |
| (230) | | (231) | |
| 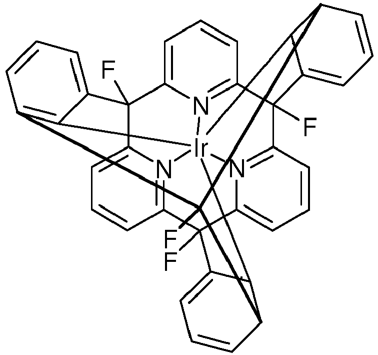 | | 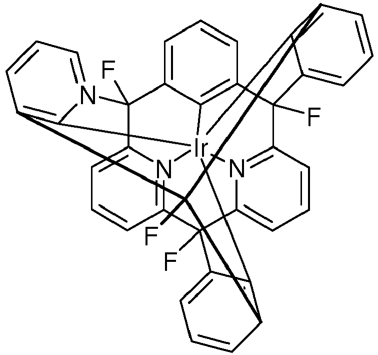 | |
| (232) | | (233) | |
| 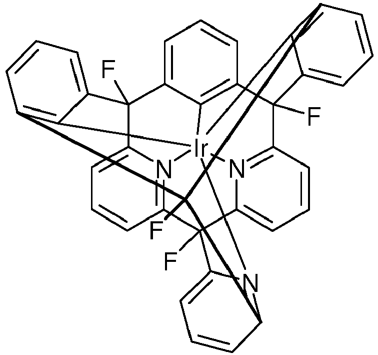 | | 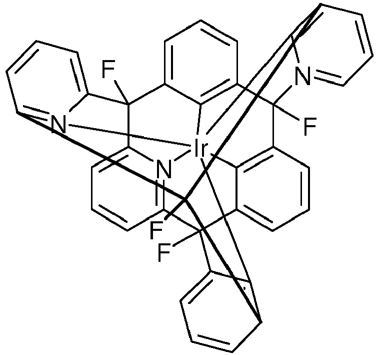 | |
| (234) | | (235) | |
| 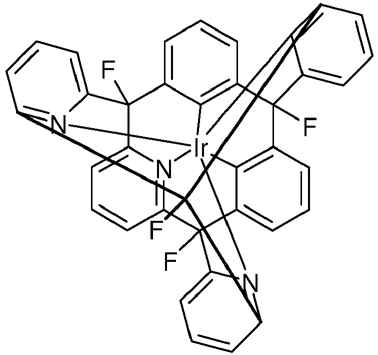 | | 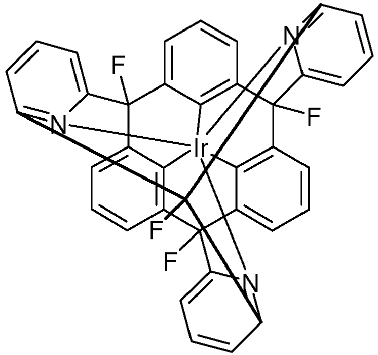 | |
| (236) | | (237) | |
| 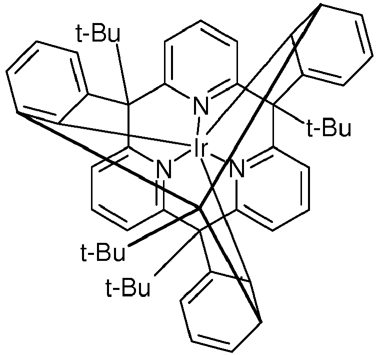 | | 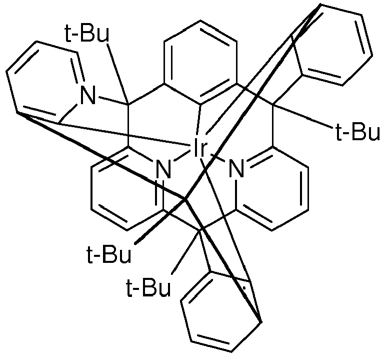 | |
| (238) | | (239) | |
| 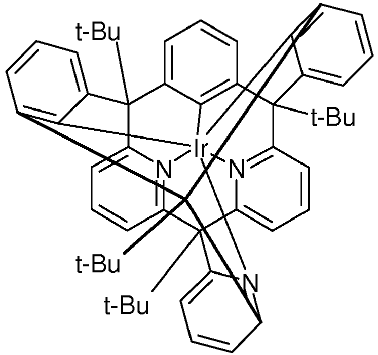 | | 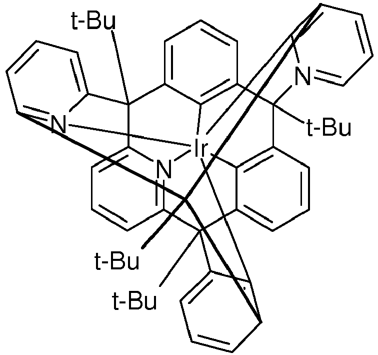 | |
| (240) | | (241) | |
| 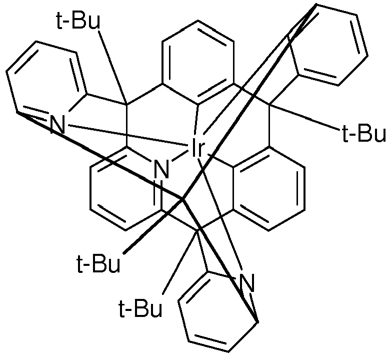 | | 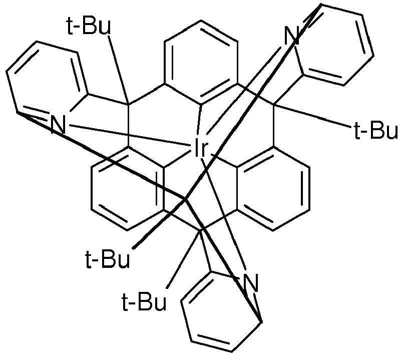 | |
| (242) | | (243) | |
| 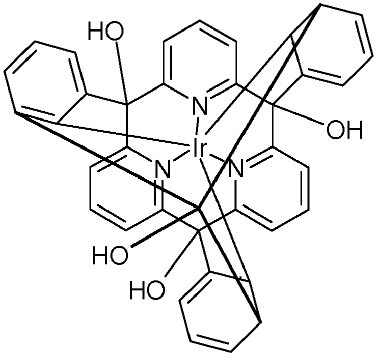 | | 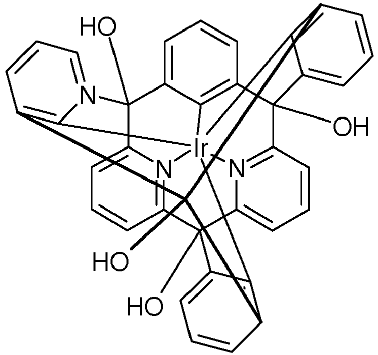 | |
| (244) | | (245) | |
| 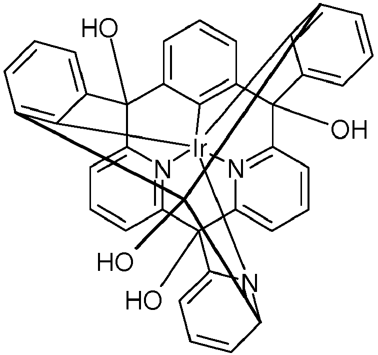 | | 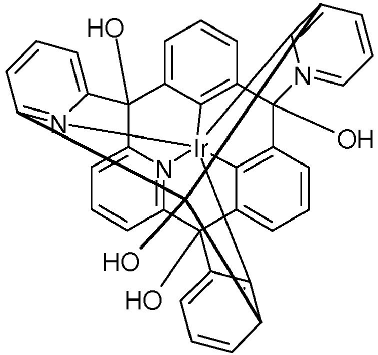 | |
| (246) | | (247) | |
| 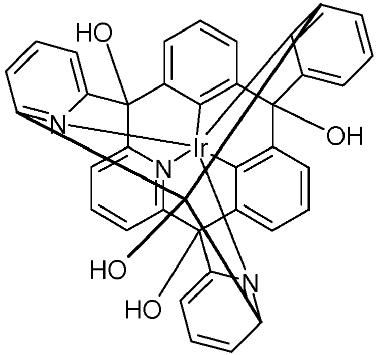 | | 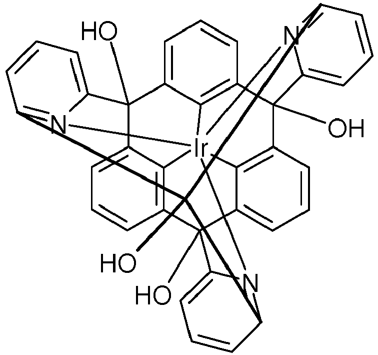 | |
| (248) | | (249) | |
| 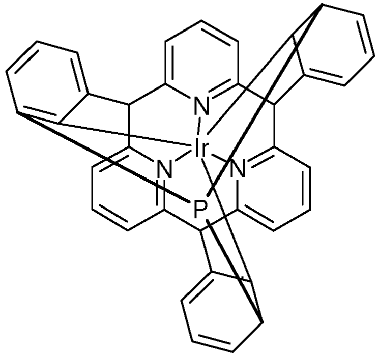 | | 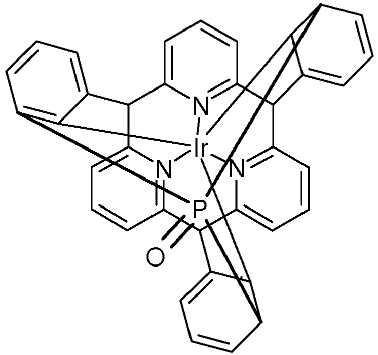 | |
| (250) | | (251) | |
| 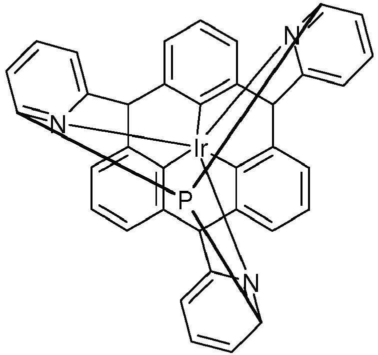 | | 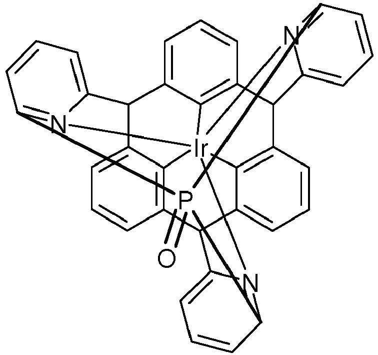 | |
| (252) | | (253) | |
| 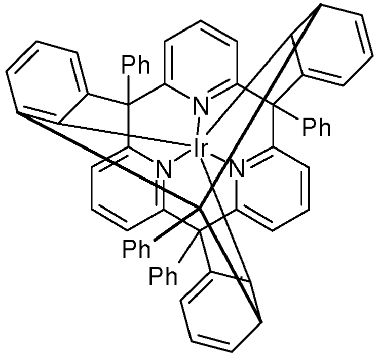 | | 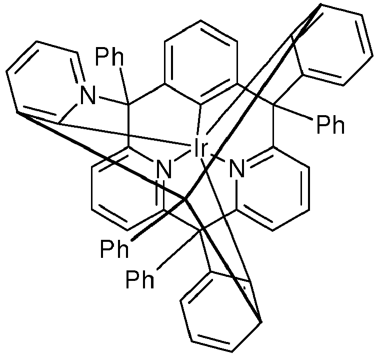 | |
| (254) | | (255) | |
| 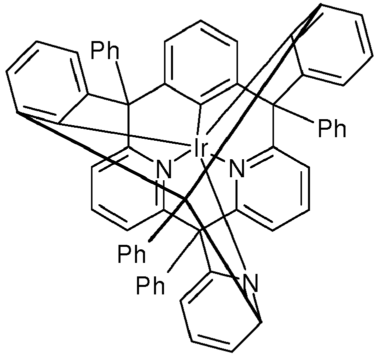 | | 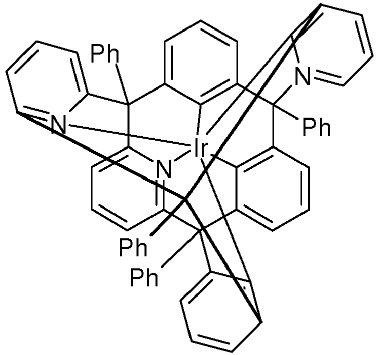 | |
| (256) | | (257) | |
| 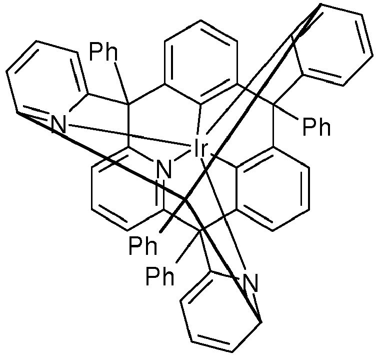 | | 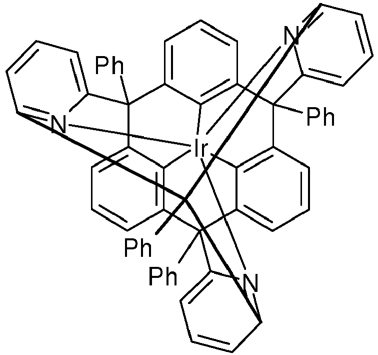 | |
| (258) | | (259) | |
| 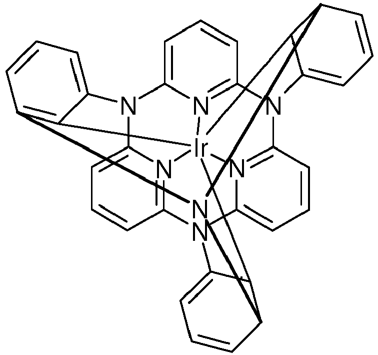 | | 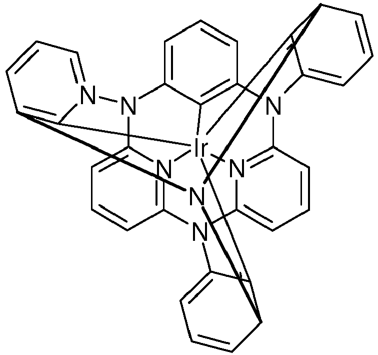 | |
| (260) | | (261) | |
| 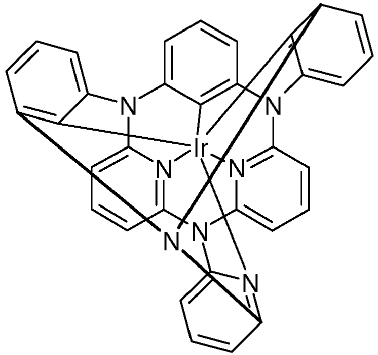 | | 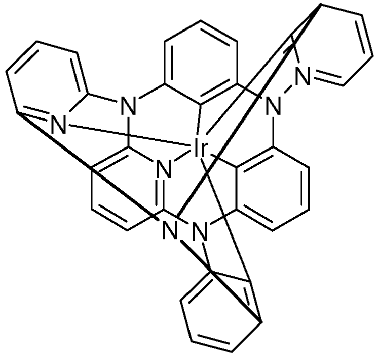 | |
| (262) | | (263) | |
| 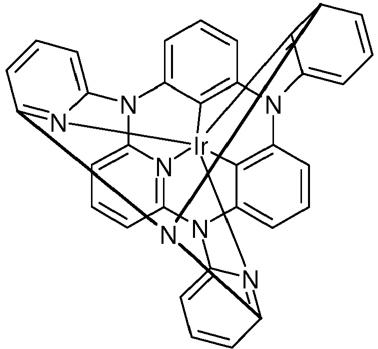 | | 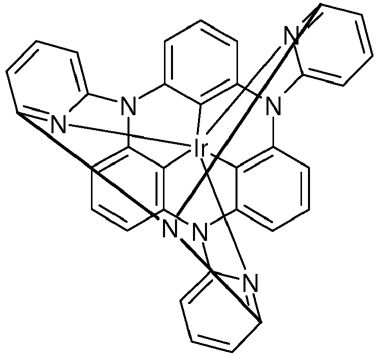 | |
| (264) | | (265) | |
| 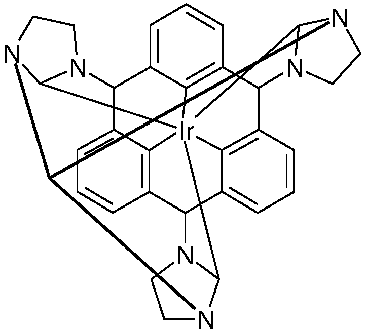 | | 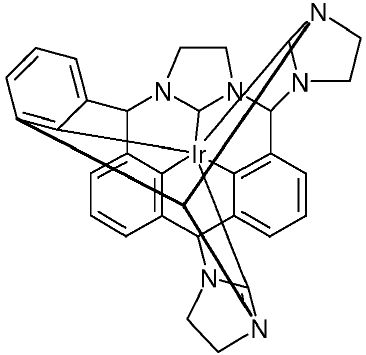 | |
| (266) | | (267) | |
| 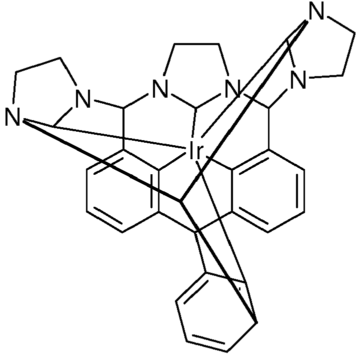 | | 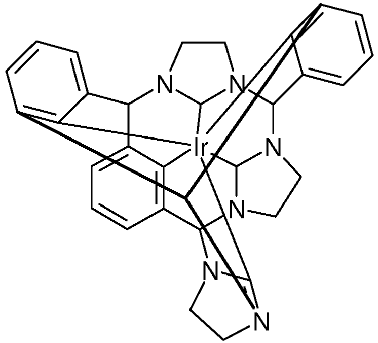 | |
| (268) | | (269) | |
| 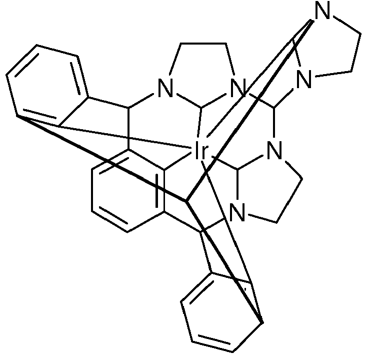 | | 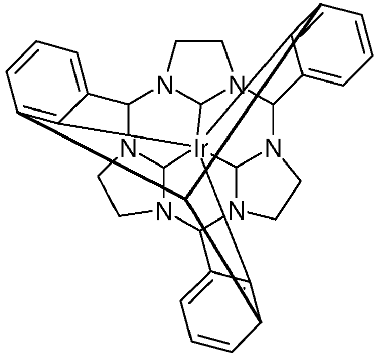 | |
| (270) | | (271) | |
| 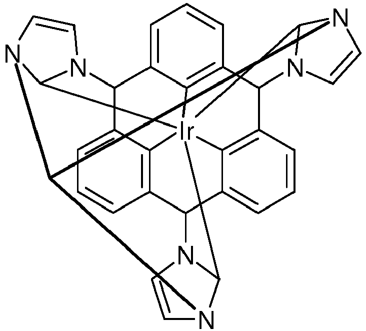 | | 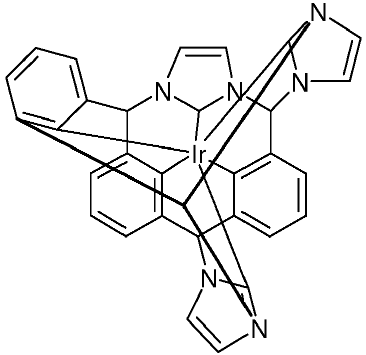 | |
| (272) | | (273) | |
| 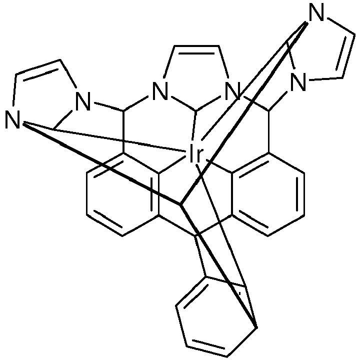 | | 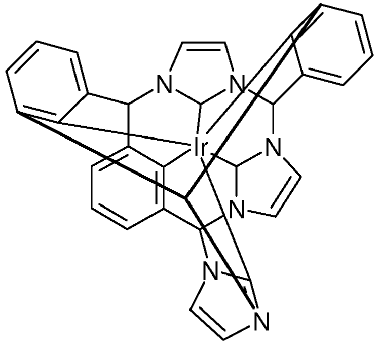 | |
| (274) | | (275) | |
| 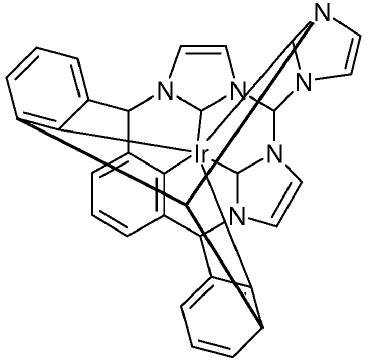 | | 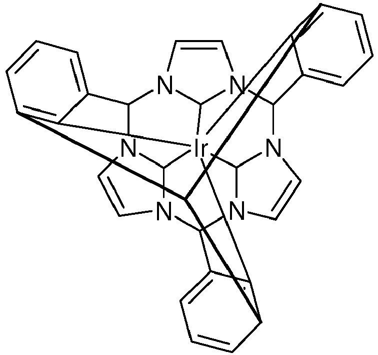 | |
| (276) | | (277) | |
| 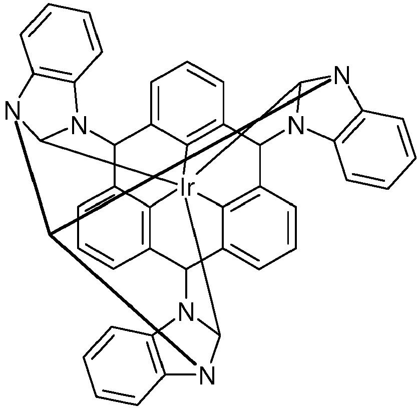 | | 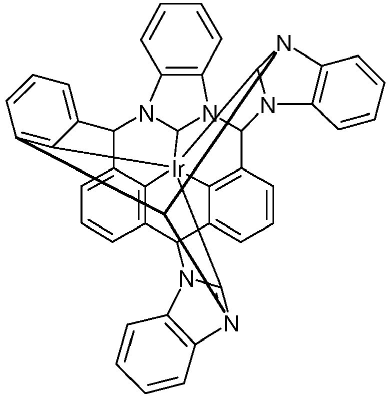 | |
| (278) | | (279) | |
| 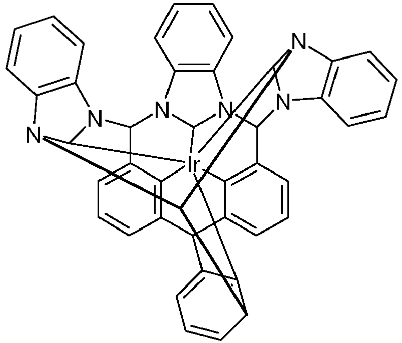 | | 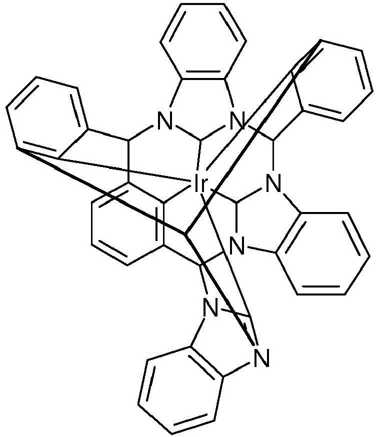 | |
| (280) | | (281) | |
| 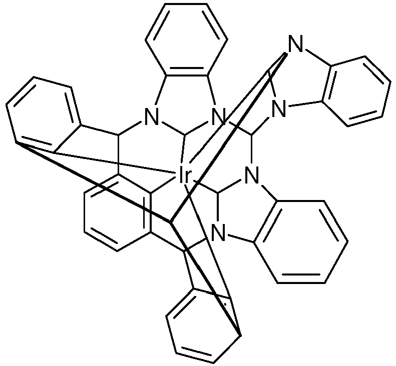 | | 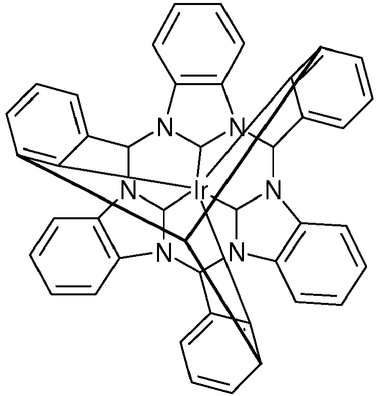 | |
| (282) | | (283) | |
| 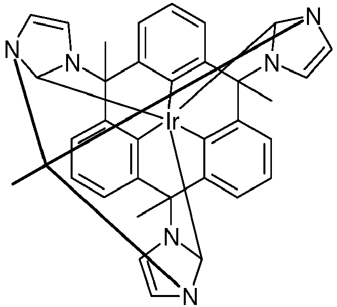 | | 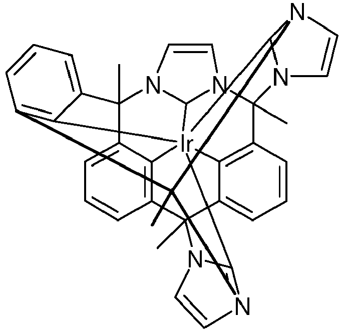 | |
| (284) | | (285) | |
| 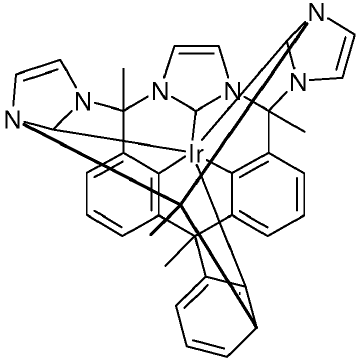 | | 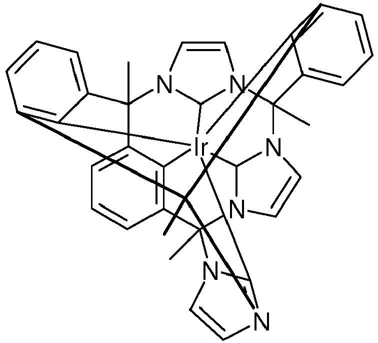 | |
| (286) | | (287) | |
| 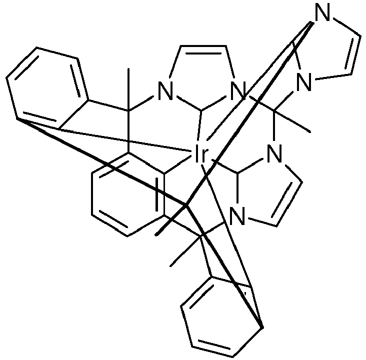 | | 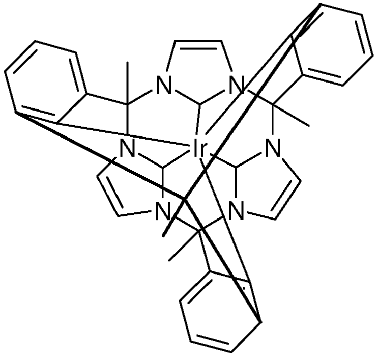 | |
| (288) | | (289) | |
| 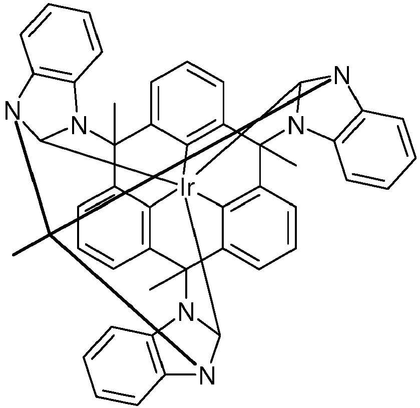 | | 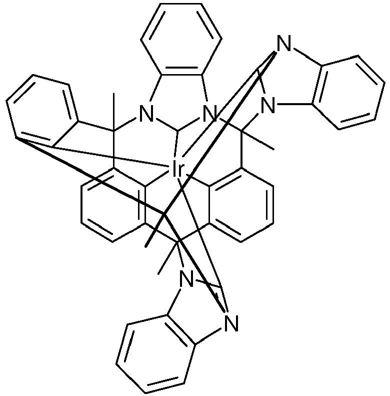 | |
| (290) | | (291) | |
| 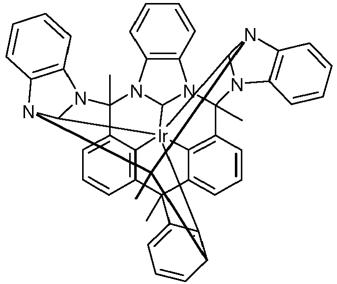 | | 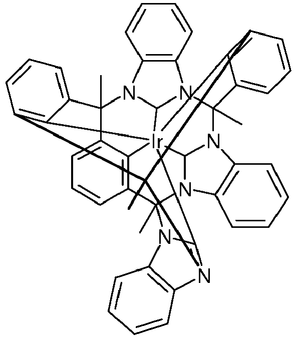 | |
| (292) | | (293) | |
| 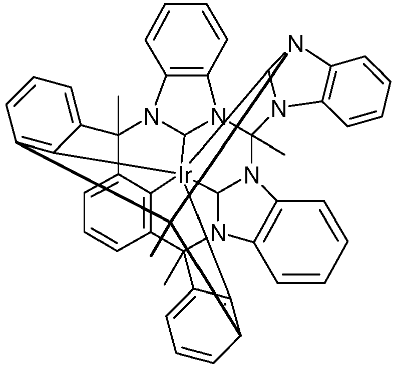 | | 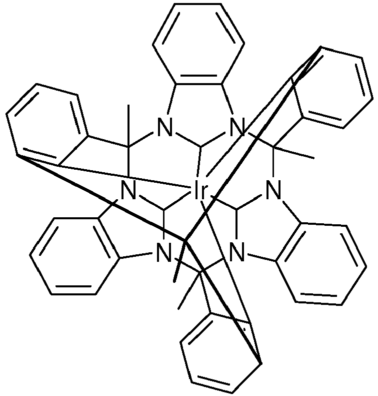 | |
| (294) | | (295) | |
| 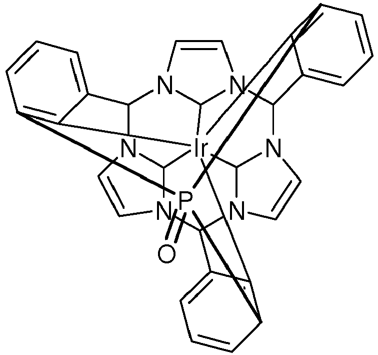 | | 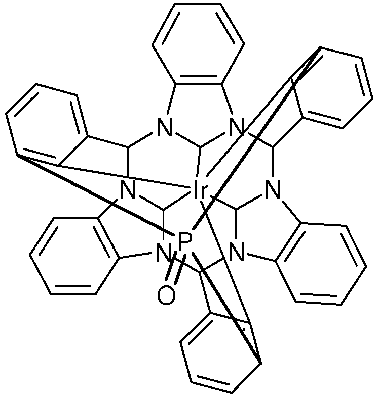 | |
| (296) | | (297) | |
| 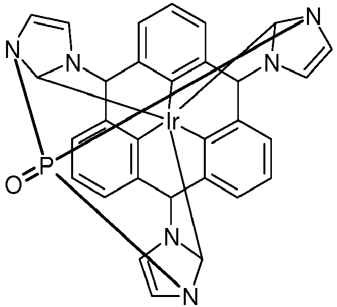 | | 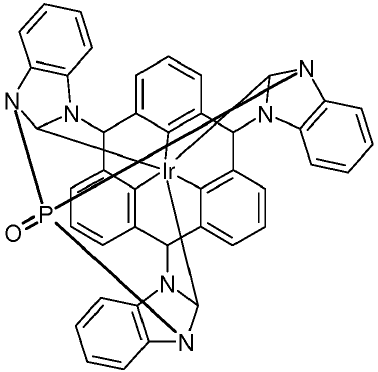 | |
| (298) | | (299) | |
| 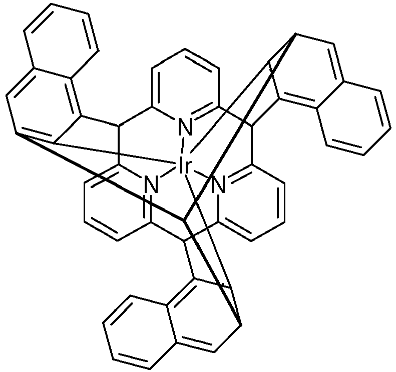 | | 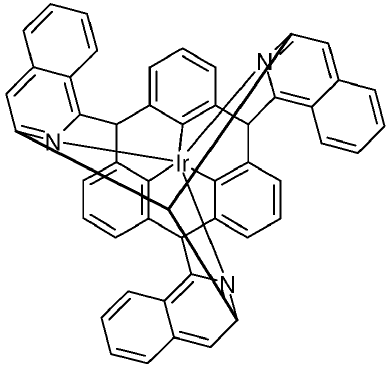 | |
| (300) | | (301) | |
| 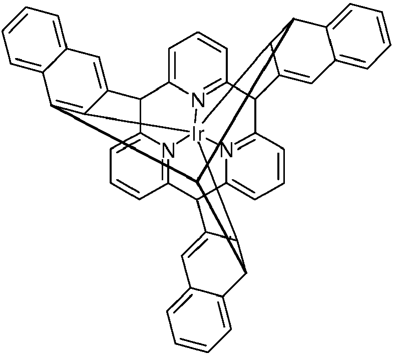 | | 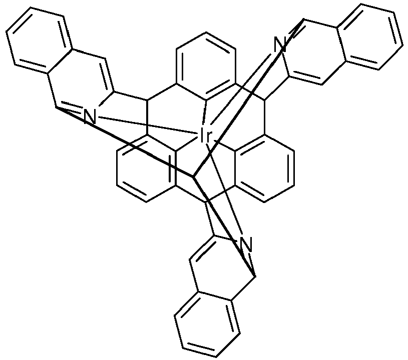 | |
| (302) | | (303) | |
| 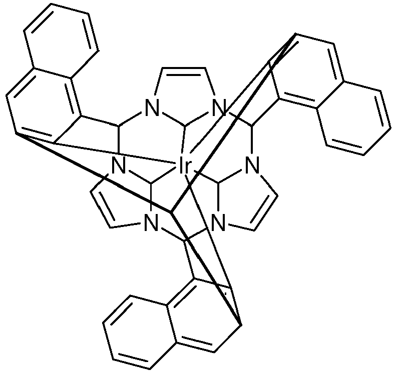 | | 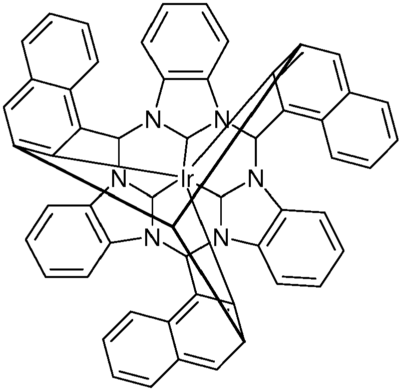 | |
| (304) | | (305) | |
| 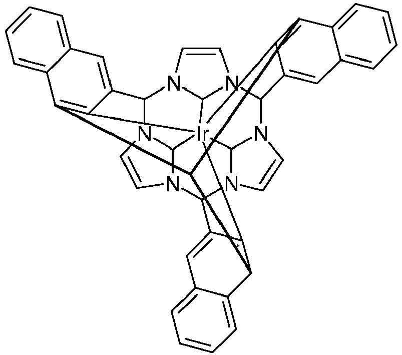 | | 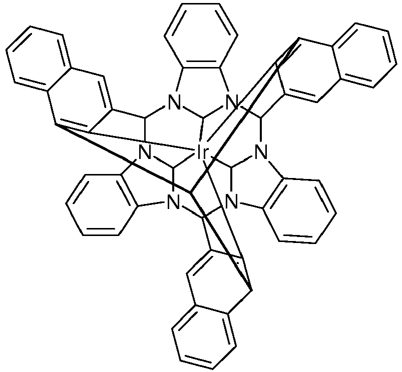 | |
| (306) | | (307) | |

Die oben beschriebenen Komplexe gemäß Formel (3) bis (7) bzw. die oben aufgeführten bevorzugten Ausführungsformen werden in der elektronischen Vorrichtung als aktive Komponente verwendet. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Für diese Funktionen zeigen die erfindungsgemäßen Verbindungen besonders gute Eigenschaften, insbesondere als Emissionsmaterial in organischen Elektrolumineszenzvorrichtungen, wie im Folgenden noch näher ausgeführt wird. Eine bevorzugte Ausführungsform der Erfindung sind daher organische Elektrolumineszenzvorrichtungen.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Excitonenblockierschichten, Ladungserzeugungsschichten und/oder organische oder anorganische p/n-Übergänge. Ebenso können zwischen zwei emittierende Schichten Interlayers eingebracht sein, welche beispielsweise eine Excitonen-blockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten, wobei mindestens eine emittierende Schicht mindestens eine Verbindung gemäß Formel (1) bzw. gemäß Formel (3) bis (7) enthält. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 05/011013).

In einer bevorzugten Ausführungsform der Erfindung enthält die organische elektronische Vorrichtung die Verbindung gemäß Formel (3) bis (7) bzw. die oben aufgeführten bevorzugten Ausführungsformen als emittierende Verbindung in einer emittierenden Schicht. Dies ist insbesondere dann der Fall, wenn das Metall M ein Übergangsmetall ist, insbesondere Iridium.

Wenn die Verbindung gemäß Formel (3) bis (7) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (3) bis (7) und dem Matrixmaterial enthält zwischen 1 und 99 Gew.-%, vorzugsweise zwischen 2 und 90 Gew.-%, besonders bevorzugt zwischen 3 und 40 Gew.-%, insbesondere zwischen 5 und 15 Gew.-% der Verbindung gemäß Formel (3) bis (7) bezogen auf die Gesamtmischung aus Emitter und Matrixmaterial. Entsprechend enthält die Mischung zwischen 99 und 1 Gew.-%, vorzugsweise zwischen 98 und 10 Gew.-%, besonders bevorzugt zwischen 97 und 60 Gew.-%, insbesondere zwischen 95 und 85 Gew.-% des Matrixmaterials bezogen auf die Gesamtmischung aus Emitter und Matrixmaterial.

Geeignete Matrixmaterialien sind Ketone, Phosphinoxide, Sulfoxide und Sulfone, z. B. gemäß WO 04/013080, WO 04/093207, WO 06/005627 oder der nicht offen gelegten Anmeldung DE 102008033943.1, Triarylamine, Carbazolderivate, z. B. CBP (N,N-Biscarbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Azacarbazole, z. B. gemäß EP 1617710, EP 1617711, EP 1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß der nicht offen gelegten Anmeldung DE 102008036982.9, WO 07/063754 oder WO 08/056746, oder Zinkkomplexe, z. B. gemäß EP 652273 oder gemäß der nicht offen gelegten Anmeldung DE 102007053771.0. Weiterhin eignen sich als Matrixmaterialien die Verbindungen gemäß der Formel (1) der vorliegenden Anmeldung, wie unten noch näher ausgeführt.

In einer weiteren bevorzugten Ausführungsform der Erfindung wird die Verbindung gemäß Formel (3) bis (7) bzw. die oben aufgeführten bevorzugten Ausführungsformen als Matrixmaterial für eine emittierende Verbindung in einer emittierenden Schicht eingesetzt. Dies ist insbesondere dann der Fall, wenn das Metall M ein Hauptgruppenmetall ist, insbesondere Aluminium, Gallium oder Indium.

Wenn die Verbindung gemäß Formel (3) bis (7) bzw. die oben aufgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand oder aus einem MLCT-Mischzustand. Im Sinne der vorliegenden Erfindung sollen alle lumineszierenden Übergangsmetallkomplexe der zweiten und dritten Übergangsmetallreihe, insbesondere alle lumineszierenden Iridium- und Platinkomplexe, als Triplettemitter angesehen werden. Die Mischung aus der Verbindung gemäß Formel (3) bis (7) bzw. der oben aufgeführten bevorzugten Ausführungsform und der emittierenden Verbindung enthält dann zwischen 99 und 1 Gew.-%, vorzugsweise zwischen 98 und 10 Gew.-%, besonders bevorzugt zwischen 97 und 60 Gew.-%, insbesondere zwischen 95 und 85 Gew.-% der Verbindung gemäß Formel (3) bis (7) bzw. der oben aufgeführten bevorzugten Ausführungsform bezogen auf die Gesamtmischung aus Emitter und Matrixmaterial. Entsprechend enthält die Mischung zwischen 1 und 99 Gew.-%, vorzugsweise zwischen 2 und 90 Gew.-%, besonders bevorzugt zwischen 3 und 40 Gew.-%, insbesondere zwischen 5 und 15 Gew.-% des Emitters bezogen auf die Gesamtmischung aus Emitter und Matrixmaterial.

Als phosphoreszierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244 oder der nicht offen gelegten Anmeldung DE 102008015526.8 entnommen werden. Weiterhin eignen sich als Emitter die oben aufgeführten Verbindungen gemäß Formel (3) bis (7) bzw. die oben aufgeführten bevorzugten Ausführungsformen. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren bevorzugten Ausführungsform der Erfindung wird die Verbindung gemäß Formel (3) bis (7) bzw. die oben aufgeführten bevorzugten Ausführungsformen als Lochblockiermaterial in einer Lochblockierschicht und/oder als Elektronentransportmaterial in einer Elektronentransportschicht eingesetzt. Dies ist insbesondere dann der Fall, wenn das Metall M ein Hauptgruppenmetall ist, insbesondere ein Alkalimetall, ein Erdalkalimetall, Aluminium, Gallium oder Indium. Dabei kann die emittierende Schicht fluoreszierend oder phosphoreszierend sein.

In einer weiteren bevorzugten Ausführungsform der Erfindung wird die Verbindung gemäß Formel (3) bis (7) bzw. die oben aufgeführten bevorzugten Ausführungsformen als Lochtransportmaterial in einer Lochtransportschicht und/oder als Elektronenblockier- bzw. Exzitonenblockiermaterial in einer Exzitonenblockierschicht eingesetzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Diese Verfahren sind dem Fachmann generell bekannt und können von ihm ohne Probleme auf organische Elektrolumineszenzvorrichtungen enthaltend Verbindungen gemäß Formel (3) bis (7) bzw. die oben aufgeführten bevorzugten Ausführungsformen angewandt werden.

Die bevorzugten Metallkomplexe der Formeln (4) bis (7) und (6a) sind neu. Ein weiterer Gegenstand der vorliegenden Erfindung sind daher die Verbindungen der oben aufgeführten Formeln (4) bis (7) und (6a), wobei die folgende Verbindung von der Erfindung ausgenommen ist:

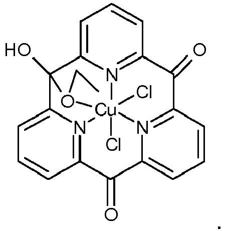

Dabei gelten die oben für die organischen elektronischen Vorrichtungen aufgeführten Bevorzugungen ganz analog auch für die erfindungsgemäßen Metallkomplexe.

Nochmals ein weiterer Gegenstand der vorliegenden Erfindung ist ein Verfahren zur Herstellung der Verbindungen gemäß den Formeln (4) bis (7) bzw. (6a) durch Umsetzung des entsprechenden freien Liganden bzw. gemäß den folgenden Formeln (69) bis (71) mit Metallverbindungen der oben aufgeführten Formeln (66), (67) oder (68).

Ein weiterer Gegenstand der vorliegenden Erfindung sind die Verbindungen gemäß den folgenden Formeln (69) bis (71). Diese Verbindungen sind die freien Liganden der erfindungsgemäßen Metallkomplexe der Formeln (4) bis (6) und sind somit eine wertvolle Zwischenstufe zur Synthese der erfindungsgemäßen Metallkomplexe: 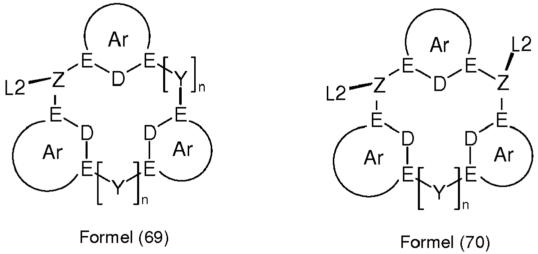 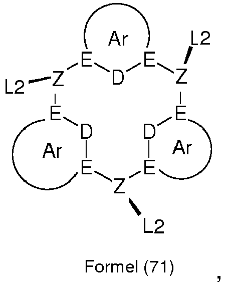 wobei die folgenden Verbindungen von der Erfindung ausgenommen sind:

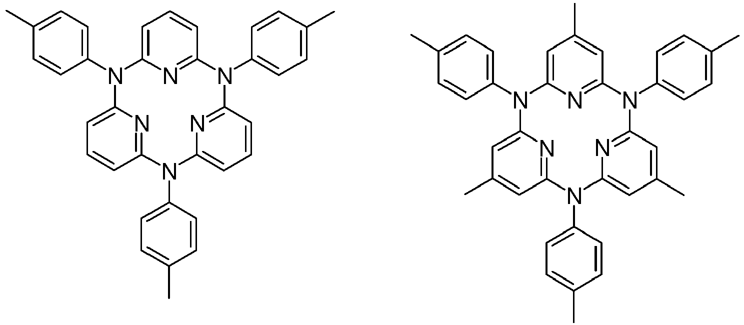

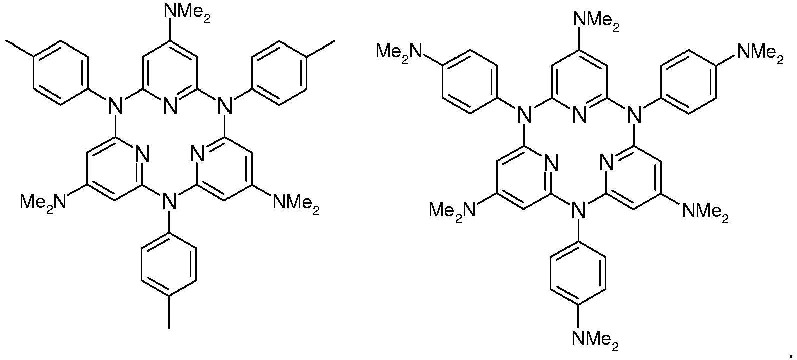

Dabei haben die Symbole und Indizes die oben genannten Bedeutungen, wobei die Gruppen D bzw. L2, wenn sie im Komplex als anionische Gruppen an das Metall M binden, noch zusätzlich jeweils ein Wasserstoffatom tragen. Weiterhin gelten für die freien Liganden ganz analog dieselben Bevorzugungen, wie oben für die Metallkomplexe beschrieben.

Die oben beschriebenen erfindungsgemäßen Verbindungen, insbesondere Verbindungen, welche mit reaktiven Abgangsgruppen, wie Brom, lod, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

Weiterer Gegenstand der Erfindung sind daher Oligomere, Polymere oder Dendrimere enthaltend eine oder mehrere Verbindungen gemäß Formel (3) bis (7), wobei ein oder mehrere Bindungen des Komplexes gemäß Formel (3) bis (7) zum Polymer, Oligomer oder Dendrimer vorhanden sind. Je nach Verknüpfung der Verbindung gemäß Formel (3) bis (7) bildet der Komplex daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein.

Für die Wiederholeinheiten gemäß Formel (3) bis (7) in Oligomeren, Dendrimeren und Polymeren gelten ganz analog dieselben Bevorzugungen wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten Formel (3) bis (7) bevorzugt zu 0.01 bis 50 mol%, besonders bevorzugt im Bereich von 0.1 bis 20 mol% vorhanden sind. Geeignete und bevorzugte Comonomere, welche das Polymergrundgerüst bilden, sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Der Anteil dieser Einheiten insgesamt liegt bevorzugt im Bereich von mindestens 50 mol%. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Weiterhin können die erfindungsgemäßen Metallkomplexe auch weiter funktionalisiert werden und so zu erweiterten Metallkomplexen umgesetzt werden. Hier ist als Beispiel die Funktionalisierung mit Arylboronsäuren gemäß SUZUKI oder mit primären oder sekundären Aminen gemäß HARTWIG-BUCHWALD zu nennen.

Die erfindungsgemäßen organischen elektronischen Vorrichtungen, insbesondere organischen Elektrolumineszenzvorrichtungen, zeichnen sich durch folgende überraschende Vorteile gegenüber dem Stand der Technik aus:
1. Im Gegensatz zu vielen Metallkomplexen gemäß dem Stand der Technik, die der teilweisen oder vollständigen pyrolytischen Zersetzung bei Sublimation unterliegen, weisen die erfindungsgemäßen Verbindungen eine hohe thermische Stabilität auf.
2. Organische Elektrolumineszenzvorrichtungen enthaltend Verbindungen gemäß Formel (3) als emittierende Materialien weisen eine exzellente Lebensdauer auf.
3. Es sind blau phosphoreszierende Komplexe zugänglich, welche eine tiefblaue Emissionsfarbe und bei Verwendung in organischen Elektrolumineszenzvorrichtungen eine hohe Lebensdauer aufweisen. Dies ist ein deutlicher Fortschritt gegenüber dem Stand der Technik, da bislang blau phosphoreszierende Vorrichtungen nur mit schlechten Farbkoordinaten und insbesondere einer schlechten Lebensdauer zugänglich waren.
4. Die erfindungsgemäßen Verbindungen, eingesetzt in organischen Elektrolumineszenzvorrichtungen, führen zu hohen Effizienzen und zu steilen Strom-Spannungs-Kurven.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße Komplexe herstellen und diese in organischen elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die Synthesen der folgenden Liganden 1 bis 4 erfolgt gemäß der angegebenen Literatur:

### Ligand 1:

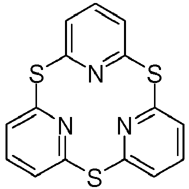

M. Tatazona et al., Macromolecules 1992, 25, 5020.

### Ligand 2:

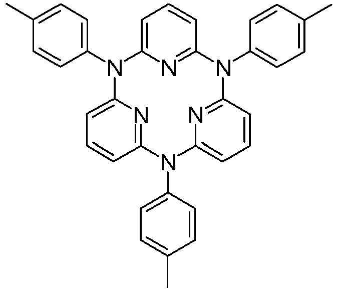

Y. Suzuki et al., Synlett 2005, 2, 263.

### Ligand 3:

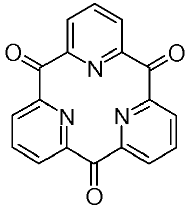

G. R. Newkome et al., Heterocycles 1978, 9 (11), 1555.

### Ligand 4:

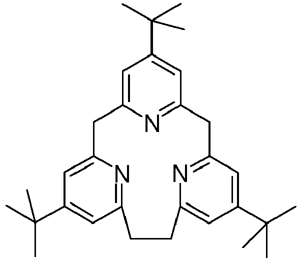

A. N. Vedernikov et al., J. Org. Chem. 2003, 68, 4806.

### Weitere Ligandensynthesen:

### Beispiel 1: Synthese von Ligand 5

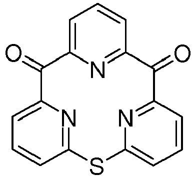

Eine Lösung von 26.8 g (50 mmol) 2,6-Bis[2-(6-bromo-2-pyridinyl)-1,3-dioxolan-2-yl]-pyridin (synthetisiert gemäß G. R. Newkome et al., J. Am. Chem. Soc. 1986, 108(19), 6074) und 2.0 g (50 mmol) wasserfreies Natriumhydrogensulfid in 300 ml 1,2-Propandiol wird 16 h bei 120 °C gerührt. Nach Erkalten gießt man in 500 ml Wasser ein und rührt 12 h nach. Man saugt vom Feststoff ab, nimmt diesen in einem Gemisch aus 50 ml Ethanol und 50 ml konz. Salzsäure auf und kocht 48 h unter Rückfluss. Dann entfernt man das Ethanol im Vakuum, stellt mit festem Kaliumhydroxid alkalisch, extrahiert dreimal mit 50 ml Dichlormethan, wäscht die vereinigten Extrakte mit 100 ml Wasser, trocknet über Magnesiumsulfat und engt zur Trockene ein. Der ölige Rückstand wird an Kieselgel chromatographiert (Laufmittel Aceton / Triethylamin 98:2) und dann aus Aceton umkristallisiert. Ausbeute: 2.2 g (7 mmol), 13.9 %. Reinheit 97 % ig nach ¹H-NMR.

### Beispiel 2: Synthese von Ligand 6

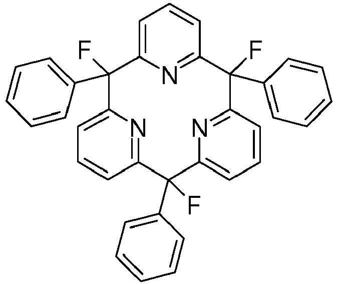

Eine Suspension von 8.3 g (20 mmol) des Komplexes Ligand 3-CuCI (Darstellung: siehe Beispiel 5) in 200 ml THF wird unter gutem Rühren bei -78 °C mit 20 ml (20 mmol) einer Lösung von Kaliumt riethylborhydrid (1 N in THF) versetzt und 10 min. nachgerührt. Anschließend tropft man 40 ml (80 mmol) einer Lösung von Phenyllithium (2 N in Di-n-butylether) zu, rührt 1 h bei -78 °C nach und lässt dann langsam auf Raum temperatur erwärmen. Man versetzt mit 50 ml einer 2 N wässrigen Natriumcyanid-Lösung, rührt 12 h bei Raumtemperatur, trennt die wässrige Phase ab und engt die organische Phase zur Trockene ein. Man löst den Rückstand in 200 ml Chloroform, filtriert von unlöslichen Anteilen ab, tropft eine Lösung von 32.0 g (240 mmol) Dimethylaminoschwefeltrifluorid in 200 ml Chloroform zu und erhitzt 30 min. unter Rückfluss. Nach Erkalten wird tropfenweise mit 50 ml Eiswasser hydrolysiert, dann mit 250 ml 4 N Natronlauge alkalisch gestellt. Die organische Phase wird abgetrennt und über Calciumchlorid getrocknet. Nach Einengen der organischen Phase im Vakuum auf ca. 10 ml wird mit 50 ml Methanol versetzt. Nach 12 h Stehen werden die Kristalle abgesaugt und erneut aus Chloroform / Methanol umkristallisiert. Ausbeute: 5.2 g (9 mmol), 46.8 %. Reinheit 97 %ig nach ¹H-NMR.

### Synthese der Metallkomplexe:

### Beispiel 3: Synthese des Komplexes Ligand 1-Cul

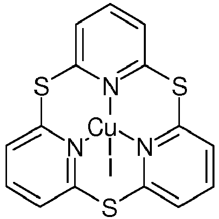

Eine Suspension von 3.3 g (10 mmol) Ligand 1, 1.9 g (10 mmol) Kupfer(I)-iodid und 50 g Glasperlen (4 mm Durchmesser) in 200 ml Dichlormethan wird 24 h bei Raumtemperatur gerührt. Man filtriert von den Glasperlen über ein grobes Sieb ab, engt das Dichlormethan im Vakuum auf ca. 20 ml ein, versetzt tropfenweise mit 50 ml Ethanol, rührt 2 h nach, filtriert von den orange-farbenen Kristallen ab, wäscht diese zweimal mit je 20 ml Ethanol und kristallisiert aus DMSO um. Ausbeute: 4.7 g (9.1 mmol), 90.1 %. Reinheit > 99 %ig nach ¹H-NMR.

### Beispiel 4: Synthese des Komplexes Ligand 2-CuI

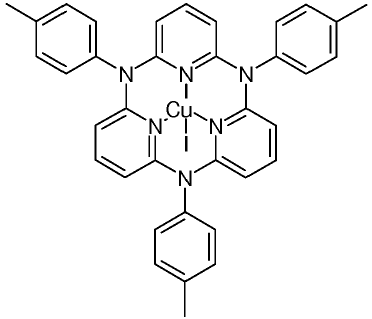

Darstellung analog zu Beispiel 3, wobei 5.5 g (10 mmol) Ligand 2 eingesetzt werden. Ausbeute: 5.5 g (7.5 mmol), 74.6 %. Reinheit > 99 %ig nach ¹H-NMR.

### Beispiel 5: Synthese des Komplexes Ligand 3-CuCl

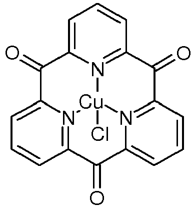

Darstellung analog zu Beispiel 3, wobei 3.2 g (10 mmol) Ligand 3 eingesetzt werden. Ausbeute: 3.9 g (9.3 mmol), 93.3 %. Reinheit > 98 %ig nach ¹H-NMR.

### Beispiel 6: Synthese des Komplexes Ligand 4-Mo(CO)₃

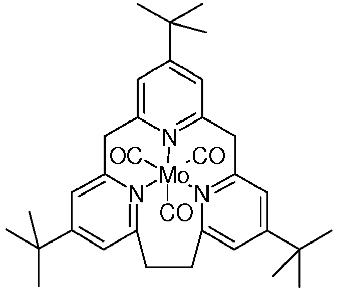

Eine Lösung von 2.3 g (5 mmol) Ligand 4 und 1.4 g (5 mmol) Cycloheptatrien-molybdän-tricarbonyl in 50 ml Toluol wird 6 h unter Rückfluss erhitzt, anschließend im Vakuum auf ca. 10 ml eingeengt und unter Rühren mit 50 ml Hexan versetzt. Nach 12 h werden die Kristalle abgesaugt und zweimal mit je 10 ml Hexan gewaschen. Ausbeute: 2.6 g (4.1 mmol), 81.8 %. Reinheit > 99 %ig nach ¹H-NMR.

### Beispiel 7: Synthese des Komplexes Ligand 5-W(CO)₃

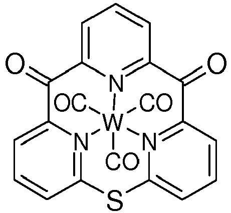

Eine Lösung von 1.6 g (5 mmol) Ligand 5, 1.8 g (5 mmol) Wolframhexacarbonyl und 10 mg Palladium(II)oxid in 50 ml Toluol wird 48 h unter Rückfluss erhitzt. Nach Erkalten wird die Lösung über Celite filtriert und das Filtrat eingeengt. Der Rückstand wird aus Dichlormethan / Hexan umkristallisiert. Ausbeute: 1.6 g (2.7 mmol), 54.0 %. Reinheit > 99 %ig nach ¹H-NMR.

### Beispiel 8: Synthese des Komplexes Ligand 6-Ir

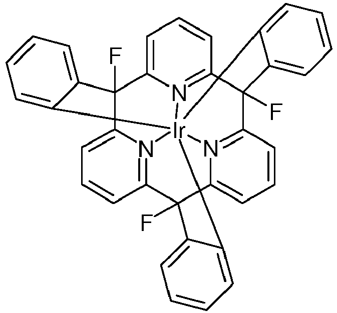

Ein Gemisch aus 2.8 g (5 mmol) Ligand 6, 1.5 g (5 mmol) Iridium(III)chlorid Hydrat und 10 ml Ethylenglykol wird 48 h bei 190 °C gerührt. Nach Erkalten wird mit 100 ml Wasser versetzt, der braune Niederschlag wird abfiltriert, getrocknet und dann an neutralem Alox mit Dichlormethan / THF (1:1) chromatographiert. Ausbeute: 820 mg (1.1 mmol), 22.0 %. Reinheit > 99 %ig nach ¹H-NMR.

### Beispiel 9: Ligand 7-Ir

### a) Synthese der Vorstufe des Liganden:

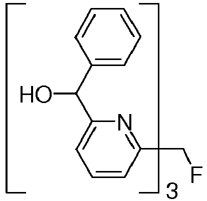

Eine Lösung von 53.6 g (100 mmol) Tris-(6-brompyrid-2-yl)fluormethan [760177-68-2] in 1500 ml THF wird unter gutem Rühren bei -78 °C tropfenweise während 10 min. mit 120 ml n-Butyllithium (2.5 M in Hexan) versetzt, weitere 30 min. bei -78 °C gerührt und da nn tropfenweise mit 32.0 ml (315 mmol) Benzaldehyd versetzt. Nach Erwärmen auf Raumtemperatur wird das THF im Vakuum entfernt, der Rückstand wird in 500 ml Dichlormethan aufgenommen, zweimal mit 200 ml Wasser gewaschen, die organische Phase über Magnesiumsulfat getrocknet und dann im Vakuum komplett eingeengt. Ausbeute: 54.3 g (93 mmol), 93.0 %, ca. 90 %ig nach ¹H-NMR (Diastereomerengemisch). Das so erhaltene zähe Öl wird ohne weitere Reinigung eingesetzt.

### b) Synthese von Ligand 7-Ir:

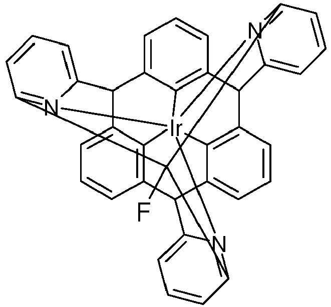

Eine Mischung von 5.8 g (10 mmol) der Ligandenvorstufe aus Beispiel 9a) und 2.4 g (5 mmol) Natrium[bis-acetylacetonato-di-chloro-iridium] in 100 ml Ethylenglykol wird unter einem kontinuierlichen Argonstrom 16 h bei 80 °C und dann 24 h bei 140°C gerührt. Nach Erk alten wird die Suspension mit 200 ml Wasser versetzt, der braune Feststoff wird abgesaugt, mit Wasser gewaschen und anschließend bei 70 °C im Stickstoffstrom getrocknet. Der so erhaltene Feststoff wird in 50 ml Eisessig suspendiert, die Suspension wird mit 0.5 ml Schwefelsäure versetzt und anschließend 2 h unter Rückfluss erhitzt. Nach Erkalten wird der Eisessig im Vakuum entfernt, der Rückstand wird in 500 ml Dichlormethan aufgenommen, die organische Phase wird einmal mit 200 ml gesättigter Natriumhydrogencarbonat-Lösung, einmal mit 200 ml Wasser gewaschen und dann über Magnesiumsulfat getrocknet. Nach Entfernen des Dichlormethans wird der Rückstand an Kieselgel mit THF chromatographiert. Der so erhaltene gelbe Feststoff wird dann zweimal im Hochvakuum (p = 10⁻⁵ mbar, T = 340 °C) sublimiert. Ausbeute: 270 mg (0. 37 mmol), 7.5 %, 99.8 %ig nach HPLC.

### Beispiel 10: Herstellung und Charakterisierung von organischen Elektrolumineszenzvorrichtungen

Die Herstellung von LEDs erfolgt nach dem im Folgenden skizzierten allgemeinen Verfahren. Dieses muss natürlich im Einzelfall auf die jeweiligen Gegebenheiten (z. B. Schichtdickenvariation, um optimale Effizienz bzw. Farbe zu erreichen) angepasst werden.

### Allgemeines Verfahren zur Herstellung der OLEDs:

Nachdem man die ITO-beschichteten Substrate (z. B. Glasträger, PET-Folie) auf die richtige Größe zugeschnitten hat, werden sie in mehreren Reinigungsschritten im Ultraschallbad gereinigt (z. B. Seifenlösung, Millipore-Wasser, Isopropanol). Zur Trocknung werden sie mit einer N₂-Pistole abgepustet und in einem Exsikkator gelagert. Vor der Bedampfung mit den organischen Schichten werden sie mit einem Ozon-Plasma-Gerät für ca. 20 Minuten behandelt. Es kann sich empfehlen, als erste organische Schicht eine polymere Lochinjektionsschicht zu verwenden. Dies ist in der Regel ein konjugiertes, leitfähiges Polymer, wie z. B. ein Polyanilinderivat (PANI) oder eine Polythiophenderivat (z. B. PEDOT, BAYTRON P™ von BAYER). Diese wird dann durch Spin-Coaten (Lackschleudern) aufgebracht. Die organischen Schichten werden der Reihe nach durch Aufdampfen in einer Hochvakuumanlage aufgebracht. Dabei werden die Schichtdicke der jeweiligen Schicht und die Bedampfungsrate über einen Schwingquarz verfolgt bzw. eingestellt. Es können auch einzelne Schichten aus mehr als einer Verbindung bestehen, d. h. in der Regel ein Wirtsmaterial (host) mit einem Gastmaterial (guest) dotiert sein. Dies wird durch Co-Verdampfung aus zwei bzw. mehreren Quellen erzielt. Auf die organischen Schichten wird noch eine Elektrode aufgebracht. Dies geschieht in der Regel durch thermisches Verdampfen (Balzer BA360 bzw. Pfeiffer PL S 500). Anschließend wird die durchsichtige ITO-Elektrode als Anode und die Metallelektrode als Kathode kontaktiert, und es werden die Device-Parameter bestimmt.

Analog dem o. g. allgemeinen Verfahren, werden OLEDs mit folgendem Aufbau 1 erzeugt:

| | |
|---|---|
| PEDOT | 20 nm (aus Wasser aufgeschleudert; PEDOT bezogen von BAYER AG; Poly-[3,4-ethylendioxy-2,5-thiophen] |
| HIM1 | 20 nm 2,2',7,7'-Tetrakis(di-p-tolylamino)-spiro-9,9'-bifluoren (aufgedampft) |
| NPB | 20 nm 4,4'-Bis(1-naphthyl-phenyl-amino)biphenyl (aufgedampft) |
| mCP | 20 nm 1,3-Bis(N-carbazolyl)benzol (aufgedampft) |
| dotiert mit 10 % | |
| Triplett-Emitter | erfindungsgemäße Beispiele s. Tabelle |
| BCP | 5 nm 2,9-Dimethyl-4,7-diphenyl-1,10-phenanthrolin (aufgedampft) |
| AlQ₃ | 30 nm (aufgedampf) |
| Li / Al | 5 nm LiF, darauf 150 nm Al als Kathode. |

Diese noch nicht optimierte OLED wird standardmäßig charakterisiert. In Tabelle sind die Effizienz und die Spannung bei 500 cd / m² sowie die Farbe aufgeführt.

| Bsp. | Emitter | Effizienz [cd/A] bei 500 cd / m² | Spannung [V] bei 500 cd / m² | Farbe CIE x / y |
|---|---|---|---|---|
| 11 | Beispiel 6 (Ligand 4-Mo(CO)3) | 17.8 | 6.5 | 0.28 / 0.61 |
| 12 | Beispiel 9 (Ligand 7-Ir) | 34.6 | 4.8 | 0.32 / 0.64 |

Analog dem o. g. allgemeinen Verfahren, werden weiterhin OLEDs mit folgendem Aufbau 2 erzeugt:

| | |
|---|---|
| PEDOT | 20 nm (aus Wasser aufgeschleudert; PEDOT bezogen von BAYER AG; Poly-[3,4-ethylendioxy-2,5-thiophen] |
| PVK | 60 nm (aus Chlorbenzol augeschleudert, PVK Mw = 1,100,000 bezogen von Aldrich, Lösung enthaltend 5 Gew.% Emitter nach Beispiel 4), |
| Ba / Ag | 10 nm Ba / 150 nm Ag als Kathode. |

Diese noch nicht optimierte OLED wird standardmäßig charakterisiert. In Tabelle sind die Effizienz und die Spannung bei 500 cd / m² sowie die Farbe aufgeführt.

| Bsp. | Emitter | Effizienz [cd/A] bei 500 cd / m² | Spannung [V] bei 500 cd / m² | Farbe CIE x / y |
|---|---|---|---|---|
| 10 | Beispiel 4 (Ligand 2-CuI) | 5.4 | 7.2 | 0.70 / 0.30 |

## Patentansprüche

1. Elektronische Vorrichtung, enthaltend mindestens einen Metallkomplex gemäß Formel (3), 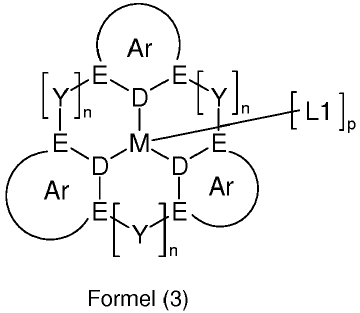 wobei für die verwendeten Symbole und Indizes gilt:
M ist ein Übergangsmetall, ein Lanthanid, ein Alkalimetall, ein Erdalkalimetall oder ein Hauptgruppenmetall der dritten oder vierten Hauptgruppe;
D ist bei jedem Auftreten gleich oder verschieden C, N, P, C-O⁻, C-S⁻, C-NR₂ oder C-PR₂, wobei die letzten vier genannten Gruppen über O, S, N bzw. P als exocyclische Donoratome an das Metall binden, oder C-N=C, wobei diese Gruppe über den Kohlenstoff der exocyclischen Isonitrilgruppe an das Metall bindet;
E ist bei jedem Auftreten gleich oder verschieden C oder N;
Ar ist bei jedem Auftreten gleich oder verschieden eine Gruppe, die zusammen mit der Gruppe E-D-E eine Aryl- oder Heteroarylgruppe mit 5 bis 40 aromatischen Ringatomen aufspannt und die durch einen oder mehrere Reste R substituiert sein kann; oder Ar ist, falls D für ein Carben-Kohlenstoffatom steht, eine Gruppe, die zusammen mit der Gruppe E-D-E eine cyclische gesättigte Gruppe mit 5 bis 10 Ringatomen darstellt;
Y ist gleich oder verschieden bei jedem Auftreten BR¹, B(R¹)₂⁻, C(R¹)⁻, C(R¹)_{2,} Si(R¹)⁻, Si(R¹)₂, C(=O), C(=NR), N⁻, NR¹, N(R¹)₂⁺, PR¹, P(R¹)₂⁺, AsR¹, As(R¹)₂⁺, P(=O)R¹, As(=O)R¹, P(=S)R¹, As(=S)R¹, O, S, S(R¹)⁺, Se, Te, S(=O), S(=O)₂, Se(=O), Se(=O)₂, Te(=O) oder Te(=O)₂;
R ist bei jedem Auftreten gleich oder verschieden H, Deuterium, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination dieser Systeme; dabei können zwei oder mehrere dieser Substituenten R auch miteinander ein mono- oder polycyclisches, aliphatisches, aromatisches und/oder benzoannelliertes Ringsystem bilden;
R¹ ist gleich oder verschieden bei jedem Auftreten R oder eine Gruppe L2;
R² ist bei jedem Auftreten gleich oder verschieden H, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches oder aromatisches Ringsystem bilden;
L2 ist bei jedem Auftreten gleich oder verschieden eine Donorgruppe mit 1 bis 40 C-Atomen, die eine weitere Bindung bzw. Koordination an das Metall M eingehen kann und die durch einen oder mehrere Reste R substituiert sein kann;
n ist bei jedem Auftreten gleich oder verschieden 0, 1, 2, 3, 4, 5 oder 6, wobei n = 0 bedeutet, dass die Gruppe Y nicht vorhanden ist und eine Einfachbindung zwischen den beiden Gruppen L vorliegt, mit der Maßgabe, dass nicht alle Indizes n gleichzeitig für 0 stehen können;
L1 ist bei jedem Auftreten gleich oder verschieden ein mono-, bi-, tri-, tetra-, penta- oder hexadentater Ligand, der an das Metall M bindet;
p ist 0, 1, 2, 3, 4, 5, 6, 7, 8 oder 9.

2. Elektronische Vorrichtung nach Anspruch 1, enthaltend Anode, Kathode und mindestens eine Schicht, welche mindestens eine Verbindung der Formel (1) enthält, ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), bevorzugt organische Elektrolumineszenzvorrichtungen.

3. Elektronische Vorrichtung nach Anspruch 1 oder 2, **dadurch gekennzeichnet, dass** M für für Zirconium, Hafnium, Molybdän, Wolfram, Rhenium, Ruthenium, Osmium, Rhodium, Iridium, Palladium, Platin, Kupfer, Silber, Gold, Scandium, Yttrium, Lanthan, Aluminium, Gallium oder Indium steht.

4. Elektronische Vorrichtung nach einem oder mehreren der Ansprüche 1 bis 3, enthaltend eine Verbindung gemäß den Formeln (4), (5), (6), (6a) oder (7), 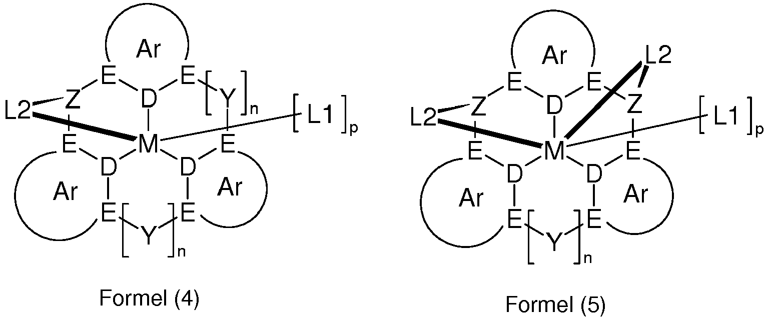 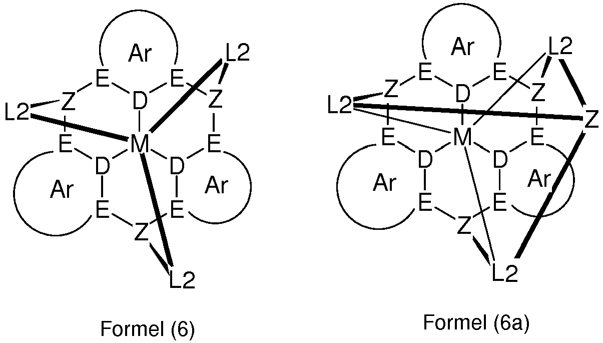 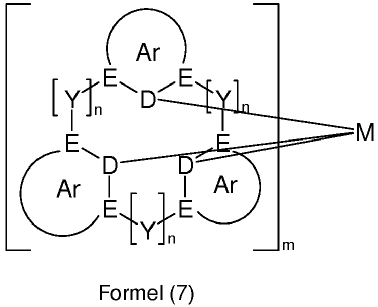 wobei Z in den Formeln (4) bis (6) gleich oder verschieden bei jedem Auftreten für B, B(R¹)⁻, C⁻, CR¹, SiR¹, N, P, As, P(=O), As(=O), P(=S) oder As(=S) steht, m für 2, 3 oder 4 steht und die weiteren Symbole und Indizes die in Anspruch 1 aufgeführten Bedeutungen haben.

5. Elektronische Vorrichtung nach einem oder mehreren der Ansprüche 1 bis 4, **dadurch gekennzeichnet, dass** die Aryl- oder Heteroarylgruppe, die durch Ar zusammen mit der Gruppe E-D-E in Verbindungen gemäß Formel (3) bis (7) aufgespannt wird, für eine Gruppe der folgenden Formel (8) bis (20) steht, wobei jeweils durch die gestrichelte Bindung die Bindung dieser Gruppe im Liganden angedeutet wird, also die Bindung zu den Gruppen Y, und wobei * jeweils die Position der Koordination an das Metall M kennzeichnet: 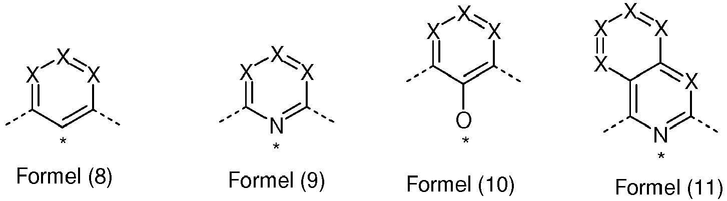 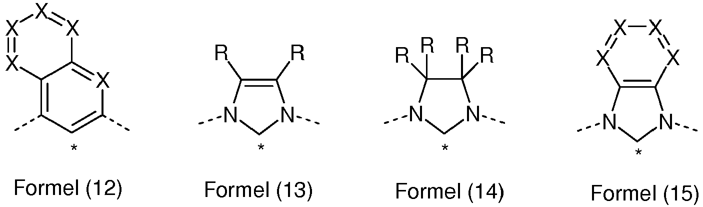 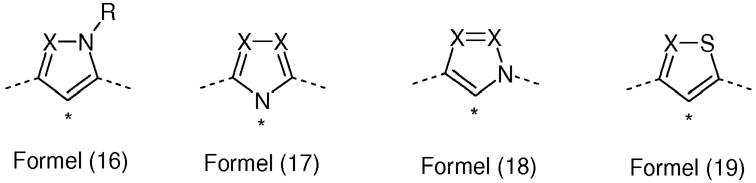 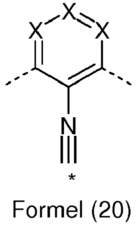 wobei die verwendeten Symbole die in Anspruch 1 aufgeführte Bedeutung haben und X, gleich oder verschieden bei jedem Auftreten, für CR oder N steht mit der Maßgabe, dass maximal drei Symbole X in jeder Gruppe für N stehen.

6. Organische elektronische Vorrichtung nach einem oder mehreren der Ansprüche 1 bis 5, **dadurch gekennzeichnet, dass** die Gruppe L2 eine Aryl- oder Heteroarylgruppe darstellt und ausgewählt ist aus Gruppen der Formel (21) bis (49), wobei jeweils durch die gestrichelte Bindung die Bindung dieser Gruppe im Liganden angedeutet wird, also die Bindung zu der Gruppe Z, wobei * jeweils die Position der Koordination an das Metall M kennzeichnet und wobei die verwendeten Symbole die in Anspruch 1 aufgeführten Bedeutungen haben: 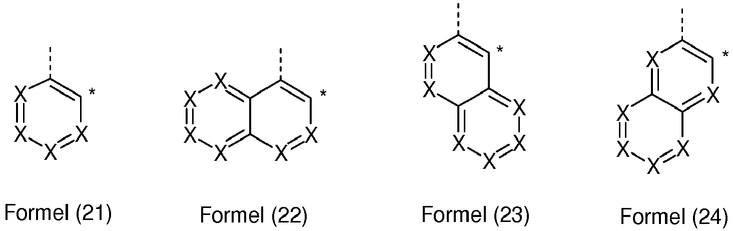 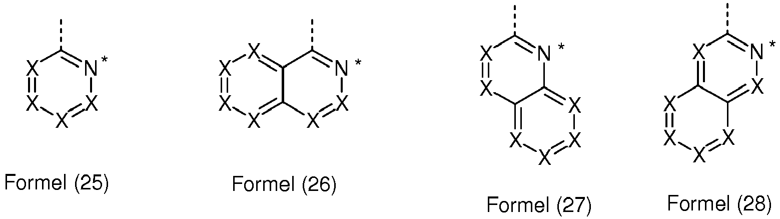 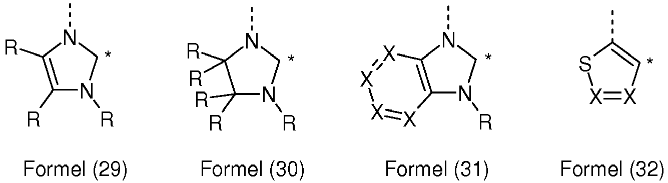 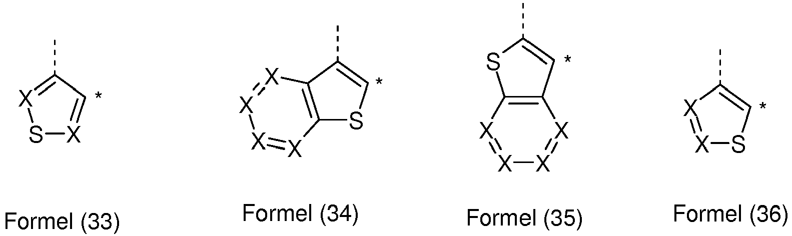 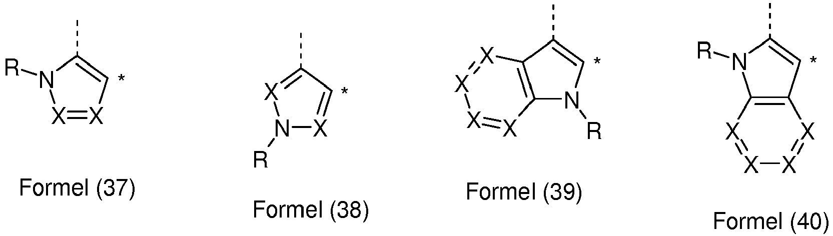 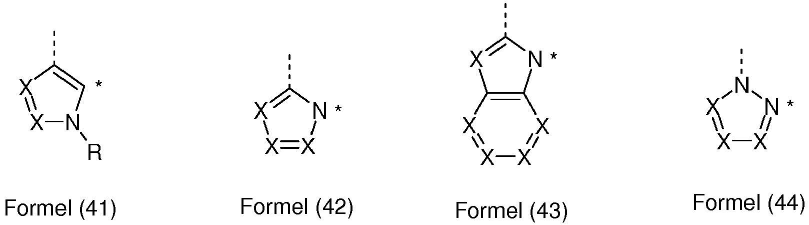 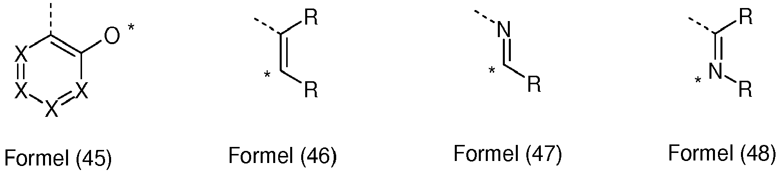 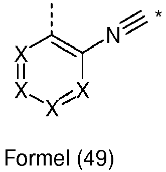 oder dass L2 eine neutrale oder anionische Donorgruppe darstellt, ausgewählt aus den Kohlenstoff-haltigen Donorgruppen der aliphatischen oder aromatischen Acetylide oder der aliphatischen oder aromatischen Isonitrile, der Stickstoff-haltigen Donorgruppen der aliphatischen Amine, aliphatischen cyclischen Amine, Nitrile, Amide, Imide und Imine, die jeweils mit Gruppen R substituiert oder unsubstituiert sein können, den Phosphor-haltigen Donorgruppen PF₂, P(NR₂)₂, wobei R gleich oder verschieden bei jedem Auftreten für eine C₁-C₂₀-Alkylgruppe oder eine Aryl- bzw. Heteroarylgruppe steht, Alkyl-, Aryl- oder gemischte Alkylarylphosphine, Alkylhalogen-, Arylhalogen- oder gemischte Alkylarylhalogenphosphine, wobei das Halogen jeweils F, Cl, Br oder I sein kann, Alkyl-, Aryl- oder gemischte Alkylarylphosphite oder Phosphaaromaten, die jeweils mit Gruppen R substituiert oder unsubstituiert sein können, der Sauerstoff-haltigen Donorgruppen der Alkohole, Alkoholate, offenkettigen oder cyclischen, aliphatischen oder aromatischen Ether, Sauerstoffheterocyclen, Aldehyde, Ketone, Phosphinoxidgruppen, Phosphate, Phosphonate, Borate, Silicate, Sulfoxidgruppen, Carboxylate, Phenole, Phenolate, Oxime, Hydroxamate, β-Ketoketonate, β-Ketoester und β-Diester, die jeweils mit Gruppen R substituiert oder unsubstituiert sein können, der Schwefel-haltigen Donorgruppen der aliphatischen oder aromatischen Thiole und Thiolate, offenkettigen oder cyclischen Thioethern, Thiocarbonylgruppen, Phosphinsulfide und Thiocarboxylate, die jeweils mit Gruppen R substituiert oder unsubstituiert sein können, oder aus diesen Gruppen gebildete zweizähnig chelatisierende Gruppen.

7. Elektronische Vorrichtung nach einem oder mehreren der Ansprüche 1 bis 6, **dadurch gekennzeichnet, dass** die Liganden L1 ausgewählt sind aus Kohlenmonoxid, Isonitrilen, Aminen, Iminen, Diiminen, Phosphinen, Phosphiten, Arsinen, Stibinen, stickstoffhaltigen Heterocyclen, Hydrid, Deuterid, den Halogeniden F, Cl, Br und I, Alkylacetyliden, Arylacetyliden, Cyanid, Cyanat, Isocyanat, Thiocyanat, Isothiocyanat, aliphatischen oder aromatischen Alkoholaten, aliphatischen oder aromatischen Thioalkoholaten, Amiden, Carboxylaten, anionischen, stickstoffhaltigen Heterocyclen, O²⁻, S²⁻, Nitrene, N³⁻, Diaminen, Heterocyclen enthaltend zwei Stickstoffatome, Diphosphinen, 1,3-Diketonaten abgeleitet von 1,3-Diketonen, 3-Ketonaten abgeleitet von 3-Ketoestern, Carboxylaten abgeleitet von Aminocarbonsäuren, Salicyliminaten abgeleitet von Salicyliminen, Dialkoholaten abgeleitet von Dialkoholen, Boraten stickstoffhaltiger Heterocyclen, bidentate monoanionische Liganden, welche mit dem Metall einen cyclometallierten Fünfring mit mindestens einer Metall-Kohlenstoff-Bindung aufweisen, η⁵-Cyclopentadienyl, η⁵-Pentamethylcyclopentadienyl, η⁶-Benzol, η⁷-Cycloheptatrienyl, welche jeweils auch durch R substituiert sein können, 1,3,5-cis-Cyclohexanderivate, 1,1,1-Tri(methylen)methanderivate und 1,1,1-trisubstituierte Methane.

8. Elektronische Vorrichtung nach einem oder mehreren der Ansprüche 1 bis 8, enthaltend mindestens eine Verbindung gemäß den Formeln (53) bis (65), 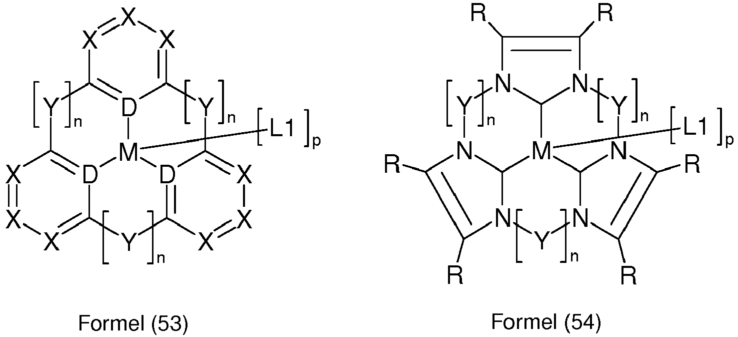 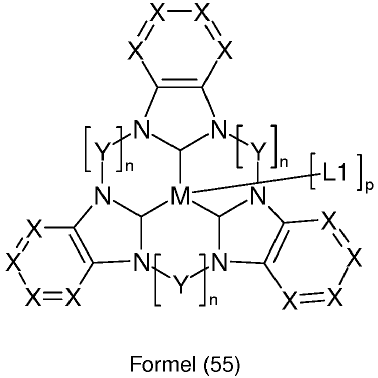 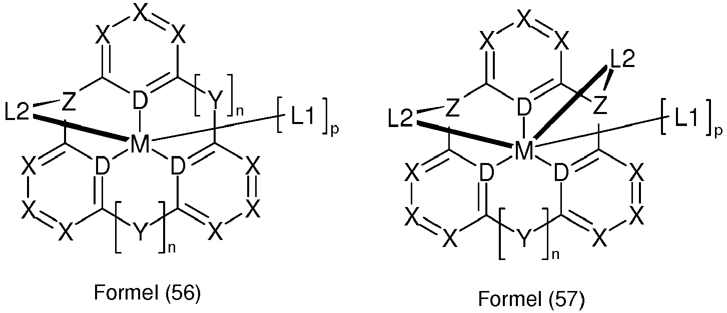 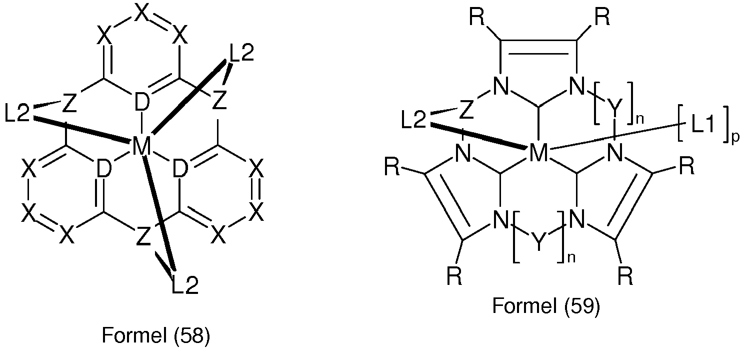 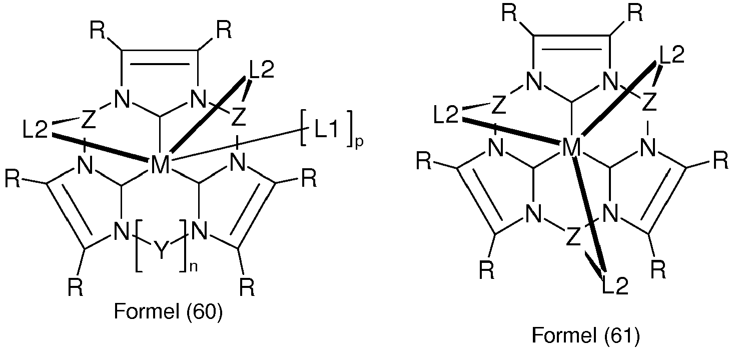 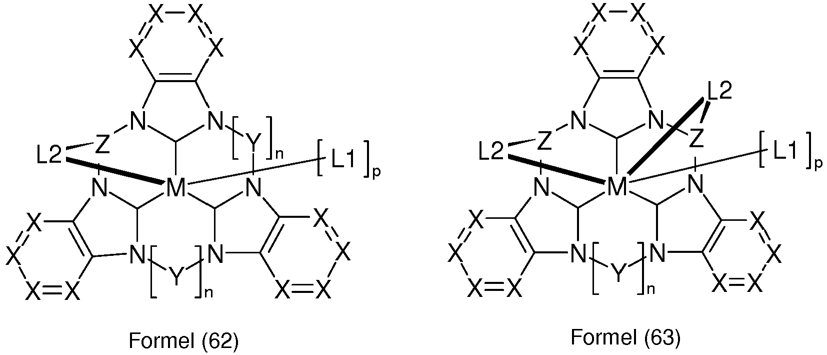 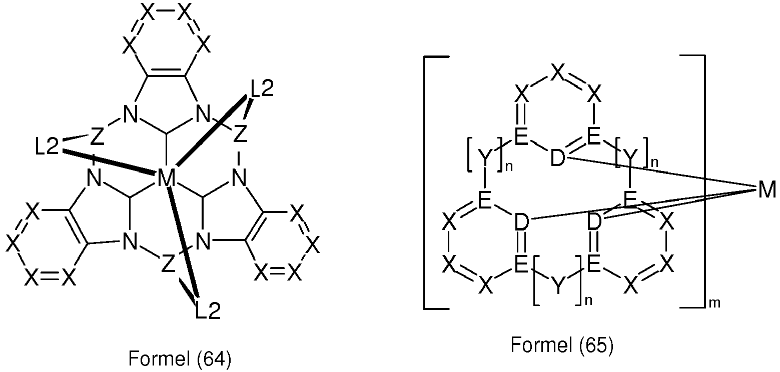 wobei M, X, Y, Z, L1, R, R¹, n und p die in Anspruch 1 und 4 genannten Bedeutungen haben und weiterhin gilt:
D steht bei jedem Auftreten gleich oder verschieden für C, N oder C-O⁻.

9. Elektronische Vorrichtung nach einem oder mehreren der Ansprüche 1 bis 8, **dadurch gekennzeichnet, dass** es sich um eine organische Elektrolumineszenzvorrichtung handelt, enthaltend Kathode, Anode und mindestens eine emittierende Schicht und optional weitere Schichten, ausgewählt aus jeweils einer oder mehreren Lochinjektionsschichten, Lochtransportschichten, Lochblockierschichten, Elektronentransportschichten, Elektroneninjektionsschichten, Exzitonenblockierschichten, Ladungserzeugungsschichten und/oder organischen oder anorganischen p/n-Übergänge.

10. Organische Elektrolumineszenzvorrichtung nach Anspruch 9, **dadurch gekennzeichnet, dass** die Verbindung gemäß Formel (3) bis (7) als emittierende Verbindung in einer emittierenden Schicht eingesetzt wird, bevorzugt in Kombination mit einem oder mehreren Matrixmaterialien, oder dass die Verbindung gemäß Formel (1) bzw. gemäß Formel (3) bis (7) als Matrixmaterial für eine emittierende Verbindung in einer emittierenden Schicht eingesetzt wird oder dass die Verbindung gemäß Formel (1) bzw. gemäß Formel (3) bis (7) als Lochblockiermaterial in einer Lochblockierschicht und/oder als Elektronentransportmaterial in einer Elektronentransportschicht oder dass die Verbindung gemäß Formel (1) bzw. gemäß Formel (3) bis (7) als Lochtransportmaterial in einer Lochtransportschicht und/oder als Exzitonenblockiermaterial in einer Exzitonenblockierschicht eingesetzt wird.

11. Verbindungen der Formeln (4) bis (7) und (6a), 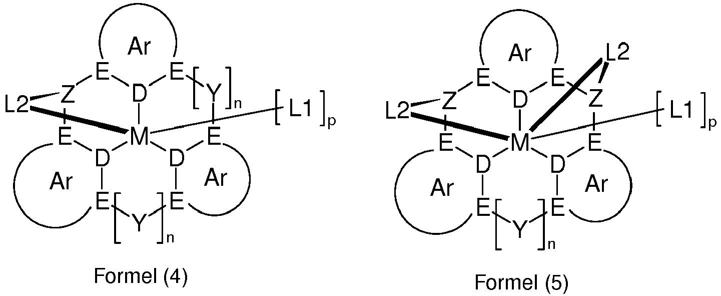 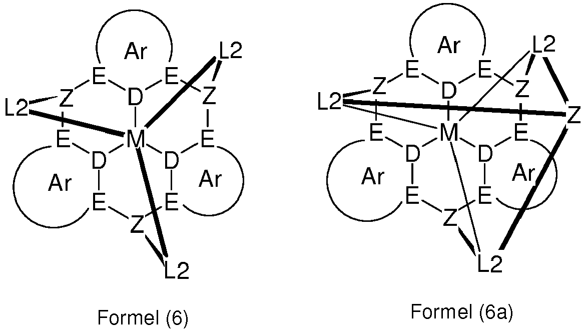 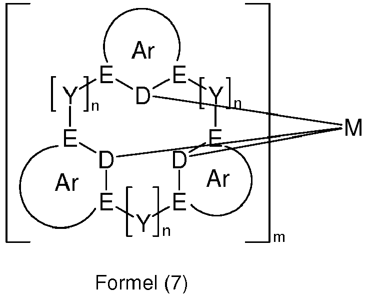 wobei die verwendeten Symbole und Indizes die in den Ansprüchen 1 und 4 aufgeführten Bedeutungen haben;
dabei ist die folgende Verbindung von der Erfindung ausgenommen:

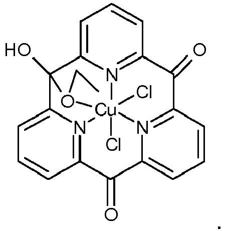

12. Verfahren zur Herstellung von Verbindungen nach Anspruch 11 durch Umsetzung des Liganden gemäß Formel (2) und gegebenenfalls weiteren Liganden L1 mit Metallalkoholaten gemäß Formel (66), mit Metallketoketonaten gemäß Formel (67) oder Metallhalogeniden gemäß Formel (68), 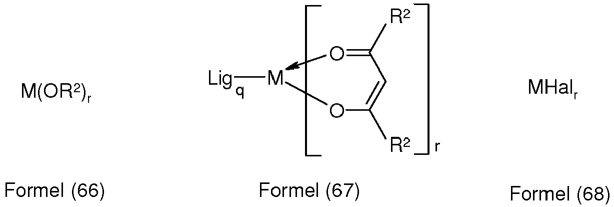 wobei M und R² dieselbe Bedeutung haben, wie in Anspruch 1 beschrieben, und für die weiteren Symbole und Indizes gilt:
Hal ist bei jedem Auftreten gleich oder verschieden F, Cl, Br oder I;
Lig ist bei jedem Auftreten gleich oder verschieden ein neutraler oder monoanionischer, einzähniger oder zweizähniger Ligand;
q ist bei jedem Auftreten gleich oder verschieden 0, 1, 2, 3 oder 4, bevorzugt 0, 1 oder 2;
r ist bei jedem Auftreten gleich oder verschieden 1, 2, 3, 4 oder 5, wobei p in Formel (66) und (68) die Wertigkeit des Metalls M angibt;
dabei kann die Verbindung gemäß Formel (67) auch geladen sein und noch ein Gegenion enthalten; weiterhin können die Verbindungen gemäß Formel (66) bis (68), insbesondere gemäß Formel (68) auch als Hydrat vorliegen.

13. Verbindungen gemäß den Formeln (69) bis (71), 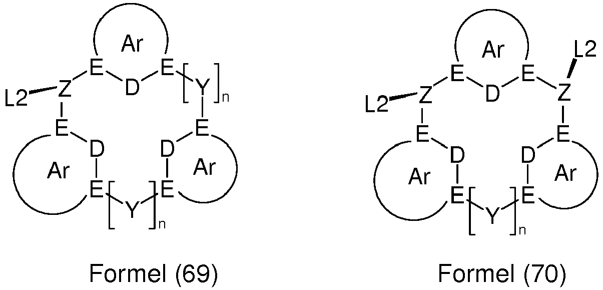 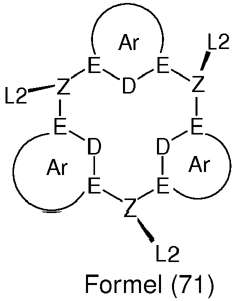 wobei die Symbole und Indizes die in Anspruch 1 und 4 genannten Bedeutungen haben; dabei tragen die Gruppen D bzw. L2, wenn sie im Komplex als anionische Gruppen an das Metall M binden, noch zusätzlich jeweils ein Wasserstoffatom; dabei sind die folgenden Verbindungen von der Erfindung ausgenommen:

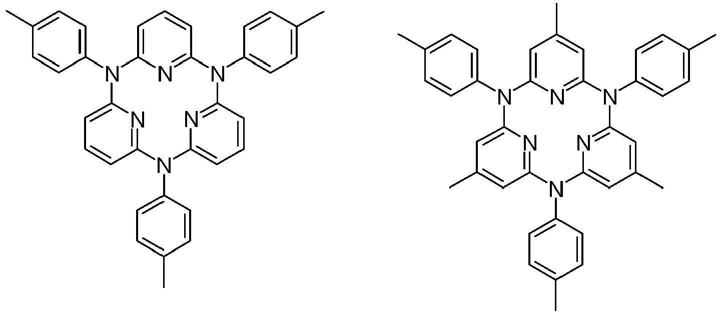

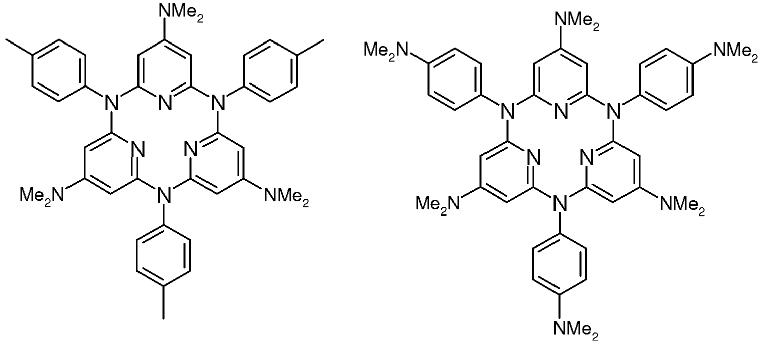

14. Oligomere, Polymere oder Dendrimere enthaltend eine oder mehrere Verbindungen gemäß Formel (3) bis (7), wobei ein oder mehrere Bindungen des Komplexes gemäß Formel (3) bis (7) zum Polymer, Oligomer oder Dendrimer vorhanden sind.

## Claims

1. Electronic device containing at least one metal complex of the formula (3), 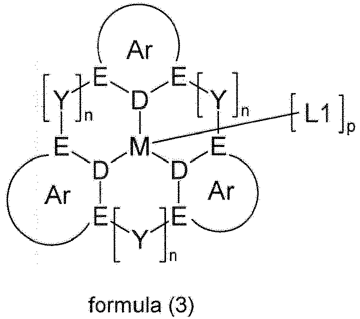 where the following applies to the symbols and indices used:
M is a transition metal, a lanthanide, an alkali metal, an alkalineearth metal or a main-group metal from the third or fourth main group;
D is on each occurrence, identically or differently, C, N, P, C-O⁻, C-S⁻, C-NR₂ or C-PR₂, where the last four groups mentioned are bonded to the metal via O, S, N or P as exocyclic donor atoms, or C-N≡C, where this group is bonded to the metal via the carbon of the exocyclic isonitrile group;
E is on each occurrence, identically or differently, C or N;
Ar is on each occurrence, identically or differently, a group which forms an aryl or heteroaryl group having 5 to 40 aromatic ring atoms together with the group E-D-E and which may be substituted by one or more radicals R; or, if D stands for a carbene carbon atom, Ar is a group which forms a cyclic saturated group having 5 to 10 ring atoms together with the group E-D-E;
Y is, identically or differently on each occurrence, BR¹, B(R¹)₂-, C(R¹)⁻, C(R¹)₂, Si(R¹)⁻, Si(R¹)₂, C(=O), C(=NR), N⁻, NR¹, N(R¹)₂⁺, PR¹, P(R¹)₂⁺, AsR¹, As(R¹)₂⁺, P(=O)R¹, As(=O)R¹, P(=S)R¹, As(=S)R¹, O, S, S(R¹)⁺, Se, Te, S(=O), S(=O)₂, Se(=O), Se(=O)₂, Te(=O) or Te(=O)₂;
R is on each occurrence, identically or differently, H, deuterium, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, which may in each case be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of these systems; two or more of these substituents R may also form a mono- or polycyclic, aliphatic, aromatic and/or benzo-fused ring system with one another;
R¹ is, identically or differently on each occurrence, R or a group L2;
R² is on each occurrence, identically or differently, H, F or an aliphatic, aromatic and/or heteroaromatic hydrocarbon radical having 1 to 20 C atoms, in which, in addition, one or more H atoms may be replaced by F; two or more substituents R² here may also form a mono- or polycyclic, aliphatic or aromatic ring system with one another;
L2 is on each occurrence, identically or differently, a donor group having 1 to 40 C atoms, which may form a further bond or coordination to the metal M and which may be substituted by one or more radicals R;
n is on each occurrence, identically or differently, 0, 1, 2, 3, 4, 5 or 6, where n = 0 means that the group Y is not present and a single bond is present between the two groups L, with the proviso that all indices n cannot simultaneously stand for 0;
L1 is on each occurrence, identically or differently, a mono-, bi-, tri-, tetra-, penta- or hexadentate ligand which is bonded to the metal M;
p is 0, 1, 2, 3, 4, 5, 6, 7, 8 or 9.

2. Electronic device according to Claim 1, comprising anode, cathode and at least one layer which comprises at least one compound of the formula (1), selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) and organic laser diodes (O-lasers), preferably organic electroluminescent devices.

3. Electronic device according to Claim 1 or 2, **characterised in that** M stands for zirconium, hafnium, molybdenum, tungsten, rhenium, ruthenium, osmium, rhodium, iridium, palladium, platinum, copper, silver, gold, scandium, yttrium, lanthanum, aluminium, gallium or indium.

4. Electronic device according to one or more of Claims 1 to 3, containing a compound of the formula (4), (5), (6), (6a) or (7), 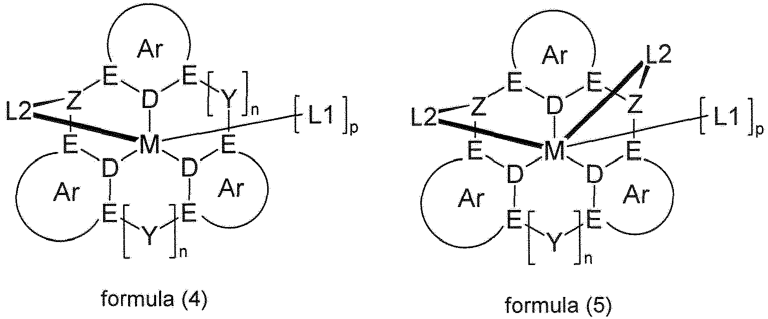 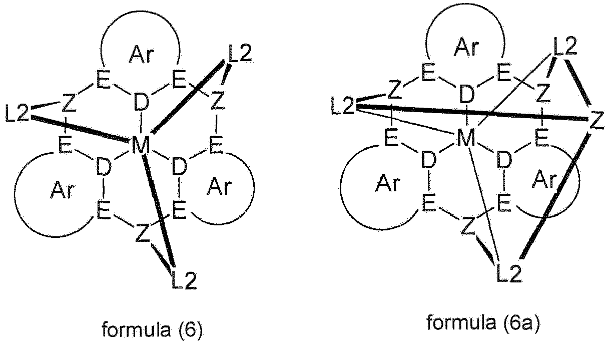 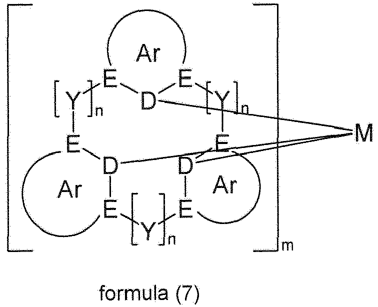 where Z in the formulae (4) to (6) stands, identically or differently on each occurrence, for B, B(R¹)⁻, C⁻, CR¹, SiR¹, N, P, As, P(=O), As(=O), P(=S) or As(=S), m stands for 2, 3 or 4, and the other symbols and indices have the meanings indicated in Claim 1.

5. Electronic device according to one or more of Claims 1 to 4, **characterised in that** the aryl or heteroaryl group formed by Ar together with the group E-D-E in compounds of the formulae (3) to (7) stands for a group of the following formulae (8) to (20), where in each case the dashed bond indicates the bonding of this group in the ligand, i.e. the bond to the groups Y, and where * in each case denotes the position of the coordination to the metal M, 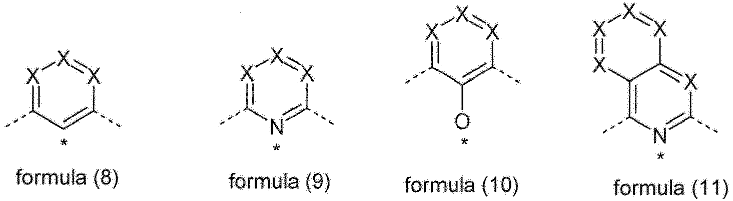 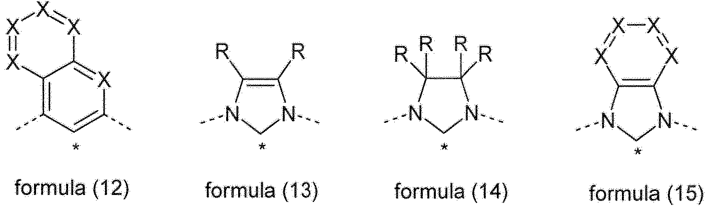 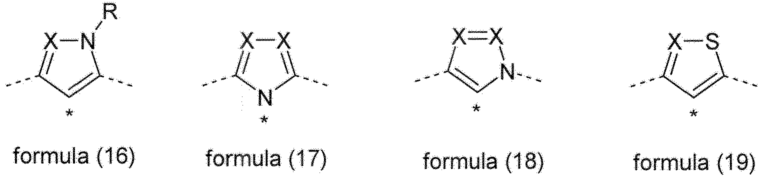 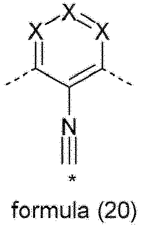 where the symbols used have the meaning indicated in Claim 1 and X stands, identically or differently on each occurrence, for CR or N, with the proviso that a maximum of three symbols X in each group stand for N.

6. Organic electronic device according to one or more of Claims 1 to 5, **characterised in that** the group L2 represents an aryl or heteroaryl group and is selected from groups of the formulae (21) to (49), where in each case the dashed bond indicates the bonding of this group in the ligand, i.e. the bond to the group Z, where * in each case denotes the position of the coordination to the metal M and where the symbols used have the meanings indicated in Claim 1: 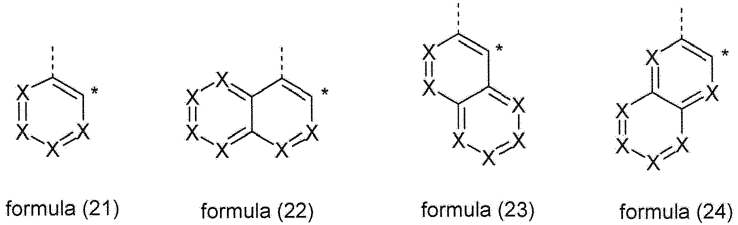 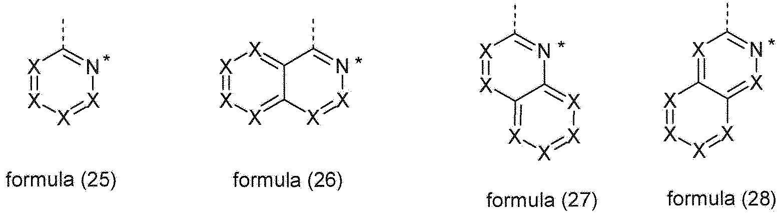 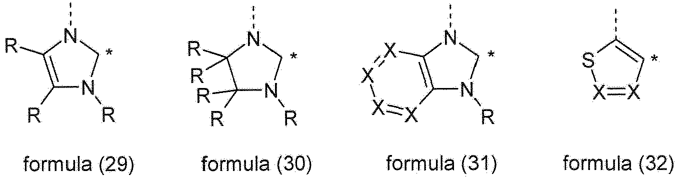 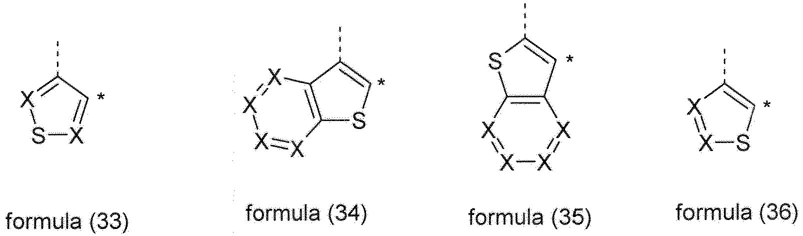 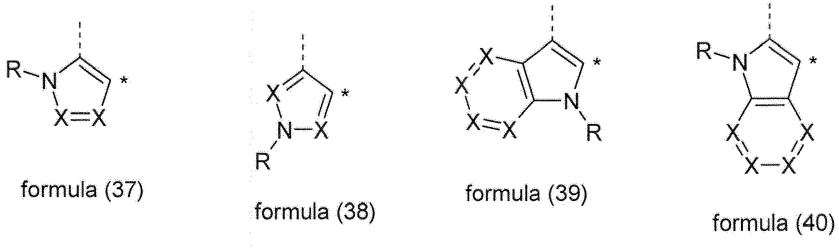 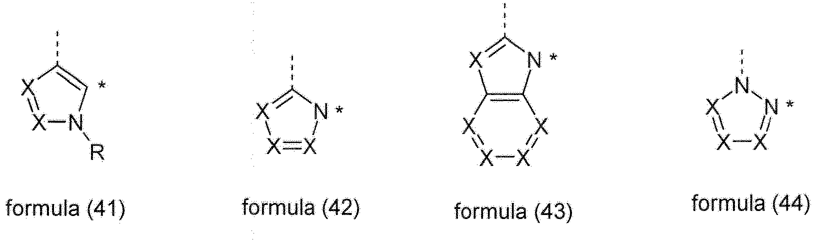 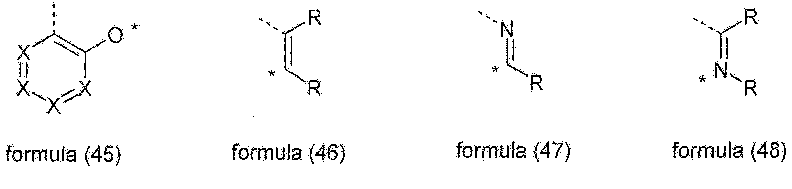 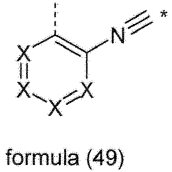 or **in that** L2 represents a neutral or anionic donor group selected from the carbon-containing donor groups of the aliphatic or aromatic acetylides or of the aliphatic or aromatic isonitriles, the nitrogen-containing donor groups of the aliphatic amines, aliphatic cyclic amines, nitriles, amides, imides and imines, which may in each case be substituted by groups R or unsubstituted, the phosphorus-containing donor groups PF₂, P(NR₂)₂, where R stands, identically or differently on each occurrence, for a C₁-C₂₀-alkyl group or an aryl or heteroaryl group, alkyl-, aryl- or mixed alkylarylphosphines, alkylhalo-, arylhalo- or mixed alkylarylhalophosphines, where the halogen may in each case be F, Cl, Br or I, alkyl, aryl or mixed alkyl aryl phosphites or phospha-aromatic compounds, which may in each case be substituted by groups R or unsubstituted, the oxygen-containing donor groups of the alcohols, alcoholates, open-chain or cyclic, aliphatic or aromatic ethers, oxygen heterocycles, aldehydes, ketones, phosphine oxide groups, phosphates, phosphonates, borates, silicates, sulfoxide groups, carboxylates, phenols, phenolates, oximes, hydroxamates, β-ketoketonates, β-ketoesters and β-diesters, which may in each case be substituted by groups R or unsubstituted, the sulfur-containing donor groups of the aliphatic or aromatic thiols and thiolates, open-chain or cyclic thioethers, thiocarbonyl groups, phosphine sulfides and thiocarboxylates, which may in each case be substituted by groups R or unsubstituted, or bidentate-chelating groups formed from these groups.

7. Electronic device according to one or more of Claims 1 to 6, **characterised in that** the ligands L1 are selected from carbon monoxide, isonitriles, amines, imines, diimines, phosphines, phosphites, arsines, stibines, nitrogen-containing heterocycles, hydride, deuteride, the halides F, Cl, Br and I, alkylacetylides, arylacetylides, cyanide, cyanate, isocyanate, thiocyanate, isothiocyanate, aliphatic or aromatic alcoholates, aliphatic or aromatic thioalcoholates, amides, carboxylates, anionic, nitrogen-containing heterocycles, O²⁻, S²⁻, nitrenes, N³⁻, diamines, heterocycles containing two nitrogen atoms, diphosphines, 1,3-diketonates derived from 1,3-diketones, 3-ketonates derived from 3-ketoesters, carboxylates derived from aminocarboxylic acids, salicyliminates derived from salicylimines, dialcoholates derived from dialcohols, borates of nitrogen-containing heterocycles, bidentate monoanionic ligands which, with the metal, form a cyclometallated five-membered ring containing at least one metal-carbon bond, η⁵-cyclopentadienyl, η⁵-pentamethylcyclopentadienyl, η⁶-benzene, η⁷-cycloheptatrienyl, which may in each case also be substituted by R, 1,3,5-cis-cyclohexane derivatives, 1,1,1-tri(methylene)methane derivatives and 1,1,1-trisubstituted methanes.

8. Electronic device according to one or more of Claims 1 to 8, containing at least one compound of the formulae (53) to (65), 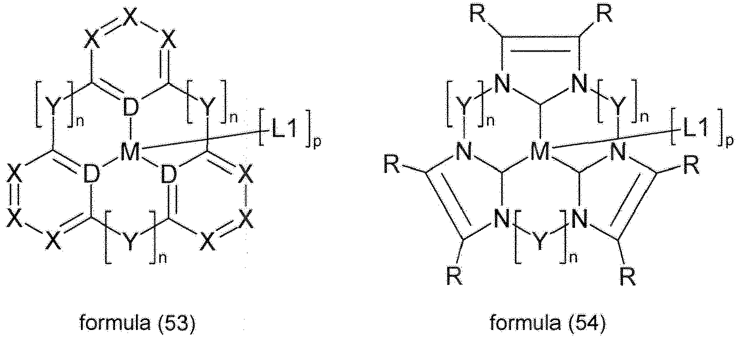 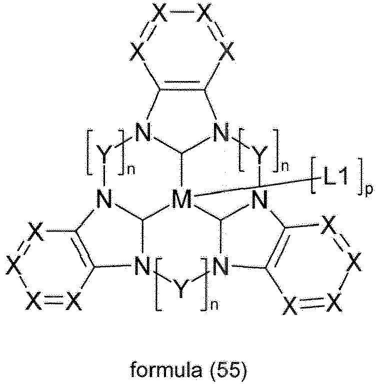 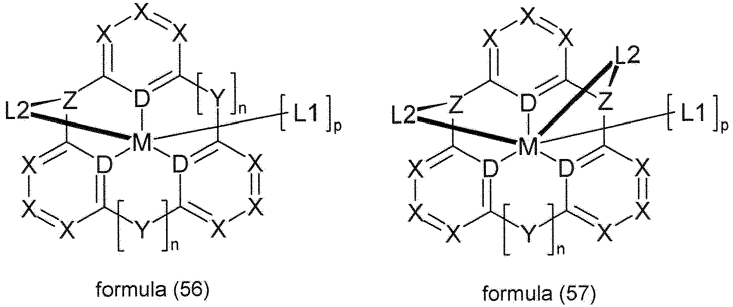 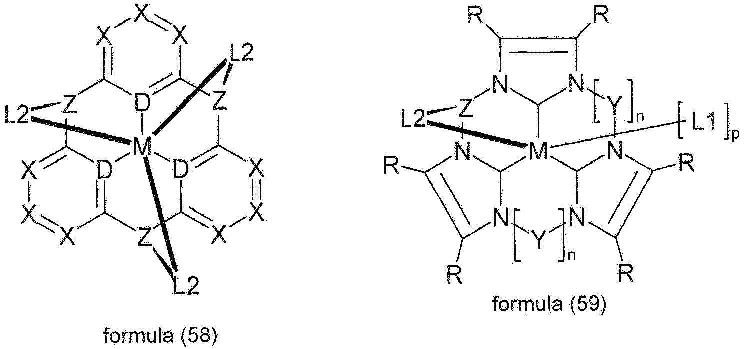 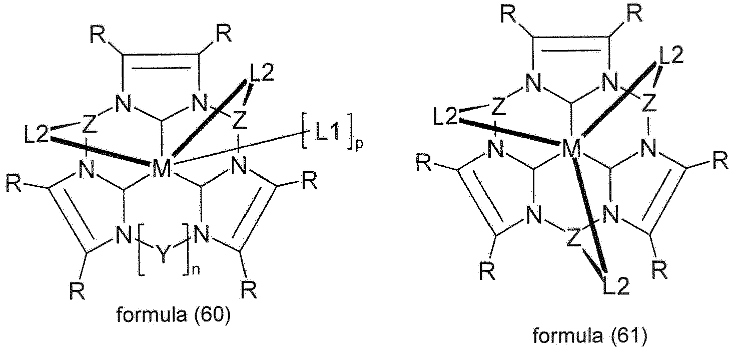 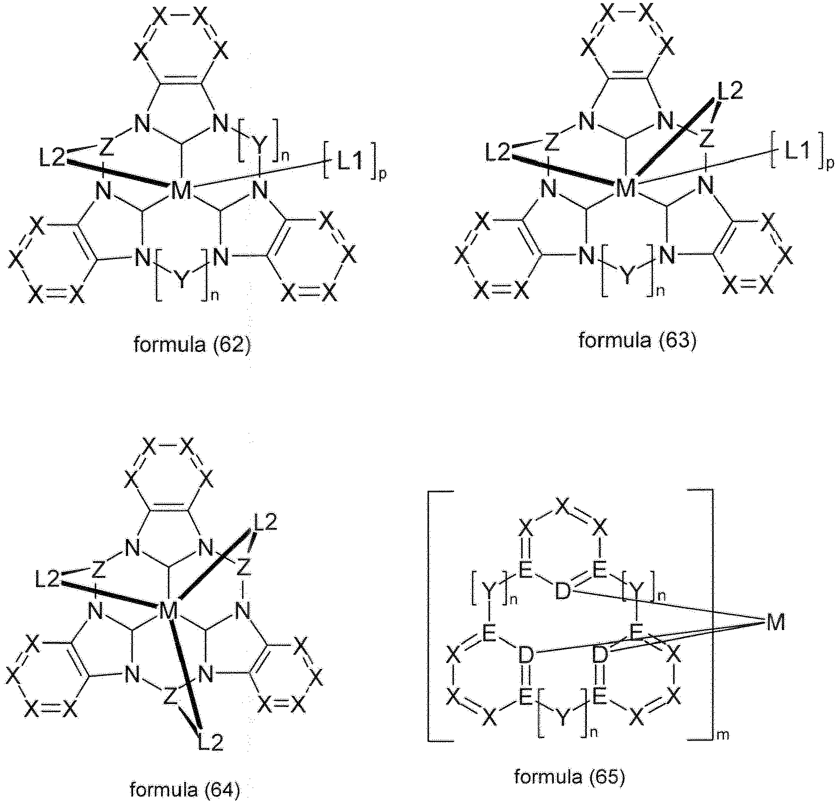 where M, X, Y, Z, L1, R, R¹, n and p have the meanings given in Claims 1 and 4, and furthermore:
D stands on each occurrence, identically or differently, for C, N or C-O⁻.

9. Electronic device according to one or more of Claims 1 to 8, **characterised in that** it is an organic electroluminescent device comprising cathode, anode and at least one emitting layer and optionally further layers selected from in each case one or more hole-injection layers, hole-transport layers, hole-blocking layers, electron-transport layers, electron-injection layers, exciton-blocking layers, charge-generation layers and/or organic or inorganic p/n junctions.

10. Organic electroluminescent device according to Claim 9, **characterised in that** the compound of the formulae (3) to (7) is employed as emitting compound in an emitting layer, preferably in combination with one or more matrix materials, or **in that** the compound of the formula (1) or of the formulae (3) to (7) is employed as matrix material for an emitting compound in an emitting layer, or **in that** the compound of the formula (1) or of the formulae (3) to (7) is employed as hole-blocking material in a hole-blocking layer and/or as electron-transport material in an electron-transport layer, or **in that** the compound of the formula (1) or of the formulae (3) to (7) is employed as hole-transport material in a hole-transport layer and/or as exciton-blocking material in an exciton-blocking layer.

11. Compounds of the formulae (4) to (7) and (6a), 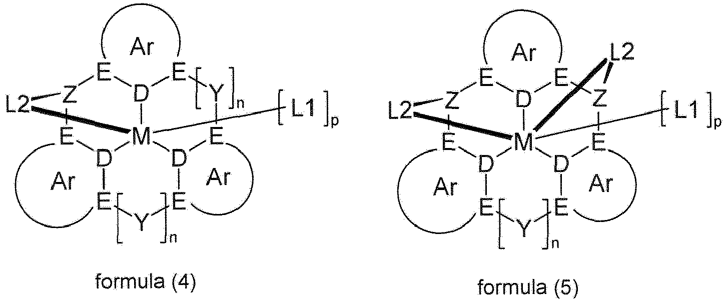 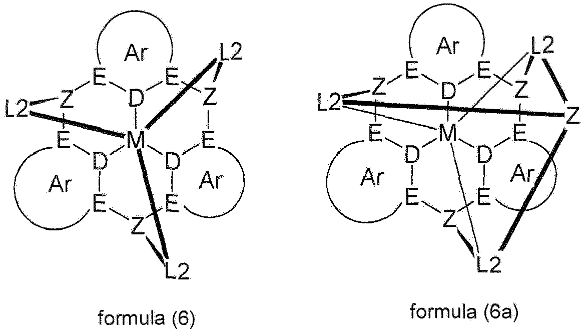 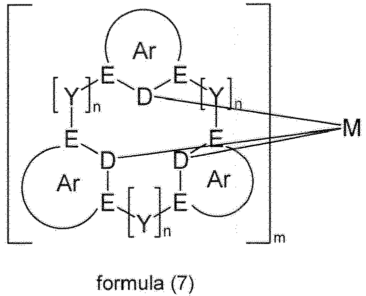 where the symbols and indices used have the meanings indicated in Claims 1 and 4;
the following compound is excluded from the invention:

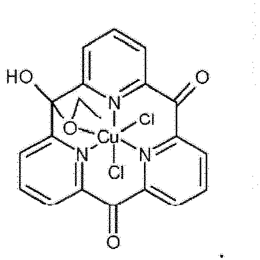

12. Process for the preparation of compounds according to Claim 11 by reaction of the ligand of the formula (2) and optionally further ligands L1 with metal alcoholates of the formula (66), with metal ketoketonates of the formula (67) or metal halides of the formula (68), 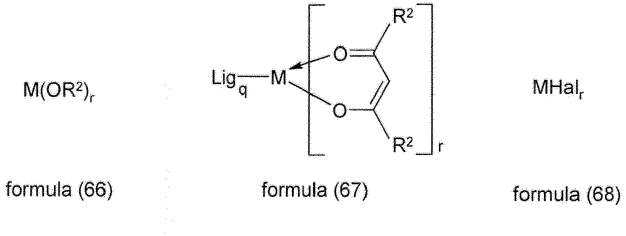 where M and R² have the same meaning as described in Claim 1, and the following applies to the other symbols and indices:
Hal is on each occurrence, identically or differently, F, Cl, Br or I;
Lig is on each occurrence, identically or differently, a neutral or monoanionic, monodentate or bidentate ligand;
q is on each occurrence, identically or differently, 0, 1, 2, 3 or 4, preferably 0, 1 or 2;
r is on each occurrence, identically or differently, 1, 2, 3, 4 or 5, where p in formulae (66) and (68) indicates the valency of the metal M;
the compound of the formula (67) may also be charged and may also contain a counterion; furthermore, the compounds of the formulae (66) to (68), in particular of the formula (68), may also be in the form of the hydrate.

13. Compounds of the formulae (69) to (71), 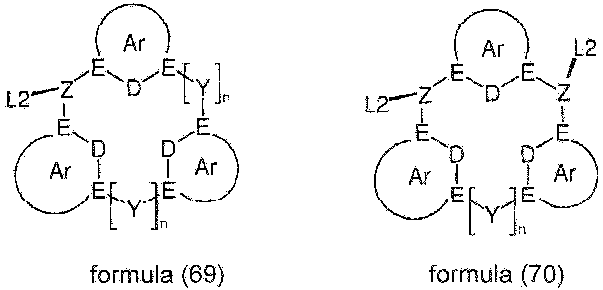 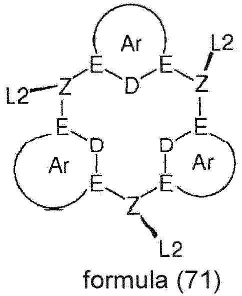 where the symbols and indices have the meanings given in Claims 1 and 4; the groups D and L2, if they are bonded to the metal M as anionic groups in the complex, additionally in each case carry a hydrogen atom; the following compounds are excluded from the invention:

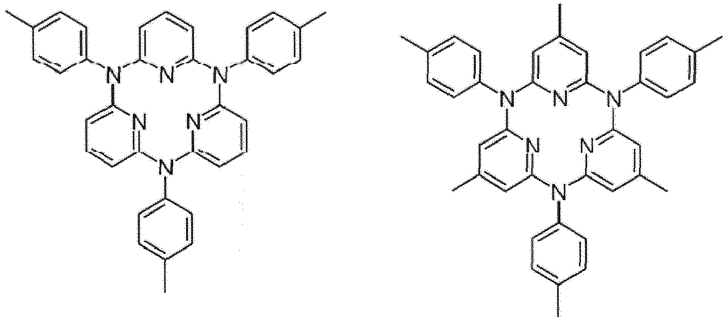

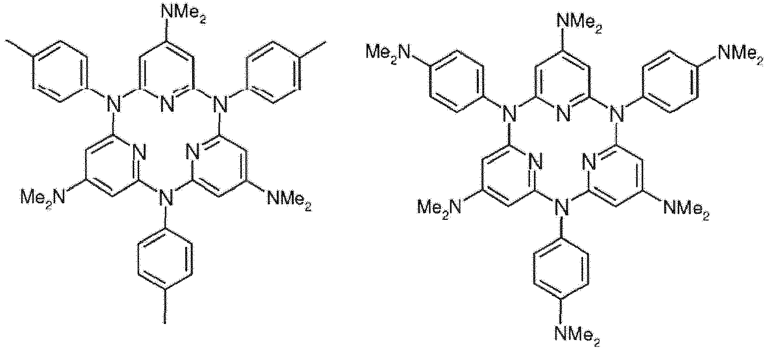

14. Oligomers, polymers or dendrimers containing one or more compounds of the formulae (3) to (7), where one or more bonds are present from the complex of the formulae (3) to (7) to the polymer, oligomer or dendrimer.

## Revendications

1. Dispositif électronique contenant au moins un complexe métallique de la formule (3) : 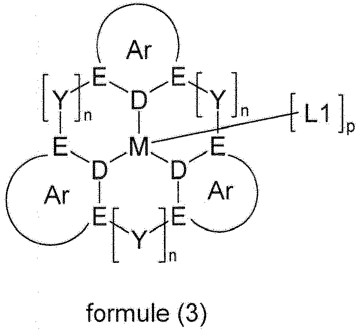 dans laquelle ce qui suit s'applique aux symboles et aux indices qui sont utilisés :
M est un métal de transition, un lanthanide, un métal alcalin, un métal alcalino-terreux ou un métal de groupes principaux qui est issu du troisième ou quatrième groupe principal ;
D est pour chaque occurrence, de manière identique ou différente, C, N, P, C-O⁻, C-S⁻, C-NR₂ ou C-PR₂, où les quatre derniers groupes qui ont été mentionnés sont liés au métal via O, S, N ou P en tant qu'atomes donneurs exocycliques, ou C-N=C, où ce groupe est lié au métal via le carbone du groupe isonitrile exocyclique ;
E est pour chaque occurrence, de manière identique ou différente, C ou N ;
Ar est pour chaque occurrence, de manière identique ou différente, un groupe qui forme un groupe aryle ou hétéroaryle qui comporte de 5 à 40 atomes de cycle aromatique en association avec le groupe E-D-E et qui peut être substitué par un radical ou par plusieurs radicaux R ; ou, si D représente un atome de carbone carbène, Ar est un groupe qui forme un groupe saturé cyclique qui comporte de 5 à 10 atomes de cycle en association avec le groupe E-D-E ;
Y est, de manière identique ou différente pour chaque occurrence, BR¹, B(R¹)₂⁻, C(R¹)⁻, C(R¹)₂, Si(R¹)⁻, Si(R¹)₂, C(=O), C(=NR), N⁻, NR¹, N(R¹)₂⁺, PR1, P(R¹)₂⁺, AsR¹, As(R¹)₂⁺, P(=O)R¹, As(=O)R¹, P(=S)R¹, As(=S)R¹, O, S, S(R¹)⁺, Se, Te, S(=O), S(=O)₂, Se(=O), Se(=O)₂, Te(=O) ou Te(=O)₂ ;
R est pour chaque occurrence, de manière identique ou différente, H, deutérium, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 60 atomes de cycle aromatique, lequel peut être substitué par un radical ou par plusieurs radicaux R², ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R², ou une combinaison de ces systèmes ; deux de ces substituants R ou plus peuvent également former un système de cycle aliphatique, aromatique et/ou benzo-fusionné mono- ou polycyclique l'un avec l'autre ou les uns avec les autres ;
R¹ est, de manière identique ou différente pour chaque occurrence, R ou un groupe L2 ;
R² est pour chaque occurrence, de manière identique ou différente, H, F ou un radical hydrocarbone aliphatique, aromatique et/ou hétéroaromatique qui comporte de 1 à 20 atome(s) de C, où, en outre, un ou plusieurs atome(s) de H peut/peuvent également être remplacé(s) par F ; deux substituants R² ou plus peuvent ici également former un système de cycle aliphatique ou aromatique mono- ou polycyclique l'un avec l'autre ou les uns avec les autres ;
L2 est pour chaque occurrence, de manière identique ou différente, un groupe donneur qui comporte de 1 à 40 atome(s) de C, lequel peut former une autre liaison ou coordination sur le métal M et lequel peut être substitué par un radical ou par plusieurs radicaux R;
n est pour chaque occurrence, de manière identique ou différente, 0, 1, 2, 3, 4, 5 ou 6, où n = 0 signifie que le groupe Y n'est pas présent et qu'une liaison simple est présente entre les deux groupes L, étant entendu que tous les indices n ne peuvent pas représenter de façon simultanée 0 ;
L1 est pour chaque occurrence, de manière identique ou différente, un ligand mono-, bi-, tri-, tétra-, penta- ou hexadenté qui est lié au métal M ;
p est 0, 1, 2, 3, 4, 5, 6, 7, 8 ou 9.

2. Dispositif électronique selon la revendication 1, comprenant une anode, une cathode et au moins une couche qui comprend au moins un composé de la formule (1), sélectionné parmi le groupe qui est constitué par les dispositifs électroluminescents organiques (OLED, PLED), les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC) et les diodes laser organiques (O-laser), de préférence les dispositifs électroluminescents organiques.

3. Dispositif électronique selon la revendication 1 ou 2, **caractérisé en ce que** M représente le zirconium, le hafnium, le molybdène, le tungstène, le rhénium, le ruthénium, l'osmium, le rhodium, l'iridium, le palladium, le platine, le cuivre, l'argent, l'or, le scandium, l'yttrium, le lanthane, l'aluminium, le gallium ou l'indium.

4. Dispositif électronique selon une ou plusieurs des revendications 1 à 3, contenant un composé de la formule (4), (5), (6), (6a) ou (7) : 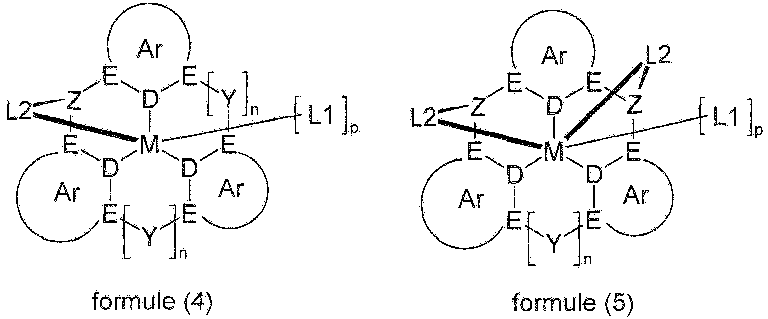 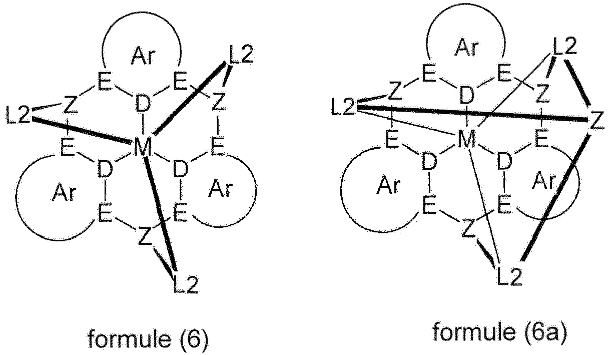 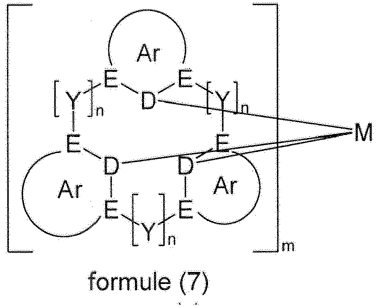 où Z dans les formules (4) à (6) représente, de manière identique ou différente pour chaque occurrence, B, B(R¹)⁻, C⁻, CR¹, SiR¹, N, P, As, P(=O), As(=O), P(=S) ou As(=S), m représente 2, 3 ou 4, et les autres symboles et indices présentent les significations qui ont été indiquées selon la revendication 1.

5. Dispositif électronique selon une ou plusieurs des revendications 1 à 4, **caractérisé en ce que** le groupe aryle ou hétéroaryle qui est formé par Ar en association avec le groupe E-D-E dans les composés des formules (3) à (7) représente un groupe des formules (8) à (20) qui suivent, dans lesquelles, dans chaque cas, la liaison en pointillés représente la liaison de ce groupe dans le ligand, c'est-à-dire la liaison sur les groupes Y, et dans lesquelles *, dans chaque cas, représente la position de la coordination sur le métal M ; 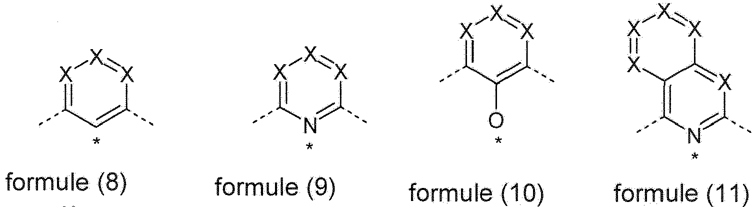 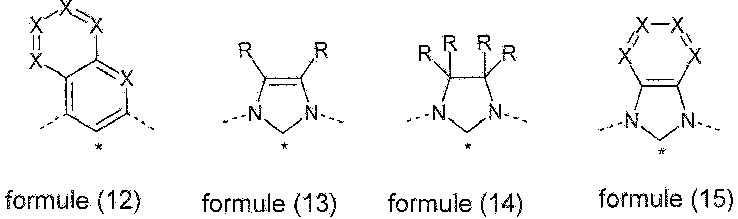 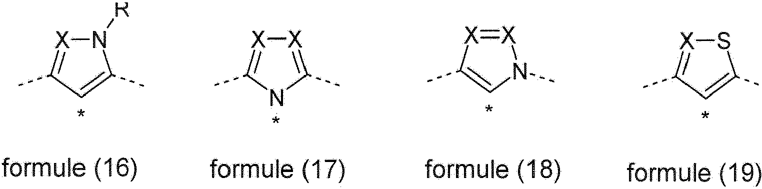 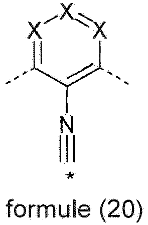 où les symboles qui sont utilisés présentent la signification qui a été indiquée selon la revendication 1 et X représente, de manière identique ou différente pour chaque occurrence, CR ou N, étant entendu qu'un maximum de trois symboles X dans chaque groupe représentent N.

6. Dispositif électronique organique selon une ou plusieurs des revendications 1 à 5, **caractérisé en ce que** le groupe L2 représente un groupe aryle ou hétéroaryle et est sélectionné parmi les groupes des formules (21) à (49), où, dans chaque cas, la liaison en pointillés indique la liaison de ce groupe dans le ligand, c'est-à-dire la liaison sur le groupe Z, où *, dans chaque cas, représente la position de la coordination sur le métal M et où les symboles qui sont utilisés présentent les significations qui ont été indiquées selon la revendication 1 : 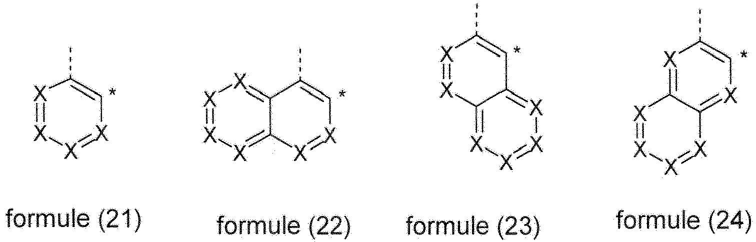 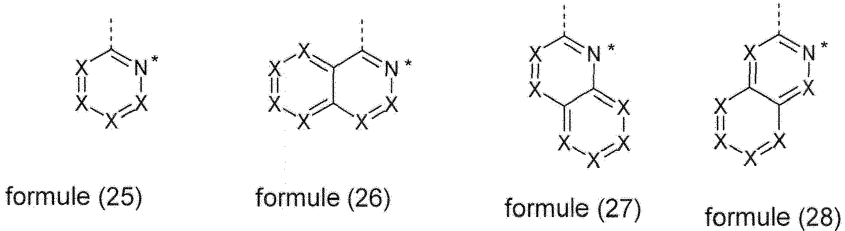 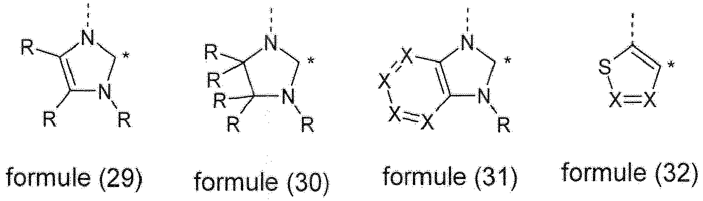 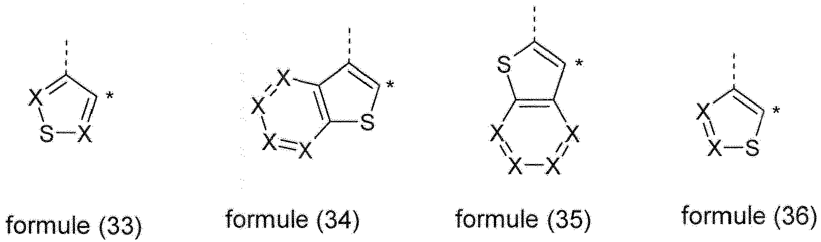 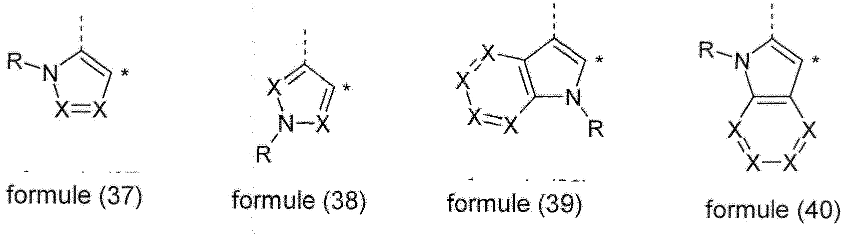 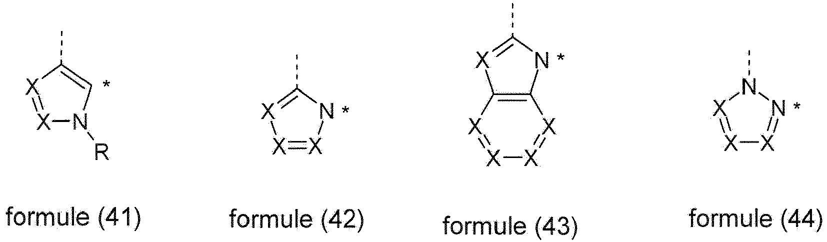 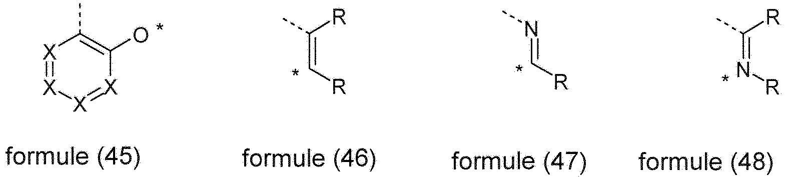 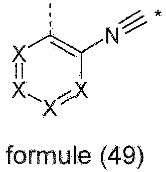 ou **en ce que** L2 représente un groupe donneur neutre ou anionique qui est sélectionné parmi les groupes donneurs contenant du carbone qui sont constitués par les acétylures aliphatiques ou aromatiques ou les isonitriles aliphatiques ou aromatiques, les groupes donneurs contenant de l'azote qui sont constitués par les amines aliphatiques, les amines cycliques aliphatiques, les nitriles, les amides, les imides et les imines, lesquels peuvent dans chaque cas être substitués par des groupes R ou peuvent être non substitués, les groupes donneurs contenant du phosphore PF₂, P(NR₂)₂, où R représente, de manière identique ou différente pour chaque occurrence, un groupe C₁-C₂₀-alkyle ou un groupe aryle ou hétéroaryle, les alkyl- ou aryl-phosphines ou les alkylarylphosphines mixtes, les alkylhalo- ou arylhalo-phosphines ou les alkylarylhalophosphines mixtes, où l'halogène peut dans chaque cas être F, Cl, Br ou I, les alkyl- ou aryl-phosphites ou les alkylarylphosphites mixtes ou les composés phospha-aromatiques, lesquels peuvent dans chaque cas être substitués par des groupes R ou peuvent être non substitués, les groupes donneurs contenant de l'oxygène qui sont constitués par les alcools, les alcoolates, les éthers aliphatiques ou aromatiques, à chaîne ouverte ou cycliques, les hétérocycles d'oxygène, les aldéhydes, les cétones, les groupes oxyde de phosphine, les phosphates, les phosphonates, les borates, les silicates, les groupes sulfoxyde, les carboxylates, les phénols, les phénolates, les oximes, les hydroxamates, les β-cétocétonates, les β-cétoesters et les β-diesters, lesquels peuvent dans chaque cas être substitués par des groupes R ou peuvent être non substitués, les groupes donneurs contenant du soufre qui sont constitués par les thiols et les thiolates aliphatiques ou aromatiques, les thioéthers à chaîne ouverte ou cycliques, les groupes thiocarbonyle, les sulfures de phosphine et les thiocarboxylates, lesquels peuvent dans chaque cas être substitués par des groupes R ou peuvent être non substitués, ou les groupes bidentés-chélatants qui sont formés à partir de ces groupes.

7. Dispositif électronique selon une ou plusieurs des revendications 1 à 6, **caractérisé en ce que** les ligands L1 sont sélectionnés parmi le monoxyde de carbone, les isonitriles, les amines, les imines, les diimines, les phosphines, les phosphites, les arsines, les stibines, les hétérocycles contenant de l'azote, l'hydrure, le deutérure, les halogénures F, Cl, Br et I, les alkylacétylures, les arylacétylures, le cyanure, le cyanate, l'isocyanate, le thiocyanate, l'isothiocyanate, les alcoolates aliphatiques ou aromatiques, les thioalcoolates aliphatiques ou aromatiques, les amides, les carboxylates, les hétérocycles anioniques contenant de l'azote, O²⁻, S²⁻, les nitrènes, N³⁻, les diamines, les hétérocycles contenant deux atomes d'azote, les diphosphines, les 1,3-dicétonates dérivés des 1,3-dicétones, les 3-cétonates dérivés des 3-cétoesters, les carboxylates dérivés des acides aminocarboxyliques, les salicyliminates dérivés des salicylimines, les dialcoolates dérivés des dialcools, les borates d'hétérocycliques contenant de l'azote, les ligands monoanioniques bidentés qui, avec le métal, forment un cycle à cinq éléments cyclométallaté qui contient au moins une liaison métal-carbone, le η⁵-cyclopentadiényle, le η⁵-pentaméthylcyclopentadiényle, le η⁶-benzène, le η⁷-cycloheptatriényle, lesquels peuvent dans chaque cas également être substitués par R, les dérivés de 1,3,5-cis-cyclohexane, les dérivés de 1,1,1-tri(méthylène)méthane et les méthanes 1,1,1-trisubstitués.

8. Dispositif électronique selon une ou plusieurs des revendications 1 à 8, contenant au moins un composé des formules (53) à (65) : 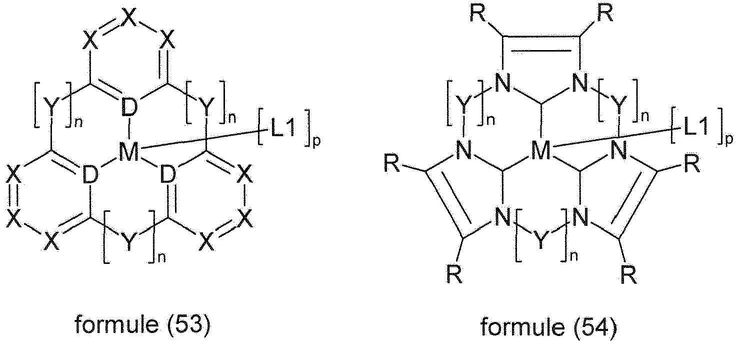 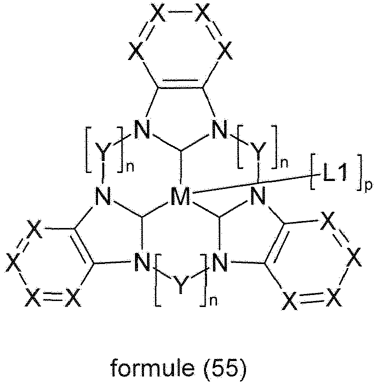 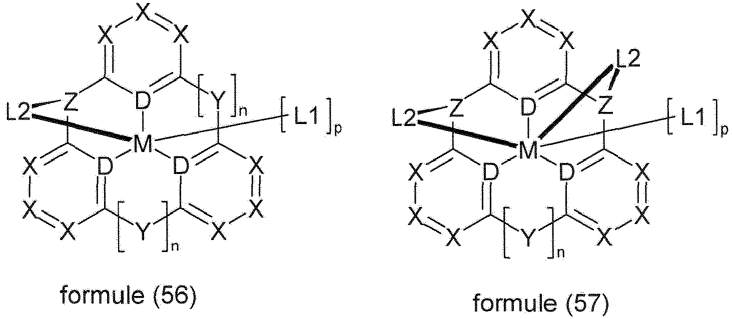 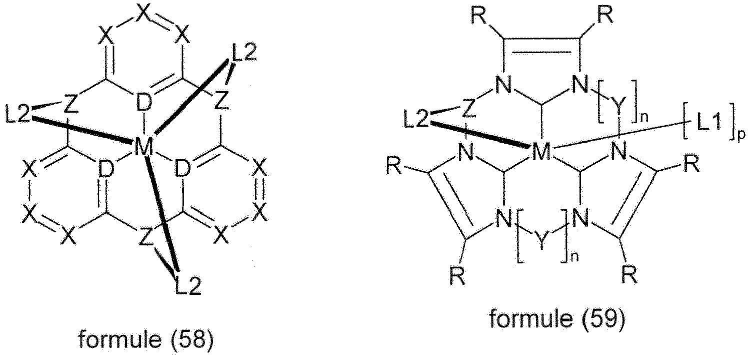 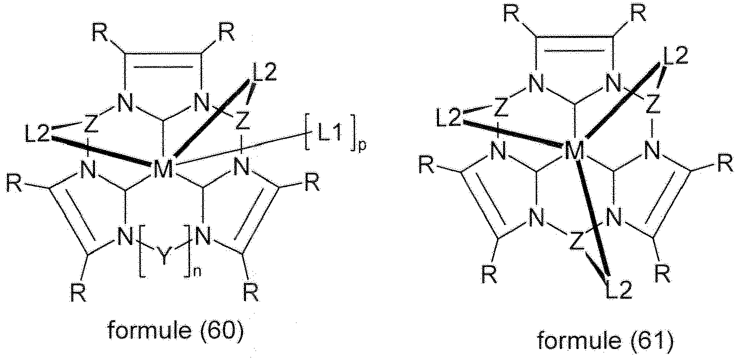 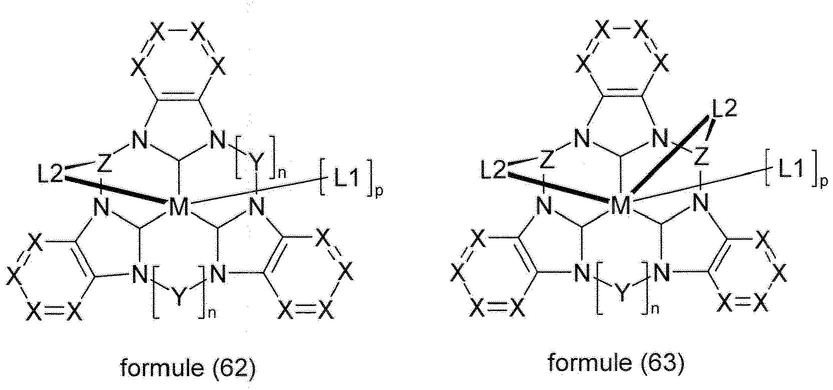 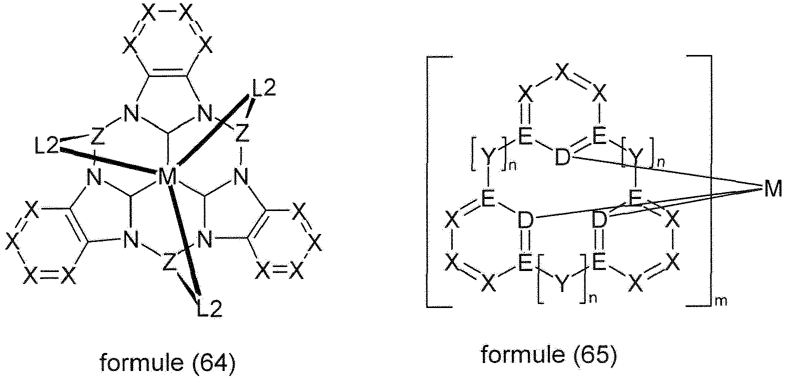 dans lesquelles M, X, Y, Z, L1, R, R¹, n et p présentent les significations qui ont été données selon les revendications 1 et 4, et en outre :
D représente pour chaque occurrence, de manière identique ou différente, C, N ou C-O⁻.

9. Dispositif électronique selon une ou plusieurs des revendications 1 à 8, **caractérisé en ce qu'**il s'agit d'un dispositif électroluminescent organique qui comprend une cathode, une anode et au moins une couche d'émission et en option, d'autres couches qui sont sélectionnées parmi, dans chaque cas, une ou plusieurs couche(s) d'injection de trous, une ou plusieurs couche(s) de transport de trous, une ou plusieurs couche(s) de blocage de trous, une ou plusieurs couche(s) de transport d'électrons, une ou plusieurs couche(s) d'injection d'électrons, une ou plusieurs couche(s) de blocage d'excitons, une ou plusieurs couche(s) de génération de charges et/ou des jonctions p/n organiques ou inorganiques.

10. Dispositif électroluminescent organique selon la revendication 9, **caractérisé en ce que** le composé des formules (3) à (7) est utilisé en tant que composé d'émission dans une couche d'émission, de préférence en combinaison avec un ou plusieurs matériau(x) de matrice, ou **en ce que** le composé de la formule (1) ou des formules (3) à (7) est utilisé en tant que matériau de matrice pour un composé d'émission dans une couche d'émission, ou **en ce que** le composé de la formule (1) ou des formules (3) à (7) est utilisé en tant que matériau de blocage de trous dans une couche de blocage de trous et/ou en tant que matériau de transport d'électrons dans une couche de transport d'électrons, ou **en ce que** le composé de la formule (1) ou des formules (3) à (7) est utilisé en tant que matériau de transport de trous dans une couche de transport de trous et/ou en tant que matériau de blocage d'excitons dans une couche de blocage d'excitons.

11. Composés des formules (4) à (7) et (6a) : 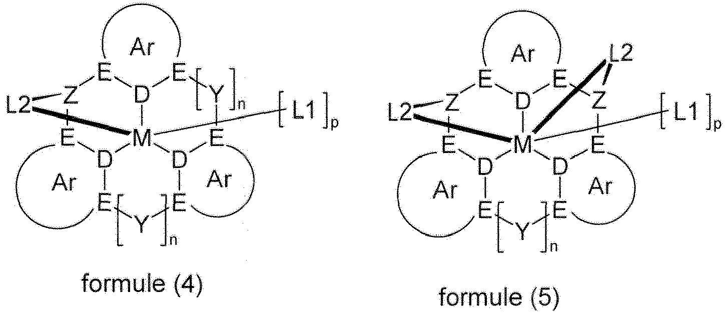 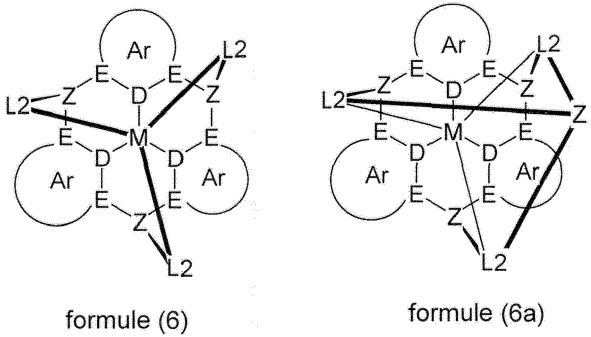 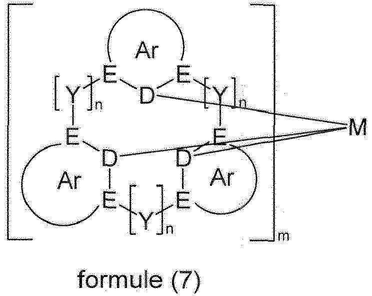 dans lesquelles les symboles et les indices qui sont utilisés présentent les significations qui ont été indiquées selon les revendications 1 et 4 ;
le composé qui suit est exclus de l'invention :

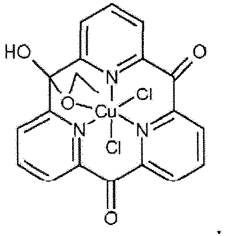

12. Procédé pour la préparation de composés selon la revendication 11 au moyen d'une réaction du ligand de la formule (2) et en option, d'autres ligands L1 avec des alcoolates de métal de la formule (66), avec des cétocétonates de métal de la formule (67) ou avec des halogénures de métal de la formule (68) : 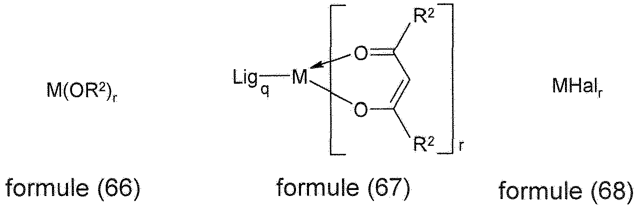 dans lesquelles M et R² présentent la même signification que décrit selon la revendication 1, et ce qui suit s'applique aux autres symboles et indices :
Hal est pour chaque occurrence, de manière identique ou différente, F, Cl, Br ou I ;
Lig est pour chaque occurrence, de manière identique ou différente, un ligand monodenté ou bidenté neutre ou monoanionique ;
q est pour chaque occurrence, de manière identique ou différente, 0, 1, 2, 3 ou 4, de préférence 0, 1 ou 2 ;
r est pour chaque occurrence, de manière identique ou différente,, 1, 2, 3, 4 ou 5, où p dans les formules (66) et (68) indique la valence du métal M ;
le composé de la formule (67) peut également être chargé et peut également contenir un contre-ion ; en outre, les composés des formules (66) à (68), en particulier de la formule (68), peuvent également être sous la forme de l'hydrate.

13. Composés des formules (69) à (71) : 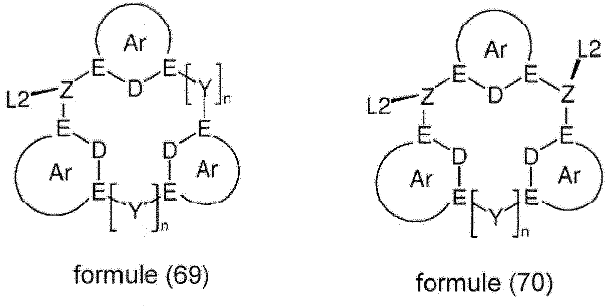 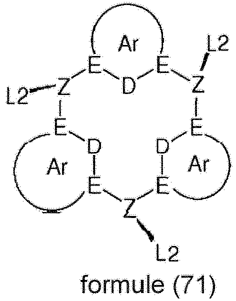 dans lesquelles les symboles et les indices présentent les significations qui ont été données selon les revendications 1 et 4 ; les groupes D et L2, s'ils sont liés au métal M en tant que groupes anioniques dans le complexe, de façon additionnelle dans chaque cas sont porteurs d'un atome d'hydrogène ; les composés qui suivent sont exclus de l'invention :

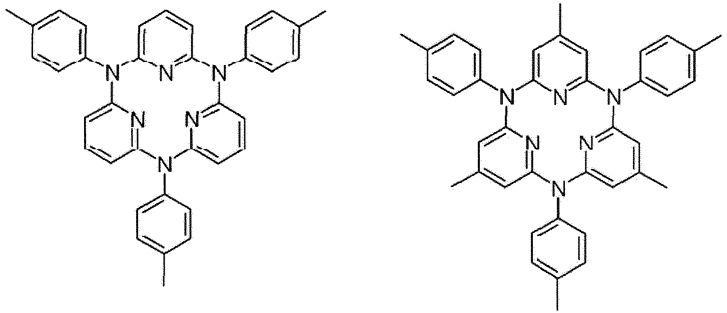

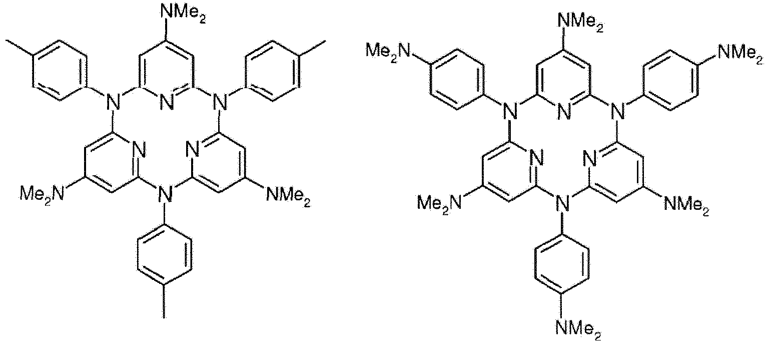

14. Oligomères, polymères ou dendrimères contenant un ou plusieurs composé(s) des formules (3) à (7), dans lesquelles une ou plusieurs liaison(s) est/sont présente(s) depuis le complexe des formules (3) à (7) jusqu'au polymère, à l'oligomère ou au dendrimère.
